# EUROPEAN PATENT APPLICATION

(11) **EP 2 309 270 A1**
(43) Date of publication of application: **13.04.2011**
(21) Application number: 09172401.3
(22) Date of filing: 07.10.2009
(51) Int. Cl.: G01N 33/569, A61K 39/118, C07K 14/295

(54) **Antigenic peptides for the detection of chlamydia-related bacteria and tests of diagnosis**

(71) Applicant: Centre Hospitaller Universitaire Vaudois (CHUV), 1011 Lausanne (CH)
(72) Inventor: Greub, Gilbert, 1073 Mollie-Margot (CH); Kebbi Beghdadi, Carole, 1007 Lausanne (CH); Baud, David, 1012 Lausanne (CH); Riederer, Beat, 1066 Epalinges (CH)
(74) Representative: Schneiter, Sorin

(57) **Abstract**

The present invention relates to the disclosed recombinant or synthetic peptides for use in the diagnosis of an infection by intracellular *Chlamydia*-like bacteria. The invention also relates to a serological diagnostic test. The recombinant peptides are selected from the proteome of *Waddlia chondrophila* properties of binding to antibodies of infected human and animals. The test may give further insight in the role of this microorganism in miscarriage, and in other infectious diseases. Furthermore, the peptides of the invention may be used in vaccination against conditions caused by *Chlamydia*-like bacteria.

## Description

### Technical Field

The present invention generally relates to the fields of diagnosis, microbiology, immunology, vaccination and more specifically to the use of recombinant and/or isolated peptides and/or antibodies binding to said peptides in the diagnosis of an infection by a *Chlamydia-like* microorganism, to a test for diagnosis, and to a method of diagnosis.

### Prior Art and the Problem Underlying the Invention

Novel chlamydiae, amoebae-resisting bacteria, *Chlamydia*-like organisms, and *Chlamydia-*related bacteria are various acronyms used to refer to a large variety of strict intracellular bacteria belonging to the *Chlamydiales* order, but exhibiting enough biological differences with *Chlamydiaceae* to be assigned to other families. The biodiversity of these *Chlamydia-*related bacteria, as evidenced by both molecular-based studies and culture-based studies, led to the proposal of several new families, genus, species and *Candidatus* species. The families of *Waddliaceae* including the species *Waddlia chondrophila* and *W. malayensis, Parachlamydiaceae* including the genera *Parachlamydia, Protochlamydia* and *Neochlamydia, Rhabdochlamydiaceae* including the genus *Rhabdochlamydia, Simkaniaceae,* including the genera *Simkania* and *Fritschea,* and *Criblamydiaceae* including the genus *Criblamydia* have been classified so far. Their taxonomic classification uses among others the 16S rRNA sequence similarity, allowing a reliable phylogeny and precise discrimination between families.

The characteristic of being obligate intracellular bacteria and emerging pathogenic agents make this group of organisms very interesting. Several species might cause severe diseases: *Simkania negevensis* was mainly associated with broncholitis in children, and *Parachlamydia acanthamoebae* was associated with pneumonia, whereas *Waddlia chondrophila* was identified as an agent of abortion in animals, may also commonly cause miscarriage in humans and is a putative agent of pneumonia in humans.

The majority of Chlamydia-like organisms can live in amoeba, which are unicellular organisms classified in the protozoan kingdom. They are ubiquitous eucaryotes living in natural environments such as fresh water, salt water, wet soils or on human mucosa. They are also frequently isolated from anthropogenic ecosystems such as tap drinking water.

Free-living amoebae move by means of cell projection called pseudopodia. Algae, plant cells and bacteria, which are ingested by phagocytosis are their principal diet components. However, several bacteria have developed mechanisms to survive phagocytosis by free-living amoebae and are able to exploit them as hosts. Most of them are members of the *Chlamydiales, Rhizobiales, Rickettsiales* or *Actinobacteriales.* Moreover, strong association between the presence of amoebae and that of *Legionella* strongly support the role of free-living amoebae as reservoirs at least for these intracellular bacteria.

In addition, free-living amoebae may also play a role of "Trojan horse". Thus, most amoebae exhibit at least two developmental stages: (i) a metabolically-active vegetative form, which feeds and multiplies, and (ii) a cystic form, induced by desiccation, low pH, osmotic pressure or temperature variation and which resists to biocides and chlorination. The internalized bacteria may thus survive within the amoeba during adverse conditions. The intracellular location in amoeba also allows wide spreading of the bacteria and increases the infection potential of secondary hosts.

*Waddlia chondrophila* is an obligate intracellular parasite isolated in 1989 from tissues of a first-trimester aborted bovine fetus. The lifecycle is almost the same as for the *Chlamydiales.* It alternates between a dense and metabolically inactive form, which is adapted to external survival and infection of new eukaryotic host cells and a large reticulate, intracellular and metabolically active form, which undergoes binary fission. Two hours post inoculation, the dense forms have already entered the host cells via endocytosis by attachment to the cell membranes (at coated pits). Once inside, the bacteria are contained in vacuoles, themselves located in the cytoplasm and consequently surrounded by an inclusion membrane. There, the bacteria change into reticulated forms and become often surrounded by mitochondria. Then, the reticulate forms multiply by binary fission, forming inclusions. The bacteria maturates back from the reticulate form into the dense form. The several bacterial inclusions inside the same host cell do not evolve at the same speed, thus different stages of maturity can be seen within the same cell. Then, new infective dense form bacteria are released in the medium by lysis of the host cell.

Little is known on *Waddlia chondrophila,* which is currently considered as an emerging pathogen. It is essentially taken as an abortigenic agent in bovine species and recently, Baud et al. ("Waddlia chondrophila, a Potential Agent of Human Fetal Death", Emerging Infectiuous Diseases, Vol. 13, No. 8, 2007) have shown that this organism causes miscarriage in humans. Even more recently, Haider et al identified a putative case of pneumonia likely due to Waddlia chondrophila (FEMS Microbiol. Lett., 2008 Apr; 281(2): 198-202). Other *Chlamydiales* species have also been clearly associated with abortion in animals. Thus, *Chlamydophila abortus* which cause as many as 45% of abortion in ruminants has an important influence on agricultural economy.

The detection of these emerging pathogenic agents is a major problem. Only few diagnostic approaches are yet available. Currently used diagnostic serological tests are very cumbersome because they are generally based on lysed or intact bacteria, which are difficult to cultivate, given their obligate intra-cellular life style.

In a view to the above, it is an objective of the present invention to provide more information and knowledge about Chlamydia-like bacteria, and in particular about *Waddlia chondrophila.*

It is a first objective of the invention, to provide a diagnostic tool and/or method of diagnosis of *Chlamydia*-like organisms, in particular *Waddliaceae,* such as *W. chondrophila.*

Furthermore, it is an objective to provide a diagnostic tool or a method of diagnosis (i) that is sensitive and thus detects low infection levels (immunohistochemistry) and (ii) that detects evidence of current and/or previous exposure to a *Waddliaceae* (ELISA). In addition, or alternatively, it is an objective to provide a diagnostic tool or a method of diagnosis that is accurate and/or specific. In other words, the different diagnostic tools (ELISA and/or immunohistochemistry, for example) preferably avoids false positive or false negative outcomes, in particular is preferably devoid of cross-reactivity towards related, even closely related species. Conversely, according to the context, some level of cross-reactivity (for instance between all genera of the *Waddliaceae* family), may be useful to detect infection by any *Waddliaceae.* A tool or method directed to the diagnosis of members of the family of *Waddliaceae,* of members of the genus *Waddlia* or of the species *W. chondrophila* preferably does not react to infections stemming from bacteria other than those from members of *Waddliaceae, Waddlia* and *W. chondrophila,* respectively. It is thus an objective to provide a diagnosis that avoids false positives.

It is also an objective of the present invention to provide a vaccine suitable for preventing infections by *Waddlia* and thus preventing miscarriage in humans and animals infected or at risk of potentially being infected by *Waddlia* in the future.

It is in particular an objective to provide polypeptides that can be used in diagnostic tests and methods and in vaccines. It is also an objective to provide proteins, in particular recombinant proteins, that have suitable antigenic and/or immunogenic properties and that thus are useful in diagnosis and treatment, in particular by vaccination.

It is also an objective to provide an improved, easy approach to perform serological test, such as an Enzyme-linked Immunosorbent Assay (ELISA) or another serological test, for example an epifluorescence immunoassay. It is also an objective to provide other rapid tests.

It is a further objective of the present invention to provide an immunohistochemic assay and a method of detecting *Waddlia* or any *Chlamydia*-related bacteria in tissue samples.

It is also an objective of the present invention to provide proteins and/or antigens against which polyclonal and/or monoclonal antibodies may be raised and used to detect *Waddlia* and/or related bacteria in animal and/or humans tissues thanks to immunochemistry or similar approaches.

More generally, it is an objective of the invention to provide ways and tools that enable to further precise the role of the obligatory intracellular bacteria of the genus *Waddlia* as human and/or animal pathogens.

### Summary of Invention

Remarkably, the present inventors identified antigenic proteins of *Chlamydia*-like intracellular microorganisms. The proteins are useful in the diagnosis of an infection by *Waddlia,* advantageously by way of a serological test or using immunohistochemistry, or in the preparation of antibodies binding to the peptides. For serological approaches, it is possible to rapidly test an individual for the presence of infection with *Waddlia* on the basis of a small blood sample, for example.

Accordingly, the present invention provides, in a first aspect, the use of at least one peptide in the diagnosis of an infection by an intracellular *Chlamydia*-like microorganism.

In a second aspect, the present invention provides the use of at least one recombinant antigenic and/or immunogenic peptide in the diagnosis of an infection by an intracellular microorganism of the *Waddliaceae.*

In a third aspect, the present invention provides the use of at least one isolated anti and/or immunogenic peptide in the diagnosis of an infection by an intracellular microorganism of the *Waddliaceae.*

In a fourth aspect, the present invention provides the use of at least one synthetic peptide anti-and/or immunogenic peptide in the diagnosis of an infection by an intracellular microorganism of the *Waddliaceae.*

In a fifth aspect, the present invention provides the use of at least one recombinant, isolated and/or synthetic antigenic and/or immunogenic peptide in a vaccine against an intracellular microorganism of the *Waddliaceae.* Accordingly, the present invention provides the use of the peptides in the treatment and/or prevention of an infection by an intracellular microorganism of the *Waddliaceae.*

In a sixth aspect, the present invention provides a vaccine composition comprising one or more isolated and/or recombinant immunogenic peptides of an intracellular bacterial strain of the family *Waddliaceae.*

In a seventh aspect, the present invention provides a recombinant and/or isolated polypeptide of a bacterial strain belonging to the genus *Waddlia* and/or to the species *W. chondrophila.*

In an eighth aspect, the present invention provides a recombinant, isolated and/or synthetic peptide comprising an amino acid sequence selected from: (a) a continuous stretch of at least 10 amino acid residues selected within any one of the amino acid reference sequences SEQ. ID. NO. 1-36; (b) an amino acid sequence having at least 30% or more sequence identity with a any one of the amino acid sequences as defined under (a); (c) a variant sequence of (a) and (b). Preferably, the peptide is recombinant or synthetic, isolated or non-isolated.

In a nineth aspect, the present invention provides a method of producing an antibody, the method comprising the steps of: (1) exposing a mammal to the isolated and/or recombinant peptide of the invention; and, (2) harvesting said antibody from serum of said mammal.

In a tenth aspect, the present invention provides an isolated antibody directly or indirectly obtained or susceptible of being obtained by the method of the invention, and/or an antibody specifically binding to a recombinant and/or isolated peptide of a *Waddliaceae* microorganism.

In a eleventh aspect, the present invention provides the use of an antibody, for example a monoclonal and/or polyclonal antibody, specifically binding to a peptide of the invention in the diagnosis of infection by a microorganism belonging to the *Waddliaceae.*

In an twelfth aspect, the present invention provides a test kit of diagnosis comprising one or more isolated and/or recombinant antigenic peptides of an intracellular bacterial strain of the family *Waddliaceae* and/or an antibody specifically binding to said isolated and/or recombinant antigenic peptides.

In an thirteenth aspect, the present invention provides a method of diagnosing infection of an individual by an intracellular *Chlamydia*-like organisms, the method comprising the steps of contacting a serum and/or a blood sample of an individual with one or more recombinant and/or isolated antigenic peptides of the *Chlamydia*-like organism; determining the presence or absence in said sample of an antibody specifically binding to at least one of said peptides; and diagnosing an infection if an antibody is detected in the previous step.

In a fourteenth aspect, the present invention provides a method of diagnosing infection by an intracellular *Chlamydia*-like microorganism, the method comprising the steps of exposing tissue to an antibody specifically binding to an antigenic peptide selected from peptides according to the invention; detecting if said antibody binds to said tissue; and, diagnosing, if there is such binding that there is an infection by said *Chlamydia*-like microorganism.

In a fifteenth aspect, the present invention provides the use of an antibody specifically binding to a peptide selected from peptides of the present invention in a immunohistochemical test for detecting the presence of a *Chlamydia*-like microorganism in tissue, in particular samples of tissue.

In yet another aspect, the present invention further provides a test designed to assess a risk of miscarriage. In particular, in a further aspect, the present invention provides a test for identifying a risk of adverse pregnancy outcomes such as miscarriage and/or any diseases due to infection by a *Waddliaceae.*

In an aspect, the present invention provides vaccines and/or vaccine composition as well as methods of vaccination for treating and/or preventing one or more conditions selected from the group of lower and/or upper respiratory tract infections, including bronchitis, bronchiolitis and/or pneumonia, conjunctivitis, infections of the urogential tract, abortion and pre-term labour in animals and miscarriage and pre-term labour in humans, amongst other conditions.

Further aspects and preferred embodiment of the present invention are as provided in the appended claims.

### In the drawings,

**Figure 1** shows a 2-dimensional, Coomassie blue stained SDS PAGE of a crude extract of *Waddlia chondrophila.* The extract was separated using a pH 3-11 NL IPG strip in the first dimension followed by a 12.5% SDS PAGE in the second dimension. Specific spots of the most frequent antigenic peptides are encircled and numbered as described in the examples. Proteins subjected to MS analysis are encircled in brighter colour.
**Figures 2A-E** show western blots ofwaddlial proteins exposed to (A), (B), (C), and (D) sera of four different human patients tested positive for *Waddlia* and (E) to serum of a rabbit immunized with *Waddlia.*
**Figures 3A-C** illustrate the method for detecting immunogenic proteins from a *Chlamydia-*like organism. Figure 3A is a 2D Coomassie blue stained SDS PAGE of a *Waddlia chondrophila* proteome (similar to Figure 1 but with spots not yet being numbered); Figure 3B is a western blot obtained with human *Waddlia* positive serum; and Figure 3C is the superposition of A and B, which allows to identify immunogenic proteins.

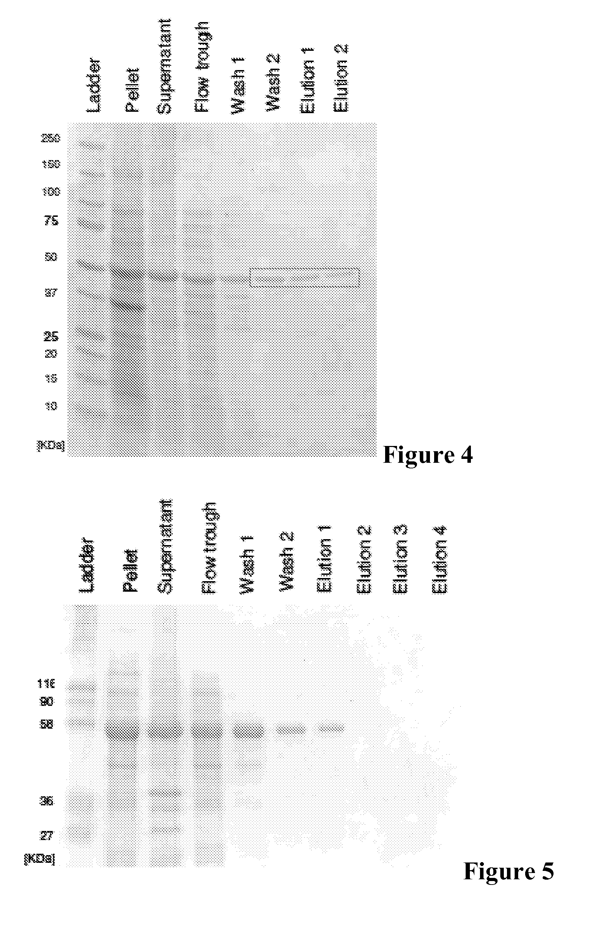

**Figure 4** shows recombinant WaA7 with a 6xHis-tag purified under native conditions from transfected and lysed *E. coli* cells, submitted to SDS-page and Coomassie-blue stained. The box indicates the purified protein.
**Figure 5** shows recombinant WaA 22 with a 6xHis-tag purified under denatured conditions from transfected and lysed *E. coli* cells and submitted to an SDS-page gel and Coomassie-blue stained.

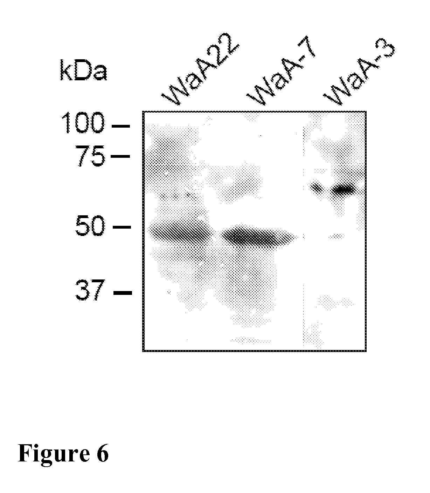

**Figure 6** shows western blots recognizing recombinant proteins according to the present invention. The proteins correspond to spots 22 (WaA 22), 7 (WaA 7) and 3 (WaA 3) in Tables 1 and 2 and Figure 1 (SEQ ID NO:10, 7, and 3, respectively) and are blotted on a nitrocellulose membrane probing with a serum from a rabbit immunized with *Waddlia chondrophila.*

### Detailed Description of the Preferred Embodiments

In an aspect, the present invention relates to the use of at least one peptide in diagnosis. Preferably, diagnosis is the diagnosis for infection by a *Chlamydia*-like microorganism. As indicated above, the term "*Chlamydia*-like" or "*Chlamydia*-related" microorganism, for the purpose of the present specification, encompasses a large variety of strict intracellular bacteria belonging to the *Chlamydiales* order, but which exhibit enough biological differences with *Chlamydiaceae* to be assigned to other families.

According to a preferred embodiment, the *Chlamydia*-like microorganism belongs to the family of *Waddliaceae,* more preferably to the genus of *Waddlia,* and most preferably to the species *Waddlia chondrophila.* When reference is made to *Chlamydia*-like in a particular aspect or embodiment of the present invention, such reference also includes in a preferred embodiment reference to *Waddliaceae* and in a more preferred embodiment reference to *Waddlia.* Similarly, reference to the genus of *Waddlia* also includes, in a preferred embodiment, reference to *W. chondrophila.*

The present invention relates to uses, tests, diagnosis, vaccines and methods. These are all interrelated such that reference herein to a specific test or use, for example, automatically also refers also to the methods and diagnosis disclosed herein, and *vice versa.*

The present invention relates to antigenic peptides. Antigenic peptides are peptides that bind to a specific antibody or to a T-cell receptor.

According to a preferred embodiment, in particular with respect to the vaccine of the invention, the peptides of the invention are immunogenic, that is, they are capable of inducing a humoral or cell-mediated immune response in a mammalian organism.

According to an embodiment, the diagnosis and or tests disclosed herein are specific. "Specificity", for the purpose of the present specification, can be at the order level, at the family level, at the genus level and at the level of the species. The tests of the invention can even be specific for a strain within a species.

In one embodiment, the methods, tests and/or diagnosis as disclosed herein are specific at the level of the order, meaning that they allow diagnosis of infection by a given clade (i.e. any *Chlamydiales*), but does not yield a positive result when there is an infection, for example, of other Eubacteria. In other words, when the result of the diagnosis and/or the test is positive, it can be said that the positivity relates to an infection by a bacterial species of the order of the Chlamydiales, and not to an infection by a bacterium from another order. This does not exclude the possibility that an infection by such a bacterium of another order is also present, but it specifies that an infection by a bacterium that is part of this order is present.

According to an embodiment, the methods, tests and/or diagnosis disclosed herein are specific at the level of the family of the *Waddliaceae.* In analogy of the before mentioned specificity with respect to the order, this means that the present invention allows the diagnosis of infection by any *Waddliaceae,* but does not yield a positive result when there is an infection, for example, by a species belonging to the same order (*Chlamydiales*) but to another family than *Waddliaceae.*

According to an embodiment, the methods, tests and/or diagnosis disclosed herein are specific at the level of the genus. In analogy to the paragraphs above, this means that the present invention allows the diagnosis of infection by any *Waddlia,* but does not yield a positive result when there is an infection, for example, of another genus of the family of the *Waddliaceae.*

According to an embodiment, the methods, tests and/or diagnosis disclosed herein are specific at the level of the species any *W. chondrophila.* In analogy to the paragraphs above, this means that the present invention allows the diagnosis of infection by any strain belonging to the species of *W. chondrophila,* but does not yield a positive result when there is an infection, for example, of another species of the genus of *Waddlia.*

According to an embodiment, the diagnosis and/or the test disclosed herein are specific for an intracellular bacterial species.

Due to the possible role of *Chlamydia*-like microorganisms as agents of several conditions, such as miscarriage, pre-term labor, and pneumonia in humans and in abortion in animals, and other conditions discussed in further detail below, the present invention also relates to the diagnosis of these conditions and/or in the assessment of a risk and/or possibility of imminent or future contracting of one or more of these conditions.

A "peptide", for the purpose of the present specification, refers to a genus of peptide or peptide fragments that encompass the amino acid sequences identified herein, as well as smaller fragments. A peptide is preferably defined in terms of its antigenic relatedness to any peptide disclosed herein. Thus, in one embodiment, a peptide within the scope of the invention is defined as an amino acid sequence comprising a linear or 3-dimensional epitope shared with any amino acid sequence disclosed herein.

According to a preferred embodiment, a peptide within the scope of the present invention is recognized by an antibody that specifically recognizes and/or binds to any peptide having an amino acid sequence as disclosed in the present specification. Antibodies are defined to be specifically binding if they bind peptides of the invention with Kₐ of greater than or equal to about 10⁷ M⁻¹, such as greater than or equal to 10⁸ M⁻¹.

The peptide may be a polypeptide (protein) or an oligopeptide, having from 3-10 amino acids. The size of the peptide is not crucial, as long as it has the minimum size for providing an antigenic entity and/or an epitope, which will be specifically targeted by the immune system of a human or animal subject. Preferably, a peptide comprises a continuous amino acid sequence of at least 10 amino acids, more preferably at least 15 and most preferably at least 20 amino acids, and/or as defined further below.

The term "to comprise" or "comprising", for the purpose of the present specification, is intended to mean "includes amongst other". It is not intended to mean "consists only of".

According to the invention, at least one peptide, as characterized by a specific amino acid sequence, is needed for the purpose of diagnosis, for example in the test of the present invention. Preferably, however, two or more different peptides are used, preferably three and most preferably four or more peptides. The use of a combination of a certain number of different, selected peptides reduces cross-reactivity and thereby enables to reduce the number of false positive and/or false negative diagnostic outcomes.

The present invention also relates to the production of an antibody by using the peptides of the invention as antigens. The antibody may be produced according to any currently known method. The antibody preferably specifically binds to a peptide of the present invention.

The peptide of the present invention is preferably a recombinant, isolated and/or synthetic peptide. A recombinant peptide can be produced by any suitable organism, for example a bacterium, an eucaryotic cell, a multicellular organism such as a transgenic plant or animal, for example. The recombinant peptide may be isolated or may not be. The recombinant peptide may, for example, be used in the form of an unpurified bacterial lysate, together with other cellular components.

The isolated peptide may be recombinant or may not be recombinant, for example isolated from the original organism. The peptide may be isolated according to any peptide and/or protein isolation and/or purification procedure.

The peptide may, of course, also be produced synthetically, by organic chemical synthesis, as opposed to recombinant production in a biological system (biosynthesis). In this former case, it may also be isolated or not isolated. According to an embodiment, the peptide may be recombinant or synthetic, and it both cases it may be isolated or not isolated. Therefore, the peptide may preferably be selected from: recombinant and isolated peptides; recombinant and non-isolated peptides; synthetic and isolated peptides, synthetic and non-isolated peptides, for example. In the methods, kits and uses of the invention, mixtures of recombinant and synthetic peptides may be used.

The peptide is preferably selected from proteins with the amino acid sequences of SEQ ID NO: 1-36. Amino acid sequences SEQ ID NO:1-13 were selected on the basis of their antigenic properties. Epitopes present on these proteins are recognized by individuals tested positive for an infection by *Waddlia.* SEQ ID NO: 14-36 are determined from DNA sequences selected from the genome of the *W. chondrophila* strain established for the purpose of the present invention (Example 5). The sequences SEQ ID NO: 14-36 are thus selected by their sequence homology with proteins known as strongly immunogenic antigens in other organisms.

According to a preferred embodiment, the peptide of the invention comprises an amino acid sequence selected from:
(a) a continuous stretch of at least 10 amino acid residues selected within any one of the amino acid reference sequences SEQ. ID. NO. 1-36;
(b) an amino acid sequence having at least 30% or more sequence identity with a any one of the amino acid sequences as defined under (a);
(c) a variant sequence of (a) and (b).

Under (a), the present invention thus encompasses peptide fragments of the amino acid sequences defined by any one of SEQ. ID. NO. 1-36. According to a preferred embodiment, the continuous stretch under (a) of amino acid residues within any one of SEQ. ID. NO. 1-36 (or fragment of any one of SEQ. ID. NO. 1-36) spans at least 20, more preferably at least 30, 40, 50, 60, 70, 80, 90, 100, 120, 140, 160, 180, 200 or more amino acid residues. The expression "or more" thus refers at most to the total number of amino acid residues of the respective sequence. Accordingly, the continuous stretch under (a) may span the entire amino acid sequences as defined in any one selected from SEQ. ID. NO. 1-36. The above figures of continuous 10 or more amino acid residues up to the entire sequence of any one of SEQ. ID. NO. 1-36 thus define the length of comparison, for example for the purpose of determining sequence identity.

Since the peptide is used in diagnosis and preferably in an antibody-based, serological test, the fragment is preferably sufficiently large to be recognized by an antibody that specifically recognizes and/or binds to the fragment having an amino acid sequence as defined herein. The fragment of the invention may be used as such or as a stretch of amino acids in a larger polypeptide and/or amino acid sequence. For example, the antigenic fragment may be combined with, for example fused to, a non-antigenic amino acid sequence.

According to a preferred embodiment, the amino acid sequence of (b) has at least 60%, more preferably at least 80%, even more preferably at least 40%, 50%, 60%, 65%, 70%, 75%, 80%, 85%, 90%, 91%, 92%, 93%, 94%, 95%, 96%, 97%, 98% or 99% identity with any one of the amino acid sequences defined under (a). These percentages may be selected independently for any peptide or amino acid sequence as defined herein.

Sequence identity is determined by way of the blast sequence comparison described in Example 7, including the parameters described there. Sequence identity of a sequence of comparison with respect to an original sequence is reduced when, for example, any one of the compared or the original sequence lacks amino acid residues, has additional amino acid residues and/or has one or more amino acid residue substituted by another residue. Sequences having as little as 30% sequence identity with any sequence as defined under (a) may still provide functional, that is, antigenic peptides that are suitable to meet the objectives of the invention.

According to an embodiment, the continuous stretch as defined under (a) is selected from within a sequence as defined in any one of the groups of SEQ. ID. NO.: 1-13; of SEQ. ID. NO.: 3, 4, 10 and 12; from the group of SEQ. ID. NO.:14-20; from the group of SEQ. ID. NO.: 21-25; from the group of SEQ. ID. NO.: 26-36; and/or from combinations of these groups, such as, for example, from one or more of the following groups:
i) SEQ. ID. NO.: 3, 4, 10, 12, 14-36;
ii) SEQ. ID. NO.: 3, 4, 10, 12, 14-25;
iii) SEQ. ID. NO.: 3, 4, 10, 12, 14-20;
iv) SEQ. ID. NO.: 3, 4, 10, 12, 14-20, 26-36;
v) SEQ. ID. NO.: 3, 4, 10, 12, 14-26;
vi) SEQ. ID. NO.: 3, 4, 10, 12, 21-36;
vii) SEQ. ID. NO.: 14-20, 26-36;
viii) SEQ. ID. NO.: 14-25;
ix) SEQ. ID. NO.: 21-36;
x) SEQ. ID. NO.: 1-20;
xi) SEQ. ID. NO.: 1-20, 26-36;
xii) SEQ. ID. NO.: 1-25;
xiii) SEQ. ID. NO.: 26-36.

Most preferably, the continuous stretch as defined under (a) is selected within any one of the group of SEQ.ID. NO.: 3, 4 and 10 (WaA 3, WaA 7 and WaA22).

Under point (c), the peptide of the invention is defined as a variant sequence of any one of the sequences as defined under (a) or (b) above.

Variants, for the purpose of the present specification, mainly cover two situations.

First, variant relate to peptides comprising amino acid sequences as defined under (a) and (b), which sequences contain insertions and/or deletions encompassing at least three but possibly more amino acids. Such deletions and/or insertions may have no effect on the immunogenic properties of the peptides, but may be used to provide further and/or other functionalities to the peptide as such. Such insertions and/or deletions of larger scale may to a large extent affect the sequence identity as defined under point (b) so that the latter is rendered meaningless, even if the immunogenic properties of the sequences disclosed herein are exploited. Therefore, the present invention also encompasses such variants. According to an embodiment, the variant includes, independently one from the other, zero, one, two or more insertions and/or deletions encompassing continuous stretches which independently may extend over three or more continuous amino acids, preferably 4 or more, 5, 10, 15, 20, 30, 40, 50, 60, 70, or more continuous amino acids. For example, the insertion and/or deletion may extend to up to 5, 10, 20, 30, 40, 50, 60, 70, 80, 90, 100, 120, 150, 170, 200 amino acid residues. A second type of variant under (c) relates to the substitution of one or more amino acid residues of any one of the amino acid sequences of (a) and/or (b) by residues having equivalent properties. This kind of substitution is generally known as conservative substitution.

Accordingly, in an embodiment, a variant sequence refers to a sequence amino acid sequence comprising conservatively substituted amino acid residues if compared to the sequences as defined under (a) and (b) above, meaning that one or more given amino acid residues are replaced by a residue having similar physiochemical characteristics. Examples of conservative substitutions include substitution of one aliphatic amino acid residue for another, such as Ile, Val, Leu, or Ala for one another, or substitutions of one polar residue for another, such as between Lys and Arg; Glu and Asp; or Gln and Asn. See Zubay, Biochemistry, Addison-Wesley Pub. Co. (1993). The effects of such substitutions can be calculated using substitution score matrices such as PAM-120, PAM-200, and PAM-250 as discussed in Altschul (J. Mol. Biol. 219:555-65, 1991). Other such conservative substitutions, for example, substitutions of entire regions having similar hydrophobicity characteristics, are well known.

These variants comprising conservatively substituted amino acids are separately considered here because such substitutions can have an impact on the identity level of compared to an original sequence as defined under (b), so that the figures of identity indicated with respect to (b) would be rendered meaningless to some extent. Accordingly, in an embodiment, conservatively substituted amino acid residues may make up to 50%, preferably however 40% or less, 30, 20, 15, 10, 9, 8, 7, 6, 5, 4, 3, 2, 1% or less of the amino acid sequences under (a) or (b).

Further variants are, for example, naturally peptide variants that result from alternate mRNA splicing events or from proteolytic cleavage of the peptides described herein. Variants attribuatable to proeolysis include, for example, differences in the N- or C-termini upon expression in different types of host cells, due to proteolytic removal of one or more terminal amino acids from the polypeptides encoded by the sequences of the invention.

Fusion peptides and/or proteins are also encompassed by the variant peptides for the purpose of the present invention. Fusions of additional peptide sequences at the amino and/or carboxyl terminal ends of the polypeptides of the invention may be used to enhance expression and/or extracellular secretion and/or may aid in the purification of the protein, for example. For example, peptides as defined herein further comprising a signal peptide and/or a his-tag or a different tag fulfilling any specific function are also encompassed by the present invention.

It is also possible to create a fusion protein containing a non-antigenic amino acid sequence combined with an amino acid sequence or fragment thereof as defined herein.

The embodiments detailed above, based mainly on SEQ. ID. NO.: 1-36 relate to preferred peptides as detailed in the examples, which are selected in order to make a diagnosis more specific with respect to the microorganism and/or more sensitive. They are also selected so as to avoid cross-reactivity.

The embodiments detailed above concerning preferred peptides relate to all uses, diagnosis, tests, assays, vaccines, vaccine compositions and methods disclosed herein, in which peptides are used. For example, the preferred peptides are also preferred with respect to the method of preparing an antibody, as disclosed further below.

The present invention also relates to a test of diagnosis. According to an embodiment, the test is a serological test, which means that a diagnosis can be made on the basis of a blood and/or serum sample of an individual. Preferably, a serum sample is used. Preferably, a sample comprises 5ml or less of blood and/or serum of an individual, for example 0.5ml or less. Preferably, the sample is diluted, the test being sufficiently sensitive to provide an accurate result with the diluted sample. For example, the dilution is at least 1/2, more preferably at least 1/4, and most preferably at least 1/8.

The test is preferably an antibody-based test kit, which means that the presence of antibodies specifically binding to a peptide as defined herein, is detected, said antibody being contained in the blood and/or serum sample of an individual. This implies the presence of a sufficient amount of such antibody in the sample.

An example for a diagnostic test of the present invention is an Enzyme-Liked Immunosorbent Assay (ELISA). Another example are immunofluorescent strips as marketed by the company InoDiag in 83'870 Signes, France, under the trademark InoMu.S.T.®. These glass strips contain a spot comprising antigenic peptides. The spot incubated with a diluted blood sample and with further reactants generally comprising an antibody selective for the constant domains of a specific human antibody type. Subsequently, the strip is fluorescence analysed in an automated process and the diagnosis is accomplished. The incubation with serum as well as the reading of the fluorescent signal and its interpretation is generally done in an automated process. The present invention thus encompasses this type of serological test, in which antigenic peptides of a *Chlamydia*-like microorganism, in particular a microorganism belonging to the *Waddliaceae,* in particular the peptides of the invention, are used. These types of serological tests are disclosed, for example, in Gouriet et al., "Comparison of the new InoDiag automated fluorescence multiplexed antigen microarray to the reference technique in the serodiagnosis of atypical bacterial pneumonia", CMI, 2008, 14, 1119-1127, and Gouriet et al., 2008, CMI, 14, 1112-1118.

According to another embodiment, the test of the invention is based on the detection of an antigen.

For example, the test of the present invention may be a urine-based test. Such a test comprises, for example, an antibody that binds to antigens possibly present in the urine. The kit may contain a second antibody recognizing the first antibody and containing a reporting mechanism or system that permits the detection of a binding between the first antibody and the antigen, exploiting the same general principle as the ELISA test above, with the difference that the presence of an antigen instead of an antibody is detected. Variations of this pattern are possible. For example, a first antibody could be provided on a column, binding *Waddlia* antigens, if present, in a urine sample. Recognition of the presence of the bound antigen can be made as is conventional, for example by adding a second antibody that is also specific to the antigen, but preferably to a different epitope, which second antibody again comprises a conveniently detectable reporting system.

Such a kit thus preferably comprises at least one antibody of the present invention, specific for the recombinant and/or isolated peptides of the invention. Such a kit preferably comprises a second antibody, which specifically binds to the first antibody or to the same antigen as the first antibody, but preferably to a different epitope.

The use, test and the method of the present invention preferably comprises one or more isolated and/or recombinant antigenic and preferably immunogenic peptides of an intracellular bacterial strain of the genus *Waddlia,* preferably of the species *W. chondrophila,* and most preferably of the ATCC *W. chondrophila* strain referred to in the examples.

The present invention also relates to a method of producing an antibody, the method comprising the steps of exposing a mammal to a peptide as disclosed herein, and, following exposure, harvesting polyclonal antibodies from the serum of said mammal. The peptide may be administered to the mammal as an isolated peptide, as a recombinant peptide, as an unpurified bacterial extract containing the peptide, for example, an unpurified lysate and/or bacterial extract of recombinant bacteria expressing said peptide. Preferably, one, two, three, four, five or more recombinant peptides are used for immunisation of the mammal. Preferably, the peptides or any composition comprising the peptides are administered by injection, for example subcutaneous injection. The peptide may be administered in the form of a composition, for example an aqueous solution comprising the peptide or a mixture of different peptides as defined and disclosed herein.

The present invention also provides a monoclonal antibody, wherein said antibody specifically binds to a peptide as defined herein, and also to a hybridoma producing the antibody. Methods for producing a hybridoma from myeloma cells and cells isolated from immunized individuals, such humans or animals, in particular mammals, in particular mice, rabbits, goats, for example, are known. With respect to the present invention, a mammal is immunized with an isolated and/or recombinant peptide as defined herein, or composition of two or more different peptides as defined herein. Specifically binding, for the purpose of the present invention, may be determined by immunofluorescence, western blotting, ELISA and/or immunohistochemistry, while using the corresponding antigen.

The polyclonal and/or monoclonal antibodies referred to herein are preferably isolated antibodies.

The antibodies of the present invention may be used, for example, in urine-based tests described above but also in immunohistochemical methods, assays and/or tests for detecting a *Chlamydia*-like microorganism in tissue, in particular tissue samples. These methods, assays and/or tests may comprise primary and secondary antibodies, wherein the primary antibody is the antibody of the present invention, and a secondary antibody is provided that specifically binds to said primary antibody and the presence of which secondary antibody can be visualized, for example by linking an enzyme to said secondary antibody and/or a fluorescent dye, as is conventional in immunohistochemistry.

The tissues and/or tissue samples mentioned herein are preferably prepared and/or treated so as to disrupt cell membranes, of the tissue and thereby expose antigens possibly present in the tissue. The tissues may also be treated so as to unmask the antigens in order to allow for better antibody-antigen recognition.

The peptides and test disclosed herein may be used in the diagnosis of an infection of *Chlamydia*-related organisms and/or in the diagnosis of various diseases and/or conditions.

*Waddliaceae,* such as *W. chondriophila* are possibly involved and/or causative agents for various conditions, such as lower and/or upper respiratory tract infections, for example infections of the nasal concha, bronchitis, bronchiolitis and/or pneumonia, conjunctivitis, uveitis, infections of the urogential tract, kidney infection, pericarditis, infertitlity, they may cause abortion and pre term labour in animals and miscarriage and pre term labour in humans, amongst other conditions.

The antigenic peptides of the invention may be used in the diagnosis of the above conditions, to assess the risk and/or possibility of imminent or future contracting of one or more of these conditions and, in particular, to find if a Chlamydia-like organism is at the origin of the condition. Furthermore, the vaccines and/or methods of vaccination of the present invention may be used prevent and/or treat the above-mentioned conditions.

The above mentioned conditions may appear in humans or animals. According to an embodiment, the vaccine is destined to humans. According to another embodiment, the vaccine of the invention may be used in particular to treat one or more of these conditions in animals, for example domestic animals, in particular livestock. For example, the vaccine is used in the prevention and/or treatment of one or more of said conditions in ruminants, such as cattle, sheeps, goats, and the like.

The peptides of the present invention may thus be used in vaccine compositions and in methods of vaccination.

The invention is now illustrated by way of the examples below, which are not intended to limit the scope of the present invention.

### Examples

### Patients

Sera were obtained from the study of Baud, D., et al. (Waddlia chondrophila, a potential agent of human fetal death. Emerg Infect Dis, 2007. 13(8): p. 1239-43) and from a study of Swiss army patients (Baud et al, Clin Microbiol Infect. 2009 May;15(5):499-501). In these studies, individuals were tested positive for an infection by *Waddlia* by immunofluorescence. In total, 13 *Waddlia*-positive human blood samples were used at 1/8 dilution, as well as rabbit sera infected with different *Chlamydia*-like bacteria at 1/25 dilution and mouse sera infected with *Waddlia chondrophila* at 1/1000.

### Example 1: Cultivation and purification of Waddlia chondrophila

*W. chondrophila* ATCC VR-1470 (obtained from LGC Standards, Molsheim) was cultivated and purified on a sucrose barrier and gastrographin gradients as described by G. Greub, J.-L. Mege and D. Raoult "Parachlamydia acanthamoebae Enters and Multiplies within Human Macrophages and Induces Their Apoptosis", Infection and Immunity, Oct. 2003, p. 5979-5985, more particularly on page 5979 under the title "Materials and methods" the paragraph "*P. acanthamoebae* culture and purification". This procedure was applied with the exception that *W. chondrophila* instead of *P*. *acanthamoeba* was used as infective agent and that the A. *castelanii* strain ATCC 30010 instead of *A*. *polyphaga* was used as a host.

Furthermore, there was no tittering, lysis test and freezing conducted. Instead, the large lower band of *Waddlia* resuspended twice in PBS was subjected to lysis and the extracted proteins were separated by 2D gel electrophoresis as reported below.

### Example 2: Crude extract sample preparation and 2-D gel electrophoresis

Purified bacteria (mostly elementary bodies, EB) were washed twice in 10mM Tris, 5mM MgAc, pH 8.0 and then lysed by 5 cycles of short-pulse sonication in lysis buffer (30 mM Tris, 7M urea, 2M Thiourea, 4% CHAPS and adjusted to pH 8.5). Proteins were recovered by centrifugation at 8'0000 rpm and quantified using a Bradford assay (Quick Start^{™} Bradford Protein Assay, Bio-Rad laboratories, Hercules, USA). Routinely about 4 mg of total *Waddlia* proteins were obtained from 60 T75 flasks of amoebal co-culture. Aliquots of 1.2 mg were stored at minus 80°C for subsequent electrophoretic analysis.

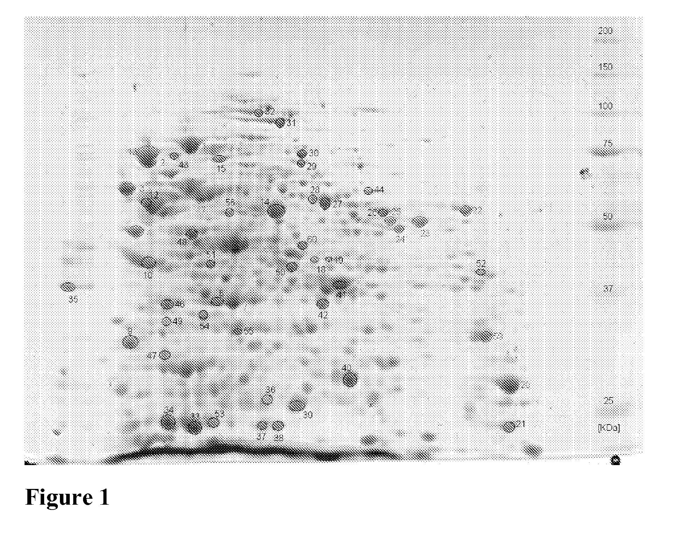

Two dimensional gel electrophoresis was performed as described by Centeno et al (Centeno et al., Cell Death and Differentiation, 2007, 14, p.240-253) using approximately 150 µg (mini gels) or 600 µg (midi-gels) of total EB proteins for each electrophoretic run. Proteins were visualized by Coomassie Blue staining or transferred to nitrocellulose. **Figure 1** shows the 2D gel obtained.

### Example 3: Immunoblot Analysis

Nitrocellulose membranes were blocked by 2 hours incubation with 5% non-fat dry-milk in Tris-buffered saline with 0.05% Tween 20 (TBS), washed 3 times with TBS, 0.5% milk and incubated overnight at 4°C with sera diluted in TBS, 0.5% milk. Membranes were probed either with human sera (see "Patients" above (dilution 1/8) or with sera of rabbits (dilution 1/25) immunized 4 times with purified and heat-inactivated bacteria (Eurogentec standard protocol). Sera of mice immunized with living bacteria was also used (dilution 1/1000).

After 3 subsequent washes with TBS, 0.5% milk, the membranes were probed with horseradish peroxidase-conjugated goat anti-human IgG (Chemicon,Temecula,CA, 1:500), anti-rabbit IgG (Cell Signaling, Allschwill, Switzerland, 1:1000) or anti-mouse IgG (BioRad, Reinach, Switzerland, 1:3000). Membranes were then washed 3 more times with TBS and immunoreactive spots were detected with a chemiluminescence-based kit (LiteAblot^{™} , Euroclone SpA, Pero, Italy).

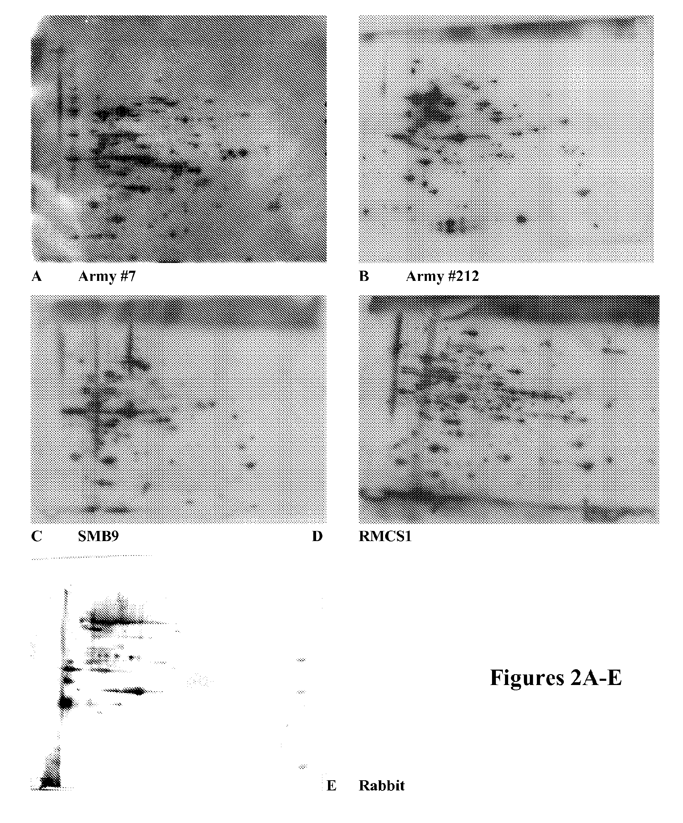

Figure 2 A-D shows the result of an exemplary immunoblot analysis obtained with sera of the patients no. 7 and 212 of the army study, patients SMB9 and RMCS1 of the other study mentioned above. Figure 2E is obtained with the serum of a rabbit.

### Example 4: Selection of immunogenic proteins (antigens)

The Coomassie Blue stained gel and the immunoblots obtained after incubation with *W*. *chondrophila* positive sera or rabbit/mouse anti-*W*. *chondrophila* sera were overlapped using the Adobe Photoshop program to select spots corresponding to immunoreactive proteins.

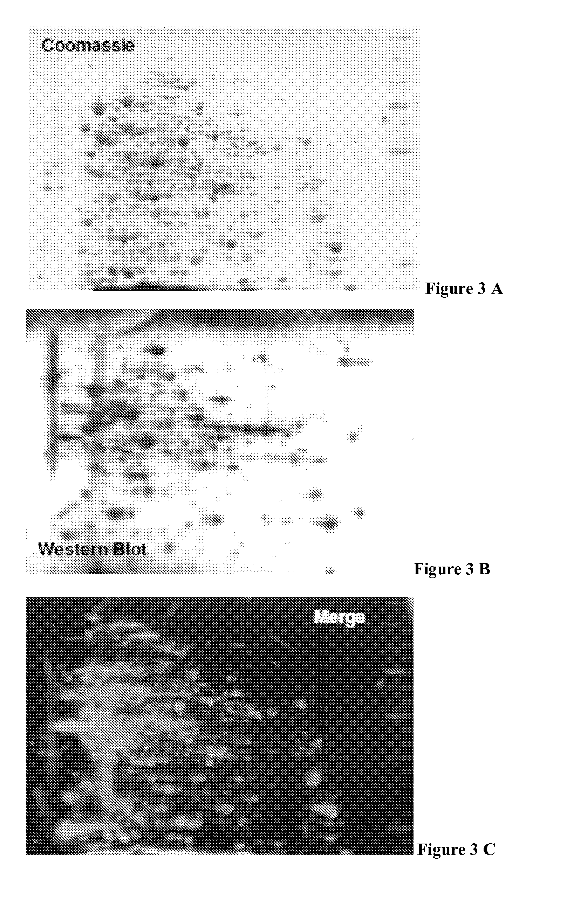

This process is illustrated in Figures 3A-C, with Figure 3A being the 2D PAGE with *Waddlia* Comassie blue stained proteins and Figure 3B is a western blotting obtained with human *Waddlia* positive serum. Figure 3C is the overlap of Figs 3A and B, wherein, in the overlap, the spots obtained with human serum appear in blue.

The overlap procedure allows differentiating spots of interest of the *Waddlia* proteome from non-antigenic spots. The spots of interest (antigen spots) were marked and numerated, as illustrated in Figure 1. By visual inspection of the 60 overlapping protein spots, 17 were found to cause a strong reaction with human serum (strong and clear spot) and thus to be particularly immunogenic. These 17 spots were used for further analysis as described below.

Table 1 below contains the result of the overlap analysis of immunoblotting obtained with serum of different patients and immunized rabbits exposed to the 2D gel of the *W*. *chondrophila* proteome. Each row corresponds to a protein of *Waddlia chondrophila* and is either recognized (filled box) or not (empty box) in Western-blot (2D gels) by the candidate sera represented in the columns. The proteins highlighted in the left column were analyzed by mass spectroscopy (MS). The stars indicate the proteins showing the strongest immunoreactivity.

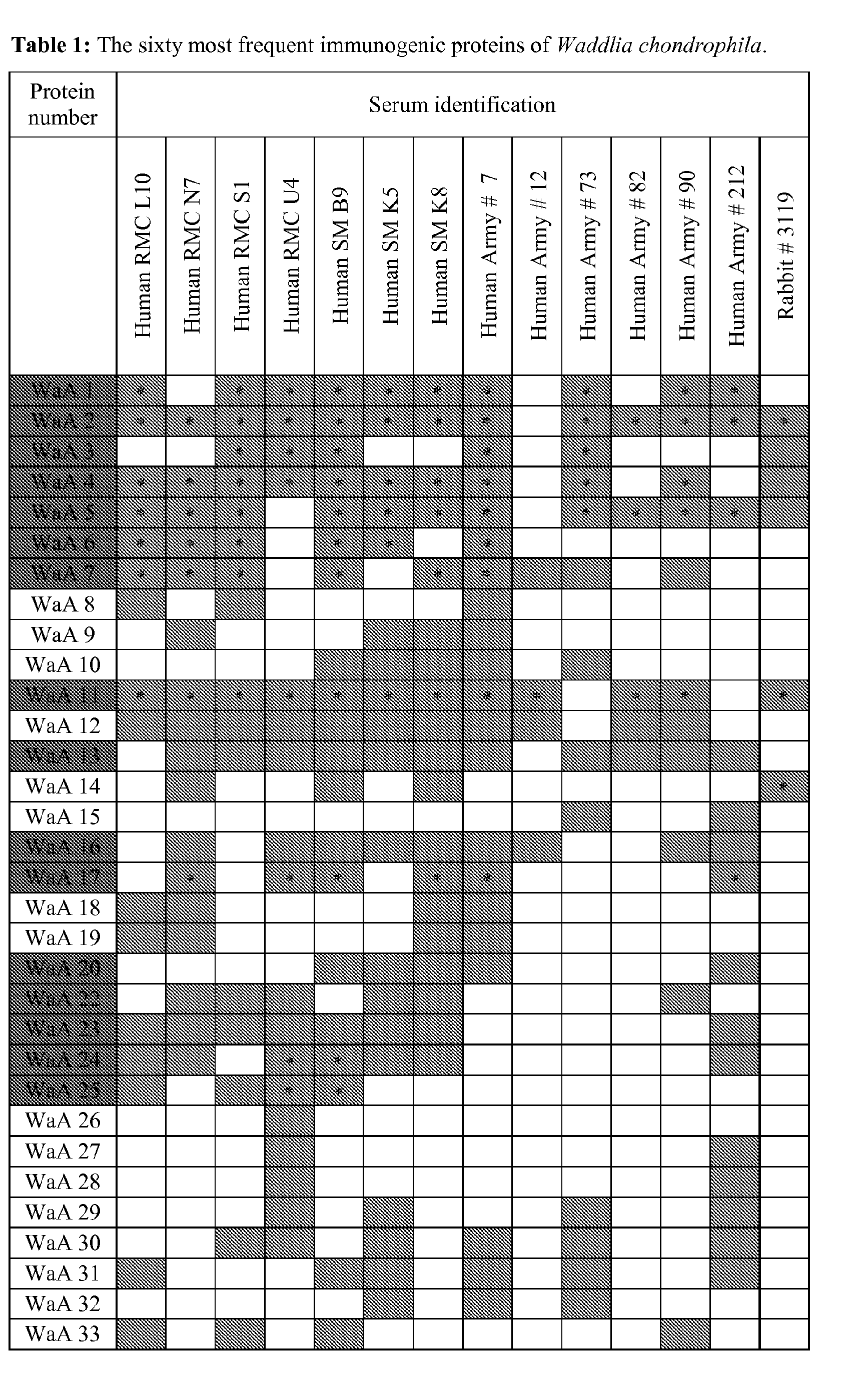

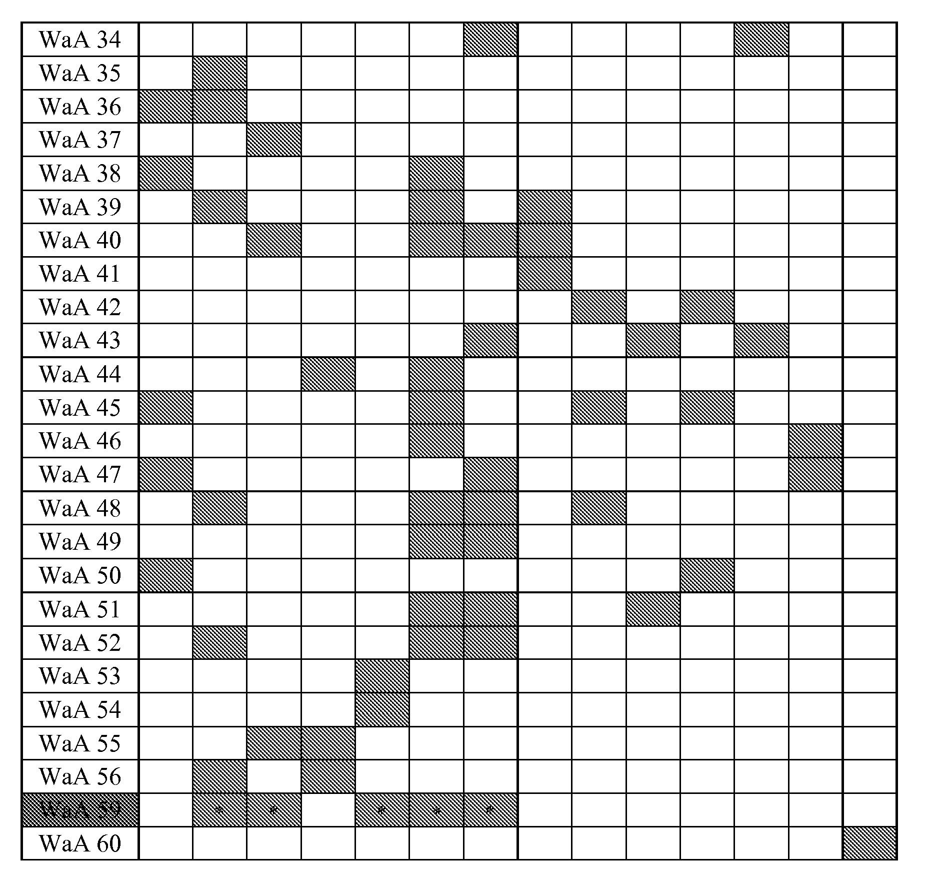

### Example 5: Genome sequencing, assembly and gap closure

Isolation of genomic DNA from *W. chondrophila* was performed with the Wizard Genomic DNA purification kit (Promega Corporation, Madison, USA). DNA was sequenced using both Genome Sequencer FLX^{™} (Droege M, Hill B. 2008. The Genome Sequencer FLX System--longer reads, more applications, straight forward bioinformatics and more complete data sets. J Biotechnol 136: 3-10) by Roche Applied Science (Penzberg, Germany) and Genome Analyzer GAII (Bennett S. 2004. Solexa Ltd. Pharmacogenomics 5: 433-8) by Fasteris (Plan-les-Ouates, Switzerland). GS FLX reads were assembled using Newbler V1.1.02.15 and the obtained 90 large contigs with a 40x coverage served as the basis for the gap closure. To scaffold the contigs, a fosmid library with 40kb DNA inserts was build in the vector pEpiFOS (Epicentre Biotechnologies, Madison, USA) by IIT-Biotech (Bielefeld, Germany). Fosmid walking as well as PCR-based techniques were used to close the gaps and solve ambiguities caused by the high number of repetitive sequences. Primers were designed with Consed (Gordon D, Abajian C, Green P. 1998. Consed: A graphical tool for sequence finishing. Genome Research 8: 195-202) and provided by Eurogentec (Seraing, Belgium). PCRs were performed using ampliTaq Gold (Applied Biosystems, Forster City, USA) according to manufacturer recommendations; Long-Range PCRs were performed with the Expand Long Range dNTPack (Roche Diagnostic, Mannheim, Germany). Sequences were produced using ABI BigDye Terminator 1.1 chemistry on ABI sequencer ABI3130X (Applied Biosystems, Forster City, USA). Sanger reads were mapped onto the contigs with Phrap and visualized with Consed. Solexa reads were then mapped to the final assembly with SOAP (Li R, Li Y, Kristiansen K, Wang J. 2008. SOAP: short oligonucleotide alignment program. Bioinformatics 24: 713-4) to correct homopolymer errors after manual inspection of discrepancies.

Curation and annotation of the genome was performed using the genome annotation system GenDB 2.4 (Meyer F, Goesmann A, McHardy AC, Bartels D, Bekel T, et al. 2003. GenDBan open source genome annotation system for prokaryote genomes. Nucleic Acids Res 31: 2187-95). Prediction of coding sequences (CDS) was accomplished using the softwares Critica (Badger JH, Olsen GJ. 1999. CRITICA: coding region identification tool invoking comparative analysis. Mol Biol Evol 16: 512-24), Glimmer (Delcher AL, Harmon D, Kasif S, White O, Salzberg SL. 1999. Improved microbial gene identification with GLIMMER. Nucleic Acids Res 27: 4636-41) and Reganor (Linke B, McHardy AC, Neuweger H, Krause L, Meyer F. 2006. REGANOR: a gene prediction server for prokaryotic genomes and a database of high quality gene predictions for prokaryotes. Appl Bioinformatics 5: 193-8; McHardy AC, Goesmann A, Puhler A, Meyer F. 2004. Development of joint application strategies for two microbial gene finders. Bioinformatics 20: 1622-31). All predicted ORFs were automatically submitted to similarity searches against nr, Swissprot, KEGG, InterPro, Pfam, TIGRPfam databases as well as the genomes of *Candidatus* Protochlamydia amoebophila UWE25 (NC_005861), *Chlamydia felis* Fe/C56 (NC_007899) and *Chlamydia trachomatis* D/UW-3/CX (NC_000117). Putative signal peptides, transmembrane helices and nucleic acid binding domains were predicted using SignalP (Bendtsen JD, Nielsen H, von Heijne G, Brunak S. 2004. Improved prediction of signal peptides: SignalP 3.0. J Mol Biol 340: 783-95) and TMHMM (Krogh A, Larsson B, von Heijne G, Sonnhammer EL. 2001. Predicting transmembrane protein topology with a hidden Markov model: application to complete genomes. J Mol Biol 305: 567-80) and Helix-Turn-Helix (Dodd IB, Egan JB. 1987. Systematic method for the detection of potential lambdaCro-like DNA-binding regions in proteins. J Mol Biol 194: 557-64) respectively. The automatic annotation of each CDS was manually checked and corrected according to the most congruent tool results.

### Example 6: Protein identification by MALDI-MS/MS

The seventeen (17) antigen spots identified as strongly immunogenic were excised from the 2-D gel and subjected to mass spectrometry (MS) to definitively identify the proteins.

Spots excised from the 2-D gel were transferred to special 96-well plates (Perkin Elmer Life Sciences). In-gel proteolytic cleavage with sequencing-grade trypsin (Promega, Madison, WI, USA) was performed automatically in the robotic workstation Investigator ProGest (Perkin Elmer Life Sciences) according to the protocol of Shevchenko et al. (Shevchenko A, Wilm M, Vorm O, Mann M. Mass spectrometric sequencing of proteins silver-stained polyacrylamide gels. Anal Chem. 1996 Mar 1;68(5):850-8.). Digests were evaporated to dryness and resuspended in 3 ul alpha-cyano-hydroxycinnamic acid matrix (5 mg / ml in 60% (v :v) acetonitrile :water), of which 0.7 µl were deposed in duplicate on a target plate.

MALDI-MS-MS analysis was performed on a 4700 Proteomics Analyser (Applied Biosystems, Framingham, MA, USA). After MALDI-TOF MS analysis, internal calibration on trypsin autolysis peaks and subtraction of matrix peaks, the 10 most intense ion signals were selected for MS/MS analysis. Non-interpreted peptide tandem mass spectra were used for direct interrogation of *W. chondrophila* ORF database using Mascot 2.0 (http://www.matrixscience.com). The mass tolerance for database searches was 50 ppm. MASCOT was set up to only report peptide matches with a score above 14. With the parameters used, the threshold for statistical significance (p<0.05) corresponded to a total (protein) MASCOT score of 17, but we considered only scores greater than 30. Proteins scoring above 80 were considered automatically as valid, while all protein identifications with a total MASCOT score between 30 and 80 were manually validated. Validation included examination of the peptide rms mass error of individual peptide matches. MS/MS Peptide matches were validated only if at least an ion series of 4 consecutive y ions were matched, in addition to ions belonging to other series.

Of the 17 excised spots, spots WaA2 and WaA13, turned out to represent the same protein. The same was the case for the pair of spots WaA4 and WaA7, and WaA23 and WaA25, respectively. One spot, WaA17, could not be identified. Therefore, this analysis of the 17 excised spots resulted in 13 different immunogenic proteins (SEQ. ID. NO.: 1-13), see Table 2 further below.

### Example 7: Selection of preferred proteins by amino acid sequence comparison

The thirteen sequences identified in the previous examples (SEQ. ID. NO.: 1-13) were subjected to sequence identity comparison by blast using the basic protein blast on the internet (http://blast.ncbi.nlm.nih.gov) with preset standard parameters and database selections. This sequence comparison tools is based on algorithms detailed in the two following publications: Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402. Stephen F. Altschul, John C. Wootton, E. Michael Gertz, Richa Agarwala, Aleksandr Morgulis, Alejandro A. Schäffer, and Yi-Kuo Yu (2005) "Protein database searches using compositionally adjusted substitution matrices", FEBS J. 272:5101-5109.

Standard parameters include the selection of blastp (protein-protein BLAST, automatic adjustment of parameters to short input sequences; expect threshold 10, word size 3, use of the matrix BLOSUM62; Gap costs: existence: 11, extension 1; conditional compositional score matrix adjustment, no filters and no masking).

Table 2 below compares the protein sequences of SEQ. ID. NO.: 1-20 with the most similar sequences in related bacterial species (Chlamydia-like) and indicates the sequence identity as determined by Blast.

**Table 2: Sequence comparison of immunogenic proteins SEQ. ID. NO.: 1-20 with most similar sequences of related species.**

| Spot number | SEQ. ID. NO. | ORF | Protein description | % identity | Gene no. and name: |
|---|---|---|---|---|---|
| WaA1 | 1 | 164 | Elongation factor G | 76% | 2780446 fusA |
| WaA2, WaA13 | 2 | 874 | Chaperone protein dnaK | 79% | 2780431 dnaK |
| WaA3 | 3 | 1180 | Hypothetical protein | | no homology |
| WaA4, | 4 | 894 | | | |
| WaA7 | | | Hypothetical protein | 34% | 2781113 pc1399 |
| WaA5 | 5 | 1435 | Elongation factor Tu | 79% | 2780753 tuf |
| WaA6 | 6 | 1515 | Elongation factor Ts | 60% | 2781312 tsf |
| | 7 | 1698 | Transcription elongation protein | | |
| WaA11 | | | nusA | 76% | 2781354 nusA |
| WaA16 | 8 | 1166 | Chaperonin GroEL | 78% | 2780167 groEL |
| | 9 | 1439 | 50S ribosomal | | |
| WaA20 | | | protein L1 | 71% | 2780868 rplA |
| | 10 | 1588 | leucyl | | |
| WaA22 | | | aminopeptidase | 53% | 2780361 pepA |
| | 11 | 865 | Transcription | | |
| WaA23, | | | termination factor | | |
| WaA25 | | | rho | 88% | 2779863 rho |
| | 12 | 1563 | | | |
| | | | 2-oxoglutarate | | |
| | | | dehydrogenase E3 | | |
| WaA24 | | | component | 54% | 5788862 sucC |
| | 13 | 1516 | 30S ribosomal | | |
| WaA59 | | | protein S2 | 80% | 2779568 rpsB |
| | 14 | 1341 | omcB | 54% | 2779865 omcB |
| | 15 | 1571 | putative chlamydial | | |
| | | | protease-like | | |
| | | | activity factor | | |
| | | | (CPAF) | 33% | 2780588 pc0916 |
| | 16 | 1708 | | | |
| | | | putative type III | | |
| | | | secreted protein | | |
| | | | SctW (lcrE) | 26% | 895078 lcrE |
| | 17 | 257 | | | |
| | | | putative 3-deoxy- | | |
| | | | manno-octulosonate | 48% | 2779609 gseA |
| | 18 | 1441 | | | |
| | | | 50S ribosomal | | |
| | | | protein L7/L12 | 67% | 2781036 rplL |
| | 19 | 1697 | | | |
| | | | 30S ribosomal | | |
| | | | protein S1 | 78% | 2780143 rpsA |
| | 20 | 369 | DO serine protease | 51% | 2779691 htrA |
| | 21 | 128 | pmpD? | | |
| | 22 | 1449 | Member of putative | | |
| | | | extended omcA and | | |
| | | | omcB family | | |
| | 23 | 1451 | Member of putative | | |
| | | | extended omcA and | | |
| | | | omcB family | | |
| | 24 | 1452 | Member of putative | | |
| | | | extended omcA and | | |
| | | | omcB family | | |
| | 25 | 1453 | Member of putative | | |
| | | | extended omcA and | | |
| | | | omcB family | | |
| | 26 | 512 | ompA family | | |
| | 27 | 513 | ompA family | | |
| | 28 | 603 | ompA family | | |
| | 29 | 607 | ompA family | | |
| | 30 | 608 | ompA family | | |
| | 31 | 609 | ompA family | | |
| | 32 | 1192 | ompA family | | |
| | 33 | 1234 | ompA family | | |
| | 34 | 1235 | ompA family | | |
| | 35 | 1622 | ompA family | | |
| | 36 | 1623 | ompA family | | |

Most of the indicated compared genes listed in the last column are from the organism Candidatus *Protochlamydia amoebophila* UWE25, with the exception of SEQ. ID. NO.: 12, which is compared to 5788862 sucC of *Gluconacetobacter diazotrophicus* PAl 5, and SEQ. ID. NO.: 16 *Chlamydophila pneumoniae* CWL029.

### Example 8: Cloning of selected immunogenic proteins of low probability of cross-reaction

From Table 2, the ORFs of the sequences sharing 55% or less sequence identity with closely related species of Chlamydia-like organisms were selected for cloning and recombinant expression.

Accordingly, the ORFs encoding the proteins WaA3, WaA4, WaA22, WaA24 were amplified by PCR using *W. chondrophila* DNA as template. The following primers were used:
WaA3 forward (SEQ. ID. NO: 37):
5'-AAAAAACCATGGAAACTAGTACAGGGGGAATACCTC-3'
WaA3 reverse (SEQ. ID. NO: 38):
5'-GATGTCGACACTTTGTCGATCAACCACTGAAA-3'
WaA4 forward (SEQ. ID. NO: 39):
5'-AAAAAACCATGGGTACAGACAGAACGC AAAACCT-3'
WaA4 reverse (SEQ. ID. NO: 40): 5'-AAAAAACTCGAGAGTTGTTTCTTCTTCCTGCTCCTGTTG-3'
WaA22 forward (SEQ. ID. NO: 41): 5'-AAAAAACCATGGGTAAATTTTCAACAGTTGCTTCTC-3'
WaA22 reverse (SEQ. ID. NO: 42):
5'-AAAAAACTCGAGTAGATGGTTTTCTAAAAACTCCATCATC-3'
WaA24 forward (SEQ. ID. NO: 43):
5'-AAAAAACCATGG AAAAGAGGCAGTTTCTTTCC-3'
WaA24 reverse (SEQ. ID. NO: 44): 5'-AAAAAACTCGAGTAAGTGAATGGGTTTTTC-3'

PCR products were cloned into the pET28 vector (Novagen EMD Chemicals Inc, San Diego, USA).This vector system allows the protein of interest to be expressed as a fusion protein with a 6 His tail fused to its C-terminus.

### Example 8: Expression and purification of selected proteins

Protein expression in *E.coli* DE-3 is induced with 500µM isopropyl-β-D-thiogalactopyranoside (IPTG, Qbiogen, Basel, Switzerland), during 2 hours at 37°C. The *W*. *chondrophila* proteins can be used as antigens in detection or diagnostic assays either as unpurified *E. coli* lysate or, for increased sensitivity and reduced background, as a purified product.

For purification, the bacterial pellet is resuspended in lysis buffer (100 mM NaH₂PO4 H₂O, 10 mM Tris, 8M urea, pH 8.0 (denaturing conditions) or 50 mM NaH₂PO4 H₂O, 300 mM NaCl and 10 mM imidazole, pH 8.0 (non denaturing conditions)) containing 1 mg/ml lysozyme, 1x Halt Protease Inhibitor (Pierce Biotechnology Inc, Rockford, USA), 5 µg/ml DNAseI and 8 µg/ml RNAseA and lysed by short pulses of sonication on ice. Protein samples are applied on a Ni-NTA column (HIS-Select^{™} Spin Columns, Sigma-Aldrich, Missouri, USA). The column is washed 2 times with washing buffer (100 mM NaH₂PO4 H₂O, 10 mM Tris, 8M urea, pH 6.3 (denaturing conditions) or 50 mM NaH₂PO4 H₂O, 300 mM NaCl and 20 mM imidazole, pH 8.0 (non denaturing conditions)). And finally, the protein of interest is eluted from the column either by decreasing the pH of the solution to pH 5.9 and then to pH 4.5 (denaturing conditions) or by adding 250 mM imidazole to the last solution (non denaturing conditions). The purest protein fractions are pooled and if necessary (denaturing conditions) dialysed to remove urea against 1× PBS containing 4M, 2M, and no urea at 4° C. Some recombinant proteins precipitate when urea concentration decreases. In these cases, urea concentration is kept high enough to ensure solubility of the protein (usually 2M).

The purified recombinant proteins are submitted to SDS PAGE and transferred to nitrocellulose membrane before probing with relevant mouse, rabbit or human sera or are used directly as antigens in serological tests.

### Example 9: Enzyme-Linked Immunsorbent Assay (ELISA)

96 wells ELISA plates are coated with any one or a combination of several purified recombinant proteins obtained in the previous example, diluted in carbonate/bicarbonate buffer and blocked with 1% non-fat dry-milk in PBS, 0.1 % Tween 20. After one washing step with PBS 0.1% Tween 20, the plate is incubated with human sera diluted 1:8 (in PBS, 0.1% Tween 20, 1% non-fat dry-milk), washed 5 times with PBS 0.1 % Tween 20 and incubated with horseradish peroxidase-conjugated goat anti-human IgG (Chemicon,Temecula, CA) diluted 1:5000 (in PBS, 0.1% Tween 20, 1 % non-fat dry-milk). After 5 washing steps with PBS 0.1% Tween 20, the plate is incubated with OPD (O_Phenylendiamine dihydrochloride, 1 mg/ml in citrate buffer) and the absorbance measured at 490 nm.

## Claims

1. Use of at least one recombinant, isolated and/or synthetic antigenic peptide in the diagnosis of an infection by an intracellular microorganism belonging to the *Waddliaceae* and/or in a vaccine against an intracellular microorganism of the *Waddliaceae.*

2. The use of claim 1 in the diagnosis of an infection by a bacterial strain of the genus *Waddlia* and/or in a vaccine against a bacterial strain of the genus *Waddlia.*

3. The use of any one of the claims 1-2, for diagnosis in a serological test and/or in an antibody-based test.

4. The use of any one of the preceding claims, wherein the peptide comprises an amino acid sequence selected from:
(a) a continuous stretch of at least 10 amino acid residues selected within any one of the amino acid reference sequences SEQ. ID. NO. 1-36;
(b) an amino acid sequence having at least 30% or more sequence identity with any one of the amino acid sequences defined under (a);
(c) a variant sequence of (a) and (b).

5. The use of claim 4, wherein the continuous stretch spans at least 50 amino acids.

6. The use of any one of claim 4 and 5, wherein the amino acid sequence of (b) has 70-100% sequence identity with any one of SEQ. ID. NO.: 1-36.

7. The use of any one of claims 4-6, wherein the reference sequence is selected from one or a combination of several of the following groups: the group of SEQ. ID. NO.: 1-13; the group of SEQ. ID. NO.: 3, 4, 10 and 12; the group of SEQ. ID. NO.: 14-20; the group of SEQ. ID. NO.: 21-25; the group of SEQ. ID. NO.: 26-36.

8. The use of any one of the preceding claims, wherein at least two different immunogenic peptides are used.

9. A recombinant or synthetic, isolated or non-isolated peptide comprising an amino acid sequence selected from:
(a) a continuous stretch of at least 10 amino acid residues selected within any one of the amino acid reference sequences SEQ. ID. NO. 1-36;
(b) an amino acid sequence having at least 70% or more sequence identity with a any one of the amino acid sequences as defined under (a);
(c) a variant sequence of (a) and (b).

10. The peptide of claim 9, wherein said amino acid sequence has antigenic and/or immunogenic properties.

11. A vaccine composition comprising one or more isolated and/or recombinant immunogenic peptides of an intracellular bacterial strain of the family *Waddliaceae.*

12. The vaccine composition of claim 11, which is used for treating and/or preventing one or more conditions selected from the group of lower and/or upper respiratory tract infections, including infections of the nasal concha, bronchitis, bronchiolitis and/or pneumonia, conjunctivitis, uveitis, infections of the urogential tract, kidney infection, pericarditis, infertility, abortion and pre-term labour in animals and miscarriage and pre-term labour in humans.

13. A method of producing an antibody, the method comprising the steps of:
(1) exposing a mammal to the isolated and/or recombinant peptide as defined in any one of claims 1-7 and/or 9-10; and,
(2) harvesting said antibody from serum of said mammal.

14. The use of a monoclonal and/or polyclonal antibody specifically binding to a peptide as defined in any one of claims 1-7 and/or 9-10 in the diagnosis of infection by a microorganism belonging to the *Waddliaceae.*

15. A test kit of diagnosis comprising one or more isolated and/or recombinant antigenic peptides of an intracellular bacterial strain of the family *Waddliaceae* and/or an antibody specifically binding to said isolated and/or recombinant antigenic peptides.


SEQUENCE LISTING

<110>  University of Lausanne

<120>  Antigenic Peptides of Chlamydia-related Bacteria and Tests of
       Diagnosis

<130>  PAC-7P-EP

<160>  44

<170>  PatentIn version 3.3

<210>  1
<211>  695
<212>  PRT
<213>  Waddlia chondrophila

<400>  1

Met Pro Arg Ser Glu Gln Asp Lys Leu Asp Lys Val Arg Asn Ile Gly
1               5                   10                  15

Ile Met Ala His Ile Asp Ala Gly Lys Thr Thr Val Thr Glu Arg Ile
            20                  25                  30

Leu Phe Phe Thr Gly Arg Thr His Arg Ile Gly Glu Thr His Asp Gly
        35                  40                  45

Thr Ala Thr Met Asp Trp Met Glu Gln Glu Gln Glu Arg Gly Ile Thr
    50                  55                  60

Ile Thr Ser Ala Ala Thr Thr Val His Trp Asn Gly His Pro Ile His
65                  70                  75                  80

Val Ile Asp Thr Pro Gly His Val Asp Phe Thr Val Glu Val Glu Arg
                85                  90                  95

Ser Leu Arg Val Leu Asp Gly Ala Val Ala Val Phe Asp Ala Val Ser
            100                 105                 110

Gly Val Glu Pro Gln Ser Glu Thr Val Trp Arg Gln Ala Glu Lys Tyr
        115                 120                 125

Gln Val Pro Arg Ile Ala Phe Ile Asn Lys Met Asp Arg Met Gly Ala
    130                 135                 140

Asp Phe Phe Asp Ala Ile Glu Ser Met Lys Glu Lys Leu His Ala Asn
145                 150                 155                 160

Ala Val Pro Val Gln Ile Pro Ile Gly Ala Glu Ala Asp Tyr Lys Gly
                165                 170                 175

Leu Ile Asp Leu Ile Thr Met Lys Ala Ile Val Phe Lys Asp Glu Thr
            180                 185                 190

Gln Gly Ile Glu Trp Asp Glu Ile Asp Ile Pro Ala Glu Tyr Leu Glu
        195                 200                 205

Lys Ala Asn Glu Leu Arg Ala Gln Leu Leu Asp Glu Leu Ala Val Ile
    210                 215                 220

Asp Glu Glu Asn Glu Thr Phe Met Ala Lys Val Leu Asp Asp Pro Asp
225                 230                 235                 240

Ser Leu Thr Pro Asp Glu Ile His Gln Val Ile Arg Lys Gly Val Cys
                245                 250                 255

Glu Asn Leu Phe Asn Pro Val Leu Cys Gly Ser Ala Leu Lys Asn Lys
            260                 265                 270

Gly Val Gln Gln Leu Leu Asn Ala Ile Ile Lys Trp Met Pro Ser Pro
        275                 280                 285

Ile Asp Arg Gly Ala Val Lys Ala His Leu Pro Asp Ser Glu Asp Glu
    290                 295                 300

Ile Leu Leu Glu Pro Ser Asp Asp Ala Pro Leu Gly Ala Leu Ala Phe
305                 310                 315                 320

Lys Val Met Thr Asp Pro Tyr Val Gly Arg Leu Thr Phe Val Arg Ile
                325                 330                 335

Tyr Ser Gly Thr Leu Thr Lys Gly Met Asn Leu Leu Asn Ser Thr Lys
            340                 345                 350

Asp Lys Lys Glu Arg Ile Ser Arg Leu Ile Glu Met His Ala Asn Thr
        355                 360                 365

Arg Glu Glu Arg Asp Glu Phe Phe Thr Gly Asp Ile Ala Ala Cys Ile
    370                 375                 380

Gly Leu Lys Asn Thr Thr Thr Gly Asp Ser Leu Cys Ser Pro Asp Lys
385                 390                 395                 400

Pro Tyr Ile Leu Glu Lys Met Glu Phe Pro Glu Pro Val Ile Ser Met
                405                 410                 415

Ala Ile Glu Pro Lys Ser Lys Ala Asp Arg Glu Lys Leu Ser Gln Ala
            420                 425                 430

Leu Ser Ala Leu Ser Glu Glu Asp Pro Thr Phe Arg Val Ser Thr Asp
        435                 440                 445

Glu Glu Thr Gly Gln Thr Ile Ile Ala Gly Met Gly Glu Leu His Leu
    450                 455                 460

Asp Val Leu His Asp Arg Met Lys Arg Glu Phe Asn Val Glu Ala Asn
465                 470                 475                 480

Val Gly Lys Pro Gln Val Ser Tyr Arg Glu Thr Ile Thr Ile Pro Gly
                485                 490                 495

Gly Thr Asp Thr Lys Phe Val Lys Gln Thr Gly Gly Arg Gly Gln Tyr
            500                 505                 510

Ala His Val Val Leu Glu Ile Glu Pro Asn Glu Lys Gly Lys Gly Asn
        515                 520                 525

Glu Val Glu Ser Lys Ile Val Gly Gly Val Ile Pro Arg Glu Tyr Ile
    530                 535                 540

Pro Ala Val Ile Lys Gly Val Glu Glu Gly Leu Leu Thr Gly Val Leu
545                 550                 555                 560

Ala Gly Tyr Asn Leu Val Asp Val Lys Val Arg Ile Val Phe Gly Ser
                565                 570                 575

Tyr His Glu Val Asp Ser Asn Glu Met Ala Phe Lys Ile Cys Gly Ser
            580                 585                 590

Met Ala Val Lys Glu Ala Ala Arg Lys Cys Lys Pro Ile Leu Leu Glu
        595                 600                 605

Pro Ile Met Lys Val Gln Val Thr Thr Pro Glu Thr Ser Met Gly Asp
    610                 615                 620

Val Ile Gly Asp Leu Asn Arg Arg Arg Gly Gln Ile Met Gly Gln Glu
625                 630                 635                 640

Thr Val Lys Gly Gly Ala Ile Val Asn Ala Glu Val Pro Leu Ser Glu
                645                 650                 655

Met Phe Gly Tyr Ser Thr Gln Leu Arg Ser Leu Thr Ser Gly Arg Ala
            660                 665                 670

Asn Tyr Ser Met Glu Pro Ser His Phe Ala Pro Val Pro Thr Lys Ile
        675                 680                 685

Ala Glu Glu Ile Met Lys Lys
    690                 695

<210>  2
<211>  644
<212>  PRT
<213>  Waddlia chondrophila

<400>  2

Met Thr Glu Lys Lys Lys Ser Gln Lys Ile Ile Gly Ile Asp Leu Gly

1                   5                        10                       15

Thr Thr Asn Ser Cys Val Ala Val Met Glu Gly Gly Ser Pro Lys Val
                20                       25                       30

Ile Ala Ser Ala Glu Gly Ser Arg Thr Thr Pro Ser Val Val Ala Phe
        35                       40                       45

Lys Gly Asp Glu Arg Leu Val Gly Ile Pro Ala Lys Arg Gln Ala Val
    50                       55                       60

Thr Asn Pro Gln Asn Thr Ile Ser Ser Ala Lys Arg Phe Ile Gly Arg
65                       70                       75                       80

Lys Tyr Glu Glu Val Ser Ser Glu Ile Gln Thr Val Pro Tyr Thr Val
                    85                       90                       95

Thr Ser Asn Asp Asn Gly Asp Ala Val Phe Glu Val His Asp Lys Val
                100                      105                      110

Phe Thr Pro Glu Glu Ile Ser Ala Gln Ile Leu Ile Lys Met Lys Glu
        115                      120                      125

Thr Ala Glu Ala Tyr Leu Gly Glu Glu Ile Thr Gln Ala Val Ile Thr
    130                      135                      140

Val Pro Ala Tyr Phe Asn Asp Ser Gln Arg Gln Ser Thr Lys Asp Ala
145                      150                      155                      160

Gly Arg Ile Ala Gly Leu Asp Val Lys Arg Ile Ile Pro Glu Pro Thr
                    165                      170                      175

Ala Ala Ala Leu Ala Tyr Gly Met Asp Lys Glu His Ser Asp Ala Lys
                180                      185                      190

Ile Ala Val Phe Asp Leu Gly Gly Gly Thr Phe Asp Ile Ser Ile Leu
        195                      200                      205

Glu Ile Gly Asp Gly Val Phe Glu Val Leu Ala Thr Asn Gly Asp Met
    210                      215                      220

His Leu Gly Gly Asp Asp Phe Asp Asn Ala Ile Leu Asn Trp Met Leu
225                      230                      235                      240

Glu Glu Phe Lys Lys Glu Asn Gly Leu Asp Leu Ser Ser Asp Lys Met
                    245                      250                      255

Ala Leu Gln Arg Leu Arg Asp Ala Ala Glu Lys Ala Lys Ile Glu Leu
                260                      265                      270

Ser Gly Thr Gln Thr Thr Glu Ile Asn Gln Pro Phe Ile Thr Met Asp

275                 280                 285

Ala Ser Gly Pro Lys His Leu Ala Leu Thr Leu Thr Arg Ser Lys Leu
    290                 295                 300

Glu Ser Leu Val Glu Asp Leu Val Gln Arg Thr Lys Gly Pro Cys Leu
305                 310                 315                 320

Lys Ala Leu Glu Asp Ala Gly Val Asn Gln Asn Glu Ile Ser Glu Val
                325                 330                 335

Ile Leu Val Gly Gly Met Ser Arg Met Pro Ala Val Gln Gln Met Val
            340                 345                 350

Lys Ser Ile Phe Gly Lys Glu Gly His Lys Gly Val Asn Pro Asp Glu
        355                 360                 365

Val Val Ala Val Gly Ala Ala Ile Gln Gly Gly Val Leu Thr Gly Val
    370                 375                 380

Val Lys Asp Val Leu Leu Leu Asp Val Ile Pro Leu Thr Leu Gly Ile
385                 390                 395                 400

Glu Thr Leu Gly Gly Val Met Thr Pro Leu Val Glu Arg Asn Thr Thr
                405                 410                 415

Ile Pro Thr Gln Lys Lys Gln Ile Phe Ser Thr Ala Ala Asp Asn Gln
            420                 425                 430

Pro Ala Val Thr Ile Arg Val Leu Gln Gly Glu Arg Lys Met Ala Asn
        435                 440                 445

Asp Asn Lys Glu Ile Gly Arg Phe Asp Leu Thr Asp Ile Pro Pro Ala
    450                 455                 460

Pro Arg Gly Thr Pro Gln Ile Glu Val Ser Phe Glu Ile Asp Ala Asp
465                 470                 475                 480

Gly Ile Leu His Val Ser Ala Lys Asp Met Ala Ser Gly Lys Glu Gln
                485                 490                 495

Lys Ile Arg Ile Glu Ala Ser Ser Gly Leu Lys Glu Glu Asp Ile Glu
            500                 505                 510

Arg Met Val Lys Asp Ala Glu Leu His Lys Glu Glu Asp Ala Lys Arg
        515                 520                 525

Arg Gln Gln Val Glu Thr Arg Asn Glu Ala Asp Ser Leu Ala Phe Arg
    530                 535                 540

Ala Gln Lys Ala Leu Asp Glu Tyr Lys Asp Lys Leu Pro Glu Glu Val

545                 550                 555                 560

Arg Asn Asp Val Gln Ser His Ile Asp Ala Leu Lys Lys Ala Leu Glu
                565                 570                 575

Gly Asp Asp Leu Ala Lys Ile Lys Ser Ala Lys Glu Ala Leu Glu Ser
            580                 585                 590

His Met Gln Lys Ile Gly Glu Val Met Ala Gln Ala Gln Gln Ala Ala
        595                 600                 605

Gly Gly Ala Gly His Thr Pro Asn Ser Gln Glu Asn Pro Gln Ala Asn
    610                 615                 620

Ala Ser Ser Asp Glu Asn Ile Glu Glu Ala Glu Val Glu Ile Ile Asp
625                 630                 635                 640

Asp Glu Asn Lys

<210>  3
<211>  461
<212>  PRT
<213>  Waddlia chondrophila

<400>  3

Met Thr Ser Thr Gly Gly Ile Pro Pro Asp Gly Gly Ser Ile His Trp
1               5                   10                  15

Gln Ser Ser Ser Thr Ser Gly Ser Asp Glu Thr Glu Arg Thr Thr Arg
            20                  25                  30

Ala Ala Thr Phe Lys Ala Asp Gly Lys Glu Tyr Thr Val Lys Val Ser
        35                  40                  45

Tyr Ser Arg Gln Ala Val Lys Glu Lys Tyr Gly Ile Ser Asp Asp Ala
    50                  55                  60

Val Asp Ala Thr Met Glu Glu Val Ile Ser Lys Ile Gly Val Glu Gln
65                  70                  75                  80

Leu Lys Asn Leu Ala Gly His Ser Ile Ser Ala Ser Leu Arg Gly Lys
                85                  90                  95

Gly Gly Glu Gln Ile Gln Tyr Lys Ala Asn Lys Asp Ser Asn Tyr Gln
            100                 105                 110

Ile Phe Ser Asp Ala Leu Lys Thr Lys Asn Leu Pro Leu Phe Gln Thr
        115                 120                 125

Ile Ser Thr Val Arg Thr Ile Phe Gln Leu Asn Thr Gly Ala Leu Lys
    130                 135                 140

Arg Val Pro Ser Ser Ser Gly Glu Val Glu Leu Asp Ile Lys Glu Glu
145                 150                 155                 160

Thr Glu Lys Leu Arg Asp Ser Ser Ile Arg Phe Lys Glu Val Pro Asn
                165                 170                 175

Glu Val Phe Ala Phe Ile Glu His Leu Ser Ser Thr Asn Pro Ile Ala
            180                 185                 190

Ser Asp Leu Glu Lys Ile Lys Lys Ile Ile Glu Ala Asp Ser Ala Asp
        195                 200                 205

Ser Ala Asp Glu Val Lys Lys Ile Leu Gln Asp Pro Lys Leu Asn Leu
    210                 215                 220

Thr Lys Glu Gln Gln Asn Glu Val Leu Lys Gln Leu Lys Asp Val Asp
225                 230                 235                 240

Phe Ala Asp Glu Ile Arg Val Ala Ile Gly Leu Gln Lys Glu Gly Asp
                245                 250                 255

Val Phe Leu Asp Val Ser Gln Gly Gly Ile Gln Ile Glu Asn Gln Pro
            260                 265                 270

Glu Glu Val Gly Lys Met Ala Cys Ala Ser Ile Cys Gly Gln Ala Ile
        275                 280                 285

Leu Trp Met Glu Ser Asn Asn Glu Tyr Leu Val Thr Arg Glu Asp Phe
    290                 295                 300

Glu Gln Val Ile Lys Asp Gly Ile Asp Arg His Val Gly Lys Glu Arg
305                 310                 315                 320

Val Glu Asn Pro Pro Glu Gly Lys Glu Phe Asn Gly Val Phe Lys Gly
                325                 330                 335

Ile Ser Glu Asp Phe Glu Ser Glu Asp Tyr Lys Phe Glu His Ser Phe
            340                 345                 350

Ile Thr Glu His Phe Asp Asp Glu Val Glu Leu Met Thr Ser Ser Asp
        355                 360                 365

Tyr Leu Val Val Thr Gly Tyr Ser Ser Ala Trp Gly Glu Glu Leu Gly
    370                 375                 380

Ser Ile Leu Leu Phe Lys Val Asp Asp Asp Glu Ser Ser Ala Gln Trp
385                 390                 395                 400

Gly Val Met Asp Ser His Gly Leu Thr Glu Asn Lys Asp Thr Lys Asp
                405                 410                 415

Glu Ile Thr Arg Gly Ala Ser Leu Arg Phe Phe Asp Thr Ala Gln Lys
            420                 425                 430

Ala Leu Asp Tyr Val Lys Gly His Val Lys Met Gly Glu Glu Asp Ser
        435                 440                 445

Ala Arg Val Asp Lys Val Ser Val Val Asp Arg Gln Ser
    450                 455                 460

<210>  4
<211>  372
<212>  PRT
<213>  Waddlia chondrophila

<400>  4

Met Thr Asp Arg Thr Gln Asn Leu Ser Phe Asn Pro Asn Lys Pro Glu
1               5                   10                  15

Asp Leu Lys Thr Glu Lys Ser Thr Pro Thr Pro Arg Lys Asp Lys Lys
            20                  25                  30

Asp Phe Lys Lys Ile Phe Gly Glu Glu Glu Gln Glu Gly Gln Gly Arg
        35                  40                  45

Pro Lys Asn Arg Glu Phe Ser Gly Lys Pro Glu Thr Lys Asp Ser Asp
    50                  55                  60

Ser Ala Tyr Glu Asp Val Glu Leu Lys Leu Glu Ala Tyr Asp Gln Lys
65                  70                  75                  80

Pro Lys Thr Lys Gly Leu Ser Leu Phe Asp Ile Ala Ser Thr Pro Val
                85                  90                  95

Glu Lys Glu Glu Ala Val Glu Ser Gln Ala Ser Glu Gly Leu Pro Leu
            100                 105                 110

Ser Asp Met Pro Glu Asp Leu Gln Lys Glu Ser Leu Ser Ala Leu Phe
        115                 120                 125

Lys Gly Tyr Gly Thr Lys Glu Lys Leu Ala Met Ile Gln Lys Glu Val
    130                 135                 140

Lys Glu Leu Pro Asn Gln Pro Val Asp Val Gln Ile Glu Ala Leu Lys
145                 150                 155                 160

Lys Lys Glu Thr Leu Val Glu Gly Leu Gly Gly Asn Arg Thr Ile Ser
                165                 170                 175

Ala Leu Ser Thr Ala Asn Thr Pro Gln Asp Asp Leu Gln Lys Pro Leu
            180                 185                 190

Glu Lys Ala Pro Glu Asn Arg Glu Lys Thr Asp Ala Phe Pro Arg Glu
        195                 200                 205

Gln Val Asp Leu Ala Ala Ile Asn Pro Ala Ala Gly Thr Leu Ala Thr
    210                 215                 220

Ala Pro Val Ala Gln Thr Gln Lys Val Glu Pro Pro Pro Ile Arg Ala
225                 230                 235                 240

Gln Glu Leu Gln Glu Val Val Asp Gln Ile Val Ser Lys Leu Tyr Thr
                245                 250                 255

Leu Ser Ser Glu Gly Lys Thr Asp Thr Leu Ile Gln Leu Lys His Pro
            260                 265                 270

Pro Leu Phe Glu Gly Ser Ser Val Val Ile Arg Ser Phe Asp Ser Ala
        275                 280                 285

Lys Gly Glu Phe Asn Ile Thr Phe Glu Asn Leu Thr Gln Ala Ala Lys
    290                 295                 300

Gln Met Leu Asp Met Gln Glu Asn Gln Asn Ser Leu Arg Leu Ala Leu
305                 310                 315                 320

Glu Gln Lys Gly Tyr Thr Val His Ile Leu Thr Ala Thr Thr Leu Ser
                325                 330                 335

Glu Thr Thr Gln Ile Val Glu Ser Gln Asp Asp Gly Arg Glu Arg His
            340                 345                 350

Glu Asn Asn Asp Glu Ser Ser Arg Glu Arg Gln Gln Gln Glu Gln Glu
        355                 360                 365

Glu Glu Thr Thr
    370

<210>  5
<211>  394
<212>  PRT
<213>  Waddlia chondrophila

<400>  5

Met Ala Lys Glu Thr Phe Gln Arg Asn Lys Pro His Val Asn Ile Gly
1               5                   10                  15

Thr Ile Gly His Val Asp His Gly Lys Thr Thr Leu Thr Ala Ala Ile
            20                  25                  30

Thr Asn Val Leu Ala Lys Lys Met Gly Gly Lys Ala Arg Ser Phe Glu
        35                  40                  45

Glu Ile Asp Asn Thr Pro Glu Glu Lys Ala Arg Gly Ile Thr Ile Asn
    50                  55                  60

Ser Ser His Val Glu Tyr Glu Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Ala Ile Leu Val Val Ala Ala Thr Asp Gly Ala
            100                 105                 110

Met Pro Gln Thr Arg Glu His Ile Leu Leu Ala His Gln Met Gln Val
        115                 120                 125

Pro Lys Ile Val Val Phe Leu Asn Lys Cys Asp Met Leu Gly Glu Gly
    130                 135                 140

Asp Gln Glu Leu Leu Asp Leu Val Glu Leu Glu Leu Gln Glu Leu Leu
145                 150                 155                 160

Glu Ala Lys Gly Tyr Glu Asn Ala Pro Ile Val Arg Gly Ser Ala Leu
                165                 170                 175

Lys Ala Leu Glu Gly Asp Ala Glu Trp Glu Glu Lys Ile Leu Glu Leu
            180                 185                 190

Met Ser Thr Val Asp Asp Asn Val Pro Glu Pro Gln Arg Asp Val Asp
        195                 200                 205

Lys Pro Phe Leu Met Pro Val Glu Asp Val Phe Ser Ile Ser Gly Arg
    210                 215                 220

Gly Thr Val Ala Thr Gly Arg Val Glu Arg Gly Val Ile Lys Leu Asn
225                 230                 235                 240

Asp Lys Val Glu Ile Val Arg Phe Gly Asp Lys Lys Glu Ser Val Ala
                245                 250                 255

Thr Gly Leu Glu Met Phe Asn Lys Leu Leu Asp Glu Ala Arg Ala Gly
            260                 265                 270

Glu Asn Val Gly Val Leu Leu Arg Gly Val Asn Lys Asp Glu Ile Gln
        275                 280                 285

Arg Gly Gln Ile Leu Ala Ala Pro Gly Ser Cys Lys Pro His Lys Lys
    290                 295                 300

Phe Lys Gly Pro Val Tyr Val Leu Thr Lys Asp Glu Gly Gly Arg His
305                 310                 315                 320

Lys Pro Phe Phe Thr Gly Tyr Arg Pro Gln Ile Tyr Ile Arg Thr Thr
                325                 330                 335

Asp Val Thr Gly Thr Val Lys Leu Pro Glu Gly Val Glu Met Val Met
            340                 345                 350

Pro Gly Asp Asn Val Glu Ile Glu Val Glu Leu Ile Tyr Pro Val Ala
        355                 360                 365

Leu Glu Lys Gly Met Arg Phe Ala Ile Arg Glu Gly Gly Arg Thr Ile
    370                 375                 380

Gly Ala Gly Thr Val Ser Glu Ile Ile Glu
385                 390

<210>  6
<211>  278
<212>  PRT
<213>  Waddlia chondrophila

<400>  6

Met Thr Ile Thr Pro Gln Leu Ile Lys Glu Leu Arg Asp Arg Thr Gly
1               5                   10                  15

Val Gly Ile Gly Lys Cys Lys Glu Ala Leu Glu Glu Ala Asn Gly Asp
            20                  25                  30

Leu Glu Ile Ala Ile Glu Asn Leu Arg Lys Ser Gly Met Ala Ser Ala
        35                  40                  45

Val Lys Lys Glu Gly Arg Ala Ala Asn Glu Gly Met Ile Ala Ile Ala
    50                  55                  60

Glu Ser Gly Ser Arg Val Ala Leu Val Glu Ile Asn Ala Glu Thr Asp
65                  70                  75                  80

Phe Val Val Lys Asn Glu Arg Phe Gln Gln Phe Leu Gln Glu Val Ala
                85                  90                  95

Gln Glu Ile Val Glu Thr Asn Pro Val Ser Leu Glu Asp Phe Leu Ala
            100                 105                 110

Gln Lys Phe Ser Lys Asp Gln Asn Met Thr Ile Asp Glu Tyr Arg Ala
        115                 120                 125

Thr Ile Val Gln Ala Ile Gly Glu Asn Ile Gln Val Arg Arg Leu Lys
    130                 135                 140

Val Phe Glu Lys Ser Ser Asp Ser Ser Leu Gly Val Tyr Ser His Leu
145                 150                 155                 160

Gly Gly Lys Ile Val Thr Met Val Lys Ile Asp Gly Ser Gly Asp Ala

                165                 170                 175

Glu Asn Leu Ala Lys Glu Ile Ala Met His Val Ala Ala Ala His Pro
            180                 185                 190

Glu Tyr Ile Arg Pro Glu Asp Val Pro Ser Lys Val Ile Asp Gln Glu
        195                 200                 205

Lys Glu Ile Ala Arg Thr Gln Met Ala Gly Lys Pro Asp His Val Ile
    210                 215                 220

Glu Lys Ile Leu Glu Gly Lys Ile Ser Lys Tyr Phe Asp Asp Val Cys
225                 230                 235                 240

Leu Asn Lys Gln Phe Phe Ile Lys Asp Asp Ser Leu Lys Ile Glu Asp
                245                 250                 255

Leu Val Lys Lys His Gly Asp Asn Leu Ala Ile Ser His Phe Glu Arg
            260                 265                 270

Trp Thr Val Gly Gln Gly
        275

<210> 7
<211> 424
<212> PRT
<213> Waddlia chondrophila

<400> 7

Met Asn Lys Asp Leu Val Ala Ile Phe Glu Tyr Leu Glu Lys Glu Lys
1               5                   10                  15

Gly Ile Gln Arg Glu Ile Val Ile His Ala Ile Glu Glu Ser Leu Gln
            20                  25                  30

Ala Ala Ala Arg Lys Ser Val Ser Gly Ala Ser Asn Val Thr Val Gln
        35                  40                  45

Ile Asp Pro Lys Thr Gly Asn Ile Asn Val Phe Ser Glu Lys Glu Ile
    50                  55                  60

Val Asp Glu Val Glu Val Pro Ala Gln Glu Ile Leu Leu Glu Glu Ala
65                  70                  75                  80

Arg Glu Ile Asp Pro Asp Cys Glu Ile Gly Gln Phe Ile Asp Ile Leu
                85                  90                  95

Ala Thr Pro Lys Asp Phe Gly Arg Ile Ala Ala Gln Lys Ala Arg Gln
            100                 105                 110

Ile Ile Ser Gln Lys Leu Arg Ser Ala Glu Arg Asp Val Ile Tyr Glu
        115                 120                 125

Glu Tyr Arg His Arg Val Asn Glu Val Val Ser Gly Thr Val Lys Arg
    130                 135                 140

Phe Val Lys Gly Ala Asn Leu Val Val Asp Leu Gly Lys Val Glu Ala
145                 150                 155                 160

Leu Met Pro Met Arg Gln Tyr Pro Lys Thr Glu Lys Tyr Lys Ile Gly
                165                 170                 175

Asp Arg Val Leu Ala Leu Leu Met Glu Val Asn Asp Thr Asp Ser Gly
            180                 185                 190

Gly Ala Glu Val Ile Leu Ser Arg Ser Ser Pro Glu Phe Val Arg Gln
        195                 200                 205

Leu Met Ile Gln Glu Val Pro Glu Ile Val Glu Gly Ile Val Val Ile
    210                 215                 220

Asp Lys Ile Glu Arg Glu Ala Gly Tyr Arg Thr Lys Leu Thr Val Arg
225                 230                 235                 240

Ala Thr Asp Gln Lys Val Asp Pro Val Gly Ala Cys Val Gly Met Arg
                245                 250                 255

Gly Asn Arg Val Lys Asn Val Val Arg Glu Leu Asn Ser Glu Lys Ile
            260                 265                 270

Asp Ile Ile Pro Tyr Ser Asp Asp Pro Ile Glu Leu Leu Gln Asn Ala
        275                 280                 285

Leu Ser Pro Ile Glu Ile Arg Lys Ile Ser Ile Ser Glu Asp Asp Arg
    290                 295                 300

Val Ile Ser Ile Val Ile Asp Asp Asp Asp Tyr Ala Ala Val Ile Gly
305                 310                 315                 320

Lys Lys Gly Met Asn Ala Arg Leu Asn Ser Arg Leu Ile Gly Tyr Glu
                325                 330                 335

Leu Glu Val Gln Arg Met Thr Asp Tyr Asn Lys Thr Met Ala Ile Gln
            340                 345                 350

Arg Thr Glu Leu Ala Glu Ser Asp Asp Pro Thr Leu Asp His Pro Leu
        355                 360                 365

Thr Gly Ile Glu Gly Val Asn Lys Leu Val Phe Glu His Leu Val Ala
    370                 375                 380

Glu Gly Phe Asn Thr Pro Arg Thr Leu Leu Leu Ala Ser Pro Glu Gln
385                 390                 395                 400

Leu Ser Asn Ala Ala Ser Ile Ser Leu Glu Met Ala Asp His Ile Leu
                405                 410                 415

Glu Gln Val Arg Lys Gln Arg Leu
            420

<210>  8
<211>  541
<212>  PRT
<213>  Waddlia chondrophila

<400>  8

Met Ala Ala Lys Asp Ile Lys Tyr Lys Glu Asp Ala Arg His Lys Ile
1               5                   10                  15

Leu Lys Gly Val Lys Thr Leu Ala Glu Ala Val Lys Ile Thr Leu Gly
            20                  25                  30

Pro Lys Gly Arg Asn Val Val Ile Asp Lys Ser Phe Gly Ala Pro His
        35                  40                  45

Ile Thr Lys Asp Gly Val Thr Val Ala Lys Glu Ile Glu Leu Glu Asp
    50                  55                  60

Lys His Glu Asn Met Gly Ala Gln Met Val Lys Glu Val Ala Ser Lys
65                  70                  75                  80

Thr Ala Asp Lys Ala Gly Asp Gly Thr Thr Thr Ala Thr Val Leu Ala
                85                  90                  95

Glu Ala Ile Tyr Ser Glu Gly Leu Arg Asn Val Thr Ala Gly Ala Asn
            100                 105                 110

Pro Met Asp Leu Lys Arg Gly Met Glu Glu Ala Val Lys Val Val Val
        115                 120                 125

Glu Glu Leu Gln Arg Leu Ser Lys Lys Val Glu Thr Gln Gln Glu Ile
    130                 135                 140

Ala Gln Val Ala Thr Ile Ser Ala Asn Asn Asp Glu Glu Ile Gly Gln
145                 150                 155                 160

Ile Ile Ala Lys Ala Met Glu Arg Val Gly Lys Asp Gly Thr Ile Thr
                165                 170                 175

Val Glu Glu Ala Lys Gly Phe Glu Thr Thr Leu Asp Val Val Glu Gly
            180                 185                 190

Met Asn Phe Asp Arg Gly Tyr Leu Ser Ala Tyr Phe Met Thr Asn Pro
        195                 200                 205

Glu Thr Gln Glu Cys Val Leu Glu Asp Val Tyr Val Leu Ile Phe Glu
    210                 215                 220

Lys Lys Ile Thr Ser Ile Lys Asp Leu Ile Pro Val Leu Gln Ser Val
225                 230                 235                 240

Ala Glu Ser Gly Lys Ser Leu Leu Ile Ile Ala Glu Asp Val Glu Gly
                245                 250                 255

Glu Ala Leu Ala Thr Leu Val Val Asn Arg Ile Arg Ala Gly Leu Lys
            260                 265                 270

Val Cys Ala Val Lys Ala Pro Gly Phe Gly Asp Arg Arg Lys Ala Met
        275                 280                 285

Leu Glu Asp Ile Ala Ile Leu Thr Gly Gly Gln Leu Ile Ser Glu Glu
    290                 295                 300

Thr Gly Met Thr Leu Glu Ser Thr Thr Val Glu His Leu Gly Lys Ala
305                 310                 315                 320

Lys Lys Ile Val Val Thr Lys Glu Asp Thr Thr Leu Ile Glu Gly Gln
                325                 330                 335

Gly Asp Gln Ser Lys Ile Lys Asp Arg Val Ala Leu Ile Lys Arg Gln
            340                 345                 350

Ile Glu Glu Thr Asp Ser Asp Tyr Asp Arg Glu Lys Leu Gln Glu Arg
        355                 360                 365

Leu Ala Lys Leu Ala Gly Gly Val Gly Val Ile Arg Val Gly Ala Ala
    370                 375                 380

Thr Glu Ile Glu Met Lys Glu Lys Lys Asp Arg Val Asp Asp Ala Gln
385                 390                 395                 400

His Ala Thr Lys Ala Ala Val Glu Glu Gly Ile Leu Pro Gly Gly Gly
                405                 410                 415

Val Ala Tyr Val Arg Cys Ile Gly Ala Val Asn Lys Lys Ala Asp Ala
            420                 425                 430

Leu Lys Gly Asp Gln Gln Thr Gly Ala Arg Ile Ile Ser Arg Ala Leu
        435                 440                 445

Ser Ala Pro Leu Arg Gln Ile Ala Ala Asn Ala Gly Gln Glu Gly Ser
    450                 455                 460

Ile Ile Leu Gln Gln Val Glu Lys Gly Lys Asp Ser Glu Gly Tyr Asn
465                 470                 475                 480

Ala Leu Thr Gly Glu Phe Val Asp Met Leu Thr Ala Gly Ile Leu Asp
                485                 490                 495

Pro Thr Lys Val Ser Arg Leu Ala Leu Glu Asn Ala Ala Ser Val Ala
            500                 505                 510

Gly Leu Leu Leu Thr Thr Glu Ala Ile Val Ala Glu Leu Pro Glu Asp
        515                 520                 525

Lys Ser Ser Ala Pro Ala Met His Ala Gly Met Asp Tyr
    530                 535                 540

<210>  9
<211>  232
<212>  PRT
<213>  Waddlia chondrophila

<400>  9

Met Gly Arg Leu Ser Lys Arg Phe Arg Glu Ile Ala Glu Ser Ile Asp
1               5                   10                  15

Val Gln Lys Gln His Thr Leu Glu Glu Ala Val Glu Leu Leu Lys Gln
            20                  25                  30

Leu Pro Pro Val Lys Phe Asp Gln Ser Ile Glu Val Ala Leu Lys Leu
        35                  40                  45

Gly Val Asp Pro Arg Lys Ser Asp Gln Asn Val Arg Gly Thr Val Ser
    50                  55                  60

Leu Pro Asn Gly Thr Gly Lys Thr Thr Arg Ile Leu Val Phe Ala Arg
65                  70                  75                  80

Gly Asp Lys Ile Gln Glu Ala Leu Asp Ala Gly Ala Asp Tyr Ala Gly
                85                  90                  95

Asp Asp Glu Tyr Phe Glu Lys Val Gly Lys Gly Trp Thr Asp Phe Asp
            100                 105                 110

Ala Val Val Ala Thr Pro Asp Leu Met Arg Asp Val Gly Lys Leu Gly
        115                 120                 125

Lys Ile Leu Gly Pro Arg Gly Leu Met Pro Thr Pro Lys Ala Gly Thr
    130                 135                 140

Val Thr Asn Glu Ile Gly Lys Ala Ile Gln Asp Leu Lys Gly Gly Lys
145                 150                 155                 160

Ile Glu Phe Lys Val Asp Arg Tyr Gly Val Val Asn Ala Ala Val Gly
                165                 170                 175

Lys Leu Ser Phe Thr Val Gln Gln Leu Val Glu Asn Ile Lys Ser Leu
            180                 185                 190

Ile Asp Ala Ile Gln Lys Gln Lys Pro Ala Ser Ser Lys Gly Gln Tyr
        195                 200                 205

Met Lys Ser Leu Val Leu Ser Ser Thr Met Gly Pro Gly Leu Lys Ile
    210                 215                 220

Glu Leu Arg Lys Val Glu Thr Ile
225                 230

<210> 10
<211> 488
<212> PRT
<213> Waddlia chondrophila

<400> 10

Met Lys Phe Ser Thr Val Ala Ser Leu Asp Lys Arg Thr Lys Ser Asp
1               5                   10                  15

Leu Leu Val Ile Pro Phe Tyr Gln Asn Lys Asn Pro Glu Pro Ala Ala
            20                  25                  30

Lys Ile Ala Ala Leu Asp Ser Glu Ile Gln Ala Ala Leu Ser Ser Gly
        35                  40                  45

Asp Phe Ser Ala Lys Glu Ala Glu Val Leu Val Thr Tyr Pro Ala Ser
    50                  55                  60

Ser Thr Asp Lys Arg Ile Ala Leu Leu Gly Leu Gly Lys Lys Asp Gln
65                  70                  75                  80

Val Ser Thr Glu Thr Phe Arg Arg Ala Phe Gly Ala Val Ile Lys Ala
                85                  90                  95

Ala Leu Ser Arg Lys Ala Cys Leu Leu Asn Val Ile Leu Pro Asp Glu
            100                 105                 110

Met Ser Asp Asp Thr Leu His Gly Ile Ala Glu Gly Leu Phe Leu Ala
        115                 120                 125

Asn Tyr Ala Tyr Asp Glu Leu Lys Glu Lys Gly Glu Glu Asn Val Lys
    130                 135                 140

Leu Val Asp Gln Cys Thr Leu Ile Gly Gly Lys Ala Ser Asp Leu Lys
145                 150                 155                 160

Lys Val Lys Lys Ile Gly Leu Ile Ala Glu Gly Val Tyr Leu Ala Arg
                165                 170                 175

Asn Leu Ile Asn Gly Asn Ala Asp Glu Ile Asn Pro Gln Lys Leu Ala

180                 185                 190

Asp Val Ala Lys Glu Ile Ala Lys Lys His Ala Ala Met Lys Ala Thr
        195                 200                 205

Val Phe Asp Lys Lys Arg Ile Glu Lys Glu Lys Met Gly Leu Leu Leu
    210                 215                 220

Ala Val Asn Arg Gly Ser Ser Val Asp Pro Ala Leu Ile Ile Leu Glu
225                 230                 235                 240

Tyr Ala Gly Asn Pro Arg Ser Lys Asp Lys Thr Val Ile Val Gly Lys
                245                 250                 255

Gly Ile Thr Tyr Asp Thr Gly Gly Leu Asn Leu Lys Pro Thr Gly Ser
            260                 265                 270

Met Glu Thr Met Lys Ser Asp Met Ser Gly Ala Ala Val Val Leu Gly
        275                 280                 285

Ile Met Gln Ala Val Ala Ser Val Gly Leu Lys Lys Asn Ile Thr Met
    290                 295                 300

Val Ile Pro Ser Thr Glu Asn Ser Ile Gly Ala Lys Ser Tyr Lys Pro
305                 310                 315                 320

Gly Asp Val Tyr Thr Ser Tyr Ser Gly Lys Thr Val Glu Ile Gly Asn
                325                 330                 335

Thr Asp Ala Glu Gly Arg Leu Val Leu Ala Asp Ala Leu Ala Tyr Ala
            340                 345                 350

Val Asp Lys Leu Lys Pro Ser Arg Met Ile Asp Phe Ala Thr Leu Thr
        355                 360                 365

Gly Ala Met Val Ile Ala Leu Gly Glu Val Thr Thr Gly Met Met Ser
    370                 375                 380

Asn Asp Asp Ser Leu Ala Ser Ala Leu Thr Glu Ala Gly Leu Thr Thr
385                 390                 395                 400

Tyr Glu Arg Val Trp Arg Leu Pro Leu Tyr Glu Glu Tyr Lys Lys Ala
                405                 410                 415

Leu Lys Ser Asp Phe Ala Asp Ile Lys Asn Val Gly Gly Arg Ala Gly
            420                 425                 430

Gly Ser Ile Thr Ala Ala Met Phe Leu Arg Glu Phe Val Lys Asp Val
        435                 440                 445

Pro Trp Val His Phe Asp Ile Ala Gly Thr Ala Phe Leu Ser Lys Gln

450                 455                 460

Gly Pro Tyr His Pro Lys Asn Gly Thr Gly Ile Gly Val Arg Leu Met
465                 470                 475                 480

Met Glu Phe Leu Glu Asn His Leu
                485

<210>  11
<211>  451
<212>  PRT
<213>  Waddlia chondrophila

<400>  11

Met Glu Ser Glu Lys Asn Ser Pro Asn Val Ala Ser Ala Val Pro Glu
1               5                   10                  15

Glu Lys Lys Pro Leu Gln Lys Asn Gly Asn Glu Gln Glu Arg Lys Ala
            20                  25                  30

Ala Arg Thr Thr Lys Ile Ser Asp Ile Gln Arg Met Asn Ile Asp Gln
        35                  40                  45

Leu Asn Gln Leu Gly Lys Gln Leu Gly Ile Lys His Thr Gly Ser Leu
    50                  55                  60

Thr Lys Ser Gln Met Val Phe Glu Ile Val Lys Ser Ile Ser Glu Asn
65                  70                  75                  80

Pro Asn Glu Ile Leu Tyr Gly Glu Gly Val Leu Glu Ile Leu Pro Asp
                85                  90                  95

Gly Phe Gly Phe Leu Arg Ser Thr Asn Tyr Asn Tyr Leu Pro Ser Ala
            100                 105                 110

Glu Asp Ile Tyr Val Ser Pro Ala Gln Ile Arg Arg Phe Asp Leu Lys
        115                 120                 125

Lys Gly Asp Thr Val Lys Gly Thr Ile Arg Pro Pro Lys Glu Lys Glu
    130                 135                 140

Lys Phe Phe Ala Leu Leu Arg Val Asp Thr Ile Asn Asp Gln Thr Pro
145                 150                 155                 160

Asp Gln Ala Arg Glu Arg Ile His Phe Glu Asn Leu Thr Pro Leu Tyr
                165                 170                 175

Pro Asp Glu Arg Leu Val Met Glu Thr Ala Ser Glu Lys Leu Ser Thr
            180                 185                 190

Arg Val Leu Asp Leu Ala Ala Pro Ile Gly Lys Gly Gln Arg Gly Leu
        195                 200                 205

Ile Val Ala Pro Pro Arg Thr Gly Lys Thr Ile Ile Leu Gln Asn Ile
    210                 215                 220

Ala Asn Ala Ile Ala Thr Asn Asn Lys Glu Ile Thr Leu Ile Val Leu
225                 230                 235                 240

Leu Ile Asp Glu Arg Pro Glu Glu Val Thr Asp Met Gln Arg Ile Val
                245                 250                 255

Lys Gly Glu Val Ile Ser Ser Thr Phe Asp Glu Pro Pro Glu Arg His
            260                 265                 270

Val Gln Val Ala Glu Met Ala Ile Glu Lys Ala Arg Arg Leu Val Glu
        275                 280                 285

His Gly Arg Asp Val Val Ile Leu Leu Asp Ser Ile Thr Arg Leu Ala
    290                 295                 300

Arg Ala Tyr Asn Thr Val Gln Pro His Ser Gly Lys Ile Leu Thr Gly
305                 310                 315                 320

Gly Ile Asp Ala Asn Ala Leu His Lys Pro Lys Arg Phe Phe Gly Ala
                325                 330                 335

Ala Arg Asn Ile Glu Gln Gly Gly Ser Leu Thr Ile Ile Ala Thr Ala
            340                 345                 350

Leu Ile Glu Thr Gly Ser Arg Met Asp Glu Val Ile Phe Glu Glu Phe
        355                 360                 365

Lys Gly Thr Gly Asn Met Glu Leu Val Leu Asp Arg Arg Leu Ala Asp
    370                 375                 380

Arg Arg Leu Tyr Pro Ala Ile Asp Leu Ile Lys Ser Gly Thr Arg Lys
385                 390                 395                 400

Glu Glu Leu Leu Tyr His Pro Ser Glu Leu Glu Lys Ile Tyr Leu Leu
                405                 410                 415

Arg Gln Ala Val Ala Asp Leu Thr Ser Val Asp Ala Met Asn Leu Leu
            420                 425                 430

Leu Gly Arg Leu Lys Lys Thr Lys Gln Asn Val Glu Phe Leu Leu Ser
        435                 440                 445

Met Lys Asp
    450

<210>  12
<211>  475

<212>  PRT
<213>  Waddlia chondrophila

<400>  12

Met Ser Arg Thr Ala Trp Lys Ile His Pro Asp Cys Tyr Trp Glu Cys
1               5                   10                  15

Lys Met Glu Asp Leu Ile Val Ile Gly Ser Gly Pro Ala Gly Tyr Val
            20                  25                  30

Ala Ala Ile Arg Ala Ser Gln Leu Gly Leu Ser Val Ala Ile Val Glu
        35                  40                  45

Lys Tyr Ser Thr Phe Gly Gly Thr Cys Leu Asn Val Gly Cys Ile Pro
    50                  55                  60

Ser Lys Ala Leu Leu Gln Ser Ser Glu His Phe Ala Phe Ile Glu Lys
65                  70                  75                  80

His Ala Asp Glu His Gly Ile Gln Leu Lys Asn Ala Thr Val Asp Phe
                85                  90                  95

Ser Lys Met Met Lys Arg Lys Glu Glu Val Val Ala Ser Leu Val Gly
            100                 105                 110

Gly Ile Glu Gly Leu Leu Lys Arg Asn Lys Val Lys Thr Val Gln Gly
        115                 120                 125

Ser Ala Arg Phe Val Ser Pro Glu Glu Ile Glu Val Asn Gly Lys Lys
    130                 135                 140

Met Arg Ala Lys His Phe Leu Ile Ala Thr Gly Ser Lys Pro Ile Glu
145                 150                 155                 160

Leu Pro Phe Leu Pro Phe Asp Glu Lys Val Val Val Ser Ser Thr Gly
                165                 170                 175

Ala Leu Ser Leu Pro Ser Val Pro Lys Lys Met Val Val Val Gly Ala
            180                 185                 190

Gly Val Ile Gly Val Glu Leu Ala Ser Val Tyr Ser Arg Leu Gly Ser
        195                 200                 205

Glu Val Ala Ile Val Glu Met Leu Asp Arg Ile Thr Pro Ala Met Asp
    210                 215                 220

Asn Gln Ile Gly Lys Thr Phe Gln Lys Ile Leu Ser Ala Gln Gly Leu
225                 230                 235                 240

Thr Phe His Leu Gly Ala Gln Val Thr Asp Ala Lys Val Ser Lys Lys
                245                 250                 255

Gly Ala Glu Leu Ser Val Arg Ile Lys Gly Lys Glu Ser Thr Leu Lys
            260                 265                 270

Ala Asp Val Val Leu Val Ala Ile Gly Arg Lys Pro Tyr Ser Glu Gly
        275                 280                 285

Leu Gly Val Glu Glu Ile Gly Ile Ala Lys Thr Lys Gln Gly Phe Ile
    290                 295                 300

Val Val Asp Gly Asn Phe Cys Thr Asn His Pro Asn Ile Leu Ala Val
305                 310                 315                 320

Gly Asp Ile Ile Asp Gly Pro Met Leu Ala His Arg Ala Ser Glu Glu
                325                 330                 335

Ala Val Ala Ala Val Glu Ser Leu Ala Gly Lys Ser Ser His Ile Asn
            340                 345                 350

Tyr Met Ala Ile Pro Asn Val Ile Tyr Thr Met Pro Glu Val Ala Ala
        355                 360                 365

Val Gly Leu Thr Glu Glu Glu Gly Lys Glu Met Gly Leu Glu Leu Ile
    370                 375                 380

Thr Gly Val Phe Pro Phe Lys Gly Asn Ala Arg Ala Arg Cys Ala Gly
385                 390                 395                 400

Glu Thr Glu Gly Ile Val Lys Val Ile Gly Glu Lys Asn Ser Gly Arg
                405                 410                 415

Leu Leu Gly Val His Leu Val Gly Ala Asn Ala Ser Glu Met Ile Gly
            420                 425                 430

Glu Gly Val Val Ala Ile Glu Lys Arg Ala Thr Val Arg Glu Leu Ala
        435                 440                 445

Tyr Thr Ser His Ala His Pro Thr Leu Ser Glu Ala Ile Lys Glu Ala
    450                 455                 460

Ala Leu Ala Ala Cys Glu Lys Pro Ile His Leu
465                 470                 475

<210>  13
<211>  290
<212>  PRT
<213>  Waddlia chondrophila

<400>  13

Leu Ile Glu Arg Gln Asp Ala Val Val Asn Gln Glu Gly Gln Gly Leu
1               5                   10                  15

Ala Glu Ile Thr Ile Lys Asn Leu Leu Glu Ala Gly Ala His Phe Gly
            20                  25                  30

His Gln Thr Ser Arg Trp Asn Pro Lys Met Lys Arg Phe Ile Phe Glu
        35                  40                  45

Glu Arg Asn Gly Leu Tyr Ile Ile Asp Leu Ala Lys Thr Leu Gln Gln
    50                  55                  60

Val Arg Asn Ala Ala Glu Ile Val Lys Glu Cys Val Lys Lys His Lys
65                  70                  75                  80

Ser Ile Leu Phe Val Gly Thr Lys Lys Gln Ala Lys Gly Val Val Lys
                85                  90                  95

Glu Leu Ala Glu Glu Cys Gly Glu Phe Tyr Val Cys Glu Arg Trp Leu
            100                 105                 110

Gly Gly Met Leu Thr Asn Leu Ser Thr Ile Arg Gln Ser Ile Lys Lys
        115                 120                 125

Leu Asp Arg Ile Glu Lys Gln Ile Ala Ala Gly Gly Glu Gly Leu Thr
    130                 135                 140

Lys Lys Glu Leu Ser Leu Leu Thr Lys Glu Gln Leu Lys Leu Asp Lys
145                 150                 155                 160

Asn Leu Ser Gly Val Arg Ser Met Arg Lys Leu Pro Gly Leu Val Val
                165                 170                 175

Val Val Asp Pro Ser Lys Glu His Ile Ala Val Ala Glu Ala Lys Lys
            180                 185                 190

Leu Gly Ile Pro Val Met Gly Leu Val Asp Thr Asn Cys Asp Pro Asp
        195                 200                 205

Pro Ile Asp Tyr Val Ile Ala Cys Asn Asp Asp Ala Leu Lys Ser Ile
    210                 215                 220

Lys Leu Ile Ile Asn Thr Leu Thr Asp Val Ile Ala Lys Glu Lys Asn
225                 230                 235                 240

Glu Met Arg Leu Val Ser Arg Lys Glu Gly Ser Glu Glu Glu Asp Lys
                245                 250                 255

Lys Ile Gly Glu Glu Ile Ala Val Gly Lys Val Asp Leu Ala Lys Ser
            260                 265                 270

Gly Lys Glu Asp Ala Glu Leu Lys Lys Thr Ala Gly Ala Ala Lys Glu
        275                 280                 285

Glu Asn
    290


<210>  14
<211>  520
<212>  PRT
<213>  Waddlia chondrophila

<400>  14

Met Arg Lys Gln Leu Gly Ile Leu Ala Ser Val Leu Leu Leu Ser Ala
1               5                   10                  15


Thr Thr Leu Leu Trp Thr Gly Cys Ser Asn Arg Ser Gly Ser Cys Asp
            20                  25                  30


Pro Cys Gly Pro Val Ser Glu Cys Gly Pro Cys Gly Pro Val Arg Glu
        35                  40                  45


Gln Val Asn Pro Asp Pro Cys Cys Glu Pro Ala Cys Lys Pro Pro Val
    50                  55                  60


Lys Cys Cys Tyr Pro Ser Ser Asn Gln Leu Lys Cys Leu Asp Gly Ile
65                  70                  75                  80


Thr Val Lys Ala Arg Asn Pro Lys Met Cys Met Leu Gly Asp Gln Tyr
                85                  90                  95


Ser Leu Asp Phe Asp Val Glu Ala Cys Asp Asp Val Cys Asp Val Thr
            100                 105                 110


Val Thr Thr His Leu Pro Glu Gly Val Asn Tyr Val Arg Ser Glu Pro
        115                 120                 125


Gln Ala Lys Val Glu Gly Arg Lys Leu Thr Trp Asp Phe Gly Ser Met
    130                 135                 140


Arg Lys Gly Glu Cys Arg Pro Ala Ser Val Trp Val Glu Cys Glu Cys
145                 150                 155                 160


Glu Gly Glu Leu Cys Ala Cys Phe Cys Ala Ser Ala Thr Pro Val Arg
                165                 170                 175


Phe Cys Ser Leu Leu Cys Ala Lys Pro Ile Leu Thr Cys Gln Lys Cys
            180                 185                 190


Gly Pro Glu Glu Val Cys Pro Gly Asp Pro Ile Asn Tyr Thr Ile Thr
        195                 200                 205


Val Thr Asn Arg Gly Ser Cys Thr Ala Glu Asp Val Val Leu Thr Asp
    210                 215                 220


Asn Leu Pro Asp Gly Val Gln His Ser Ser Gly Leu Arg Thr Leu Thr

225                 230                 235                 240

Tyr Lys Leu Gly Cys Leu Ala Pro Cys Glu Thr Lys Lys Val Asn Ile
                245                 250                 255

Cys Ala Thr Ala Cys Thr Arg Gly Glu Val Cys Asn Thr Ala Val Val
            260                 265                 270

Ser Ala Cys Asn Ala Asp Ser Val Ser Cys Gln Trp Cys Thr Cys Ile
        275                 280                 285

Cys Cys Cys Asp Cys Glu Val Val Lys Val Gly Pro Lys Glu Val Gln
    290                 295                 300

Ile Gly Lys Asn Ala Asp Tyr Gln Ile Thr Val Thr Asn Thr Gly Asp
305                 310                 315                 320

Lys Pro Leu Thr Asp Val Val Val Ser Asp Cys Ala Pro Asn Ala Thr
                325                 330                 335

Ser Ile Val Ser Ala Asn Gly Ala Met Ile Asn Gly Asn Gln Ala Val
            340                 345                 350

Trp Arg Leu Arg Glu Leu Lys Pro Gly Glu Lys Val Ser Phe Asn Ile
        355                 360                 365

Thr Leu Thr Thr Cys Thr Pro Gly Cys Phe Thr Asn Arg Val Ser Val
    370                 375                 380

Asp Asn Ser Gln Gly Cys Lys Cys Cys Asp Asp Phe Val Thr Arg Trp
385                 390                 395                 400

Arg Gly Arg Pro Ala Leu Asn Val Cys Ile Cys Asp Thr Glu Asp Pro
                405                 410                 415

Val Cys Val Gly Asp Pro Thr Ser Tyr Cys Ile Thr Val Val Asn Gln
            420                 425                 430

Gly Ser Glu Pro Asp Asp Asn Val Arg Val Val Val Arg Phe Pro Pro
        435                 440                 445

Glu Ile Val Pro Val Ser Ala Ser Gly Asp Ser His Gly Thr Val Glu
    450                 455                 460

Gly Asn Thr Val Thr Phe Ala Pro Tyr Asp Asn Phe Gly Pro Arg Gln
465                 470                 475                 480

Thr Leu Lys Phe Arg Val Asp Ala Arg Ala Asn Arg Ser Gly Asp Gly
                485                 490                 495

Arg Val Leu Ala Glu Val Thr Ser Glu Ser Ile Thr Thr Pro Ile Val

500                 505                 510

Gln Gln Glu Ser Thr Ile Val Asn
        515                 520

<210>  15
<211>  590
<212>  PRT
<213>  Waddlia chondrophila

<400>  15

Met Asn Phe Lys Ser Phe Phe Leu Gly Ala Ile Leu Phe Phe Pro Leu
1               5                   10                  15

Ala Ala Gln Val Ser Ala Asp Thr Lys Arg Gln Glu Ala Phe Leu Gln
            20                  25                  30

Glu Leu Glu Ala Ala Lys Tyr Thr Leu Thr Phe Lys Tyr Gly Pro Thr
        35                  40                  45

Glu Trp Lys Ser Glu Tyr Leu Gln Trp Asp Ala Asp Ala Ala Phe Glu
    50                  55                  60

Glu Ala Lys Ala Gln Met Ile Glu Ser Pro Gln Leu Thr Met Arg Asp
65                  70                  75                  80

Tyr Gln Arg Ile Tyr Arg Asp Phe Phe Gly Ser Leu Lys Asp Tyr His
                85                  90                  95

Val Ser Thr Leu Phe Tyr Ser Ser Glu Trp Ser Ala Phe Pro Ile Val
            100                 105                 110

Val Lys Ser Val Asn Asn Arg Tyr Phe Ile Thr Lys Phe Asp Phe Gln
        115                 120                 125

Leu Ser Leu Ser Tyr Ser Asp Leu Ala Phe Asp Phe Asp Glu Ile Asp
    130                 135                 140

Ile Glu Arg Leu Glu Ala Glu Leu Gln Lys Cys Asn Leu Gly Asp Glu
145                 150                 155                 160

Val Ile Ala Val Asn Gly Val Pro Val Ala Thr Leu Ile Glu Glu Leu
                165                 170                 175

Ile Asp Ser Glu Leu Asn Gly Asp Arg Thr Pro Thr Gly Tyr Ala Leu
            180                 185                 190

Ala Glu Lys Met Leu Phe Thr Arg Tyr Gly Arg Arg Gly His Gln Val
        195                 200                 205

Pro Lys Gly Asp Phe Thr Leu Thr Val Lys Pro Ile Phe Gly Lys Gln
    210                 215                 220

His Thr Val Asn Leu Ala Trp Ile His Val Ala Glu Trp Val Asn Asp
225                 230                 235                 240

Pro Gln Lys Lys Thr Val Glu Gln Lys Pro Ala Thr Lys Trp Gln Lys
                245                 250                 255

Leu Glu Lys Tyr Leu Val Arg Asp Tyr Ser Val Gly Leu Ala Lys Asp
            260                 265                 270

Met Leu Lys Ser Pro Leu Ala Ser Leu Gln Ala Asn Asp Asn Asp Glu
        275                 280                 285

Glu Glu Glu Glu Tyr Asp Trp Arg Glu Lys Ser Phe Val Pro Pro Leu
    290                 295                 300

Gly Gln Ile Val Trp Glu Thr Asp Ile Asp Glu Ala Phe Tyr Ala Tyr
305                 310                 315                 320

Ile Phe Lys Asn Gln Gln Gly Lys Lys Phe Gly Tyr Leu Tyr Leu Pro
                325                 330                 335

Asp Phe Ser Ala Ser Gly Trp Ser Ala Glu Asp Phe Met Gly Gln Leu
            340                 345                 350

Ile Thr Ile Ile Asp Lys Phe Glu Ser Glu Thr Glu Ala Leu Val Val
        355                 360                 365

Asp Ile Asn Asp Asn Pro Gly Gly Asn Leu Phe Tyr Met Tyr Gly Val
    370                 375                 380

Leu Ser Leu Leu Ala Asp Lys Pro Leu Glu Thr Cys Lys Asn Thr Glu
385                 390                 395                 400

Val Leu Val Gln Glu Asp Ile Tyr Asn Met Ala Val Ile Tyr Asn Glu
                405                 410                 415

Leu Ile Glu Met Leu Asn Asp Gly Glu Val Glu Gly Thr Ile Ser Gly
            420                 425                 430

Tyr Pro Ile Asp Glu Thr Val Ile Arg Lys Ile Ile Gly Tyr Ala Lys
        435                 440                 445

Ser Leu Ile Asp Gln Trp Asn Ser Gly Glu Thr Arg Thr Ile Pro Asp
    450                 455                 460

Tyr Val Phe Gly Ile Asp Thr Leu Asn Pro His His Glu Val His Tyr
465                 470                 475                 480

Thr Lys Pro Ile Leu Val Leu Ile Asn Glu Leu Asp Phe Ser Cys Gly
                485                 490                 495

Asp Phe Phe Pro Ala Ile Leu Gln Asp Asn Gly Arg Ala Thr Leu Phe
            500                 505                 510

Gly Arg Lys Thr Ala Gly Ala Gly Gly Tyr Val Arg Pro Tyr Gln Gln
        515                 520                 525

Thr Ser Gln Phe Gly Val Ala Leu Tyr Thr Leu Thr Gly Ser Leu Ala
    530                 535                 540

Tyr Arg Val Asp Gly Lys Val Val Glu Asn Leu Gly Val Thr Pro Asp
545                 550                 555                 560

Ile Glu Asn Gln Leu Thr Glu Lys Asp Leu Gln His Gly Phe Ile Asp
                565                 570                 575

Tyr Val Arg Ser Val Asn Lys Gln Leu Asn Ala Leu Val Lys
            580                 585                 590

<210>  16
<211>  399
<212>  PRT
<213>  Waddlia chondrophila

<400>  16

Met Phe Gly Gly Gly Ala Gly Gly Ile Gln Gly Pro His Ala Arg Gln
1               5                   10                  15

Thr Leu Glu Ala Met Lys Glu Val Gly Lys Glu Thr Ala Glu Glu Val
            20                  25                  30

Arg Gln Glu Gln Lys Val Ala Ala Gln Ser Ala Gln Ala Ser Asn Ala
        35                  40                  45

Leu Thr Ala Phe Arg Pro Lys Val Lys Thr Glu Lys Lys Lys Leu Lys
    50                  55                  60

Gln Ile Ser Thr Thr Val Ala Lys Met Ala Lys Met Lys Lys Glu Lys
65                  70                  75                  80

Lys Leu Leu Pro Ile Gln Gln Ile Ala Lys Lys Ala Glu Glu His Glu
                85                  90                  95

Arg Lys Asn Pro Glu Leu Lys Ala Lys Val Leu Lys Leu Leu Arg Glu
            100                 105                 110

Arg Ile Lys Pro Asp Ser Ser Ser Glu Asp Ile Gln Glu Ile Leu Asp
        115                 120                 125

Glu Phe Tyr Pro Asp Pro Thr Leu Ala Asp Asp Ala Met Glu Phe Leu
    130                 135                 140

Leu Asp Thr Thr Glu Gly Gln Leu His Gln Gln Val Gln Glu Ala Lys
145                 150                 155                 160

Glu Lys Leu Asn Glu Asp Lys Gly Arg Glu Ile Thr Ala Gly Arg Asn
                165                 170                 175

Ile Ser Lys Gln Ala Arg Gln Ala Ser Asp Lys Gly Leu Gly Thr Pro
            180                 185                 190

Thr Ser Leu Arg Glu Met Tyr Arg Asp Ile Thr Gly Asn Pro Arg Asp
        195                 200                 205

Ser Asn Thr Met Phe Gln Glu Leu Ser Glu Lys Tyr Ala Phe Lys Glu
    210                 215                 220

Met His Lys Val Val Lys Phe Leu Leu His Ser Leu Gly Ser Asp Met
225                 230                 235                 240

Lys Ser Lys Gly Pro Ser Ile Ser Arg Gly Glu Leu His Arg Leu Ile
                245                 250                 255

Thr Glu Thr Arg Ser Leu Gln Ala Ile Leu Gly Val Tyr Arg Phe Phe
            260                 265                 270

Lys Thr Arg Met Gly Leu Met Glu Lys Met Phe Ala Lys Ser Gly Leu
        275                 280                 285

Asp Met Pro Lys Lys Leu Asn Phe Glu Leu Met Ser Lys Cys Phe Met
    290                 295                 300

Asn Leu Ala Gly Glu Arg Tyr Pro Ser Ser Asp Lys Val Leu Ala Gln
305                 310                 315                 320

Ala Lys Arg Leu Gly Ile Glu Asp Trp Ile Leu Ala Lys Ile Ile Ala
                325                 330                 335

Phe Ser Gln Phe Arg Asp Ser Ile Lys Glu Met Ala Met Ser Gln Ile
            340                 345                 350

Tyr Arg Ser Leu Gln His Arg Asp Glu Leu Leu Phe Ala Ile Ile Glu
        355                 360                 365

Ala Leu Glu Glu Leu Glu Asp Gln Leu Glu Glu Leu Glu Glu Gln Asp
    370                 375                 380

Glu Glu Glu Glu Asp Trp Glu Glu Asp Glu Glu Glu Glu Glu Lys
385                 390                 395

<210>   17
<211>   417
<212>   PRT

<213>  Waddlia chondrophila

<400>  17

Met Tyr Asp Ile Leu Leu Cys Leu Val Ser Leu Ile Ala Leu Pro Lys
1               5                   10                  15

Leu Leu Tyr Gln Met Ala Phe His Lys Lys Tyr Arg Asn Ser Leu Lys
            20                  25                  30

Gln Arg Leu Gly Ile Gly Phe Pro Glu Ile Glu Lys Gly Asn Lys Lys
        35                  40                  45

Leu Ile Trp Val His Ala Val Ser Met Gly Glu Ala Lys Ala Val Ala
    50                  55                  60

Ala Leu Ala Arg Glu Leu Lys Lys Arg Ser Asp Asn Asp Ala Ile Leu
65                  70                  75                  80

Leu Phe Ser Thr Val Thr Glu Thr Gly Leu Ala Glu Gly Glu Lys Glu
                85                  90                  95

Leu Pro Glu Ala Asp Tyr His Val Phe Leu Pro Leu Asp Phe Ser Trp
            100                 105                 110

Ile Ile Arg Pro Ile Ile Arg Arg Val Arg Pro Asp Gln Val Ile Val
        115                 120                 125

Cys Glu Thr Asp Tyr Trp Tyr Asn Phe Leu Ser Ser Ser Lys Asn Ala
    130                 135                 140

Gly Ala Arg Leu Ser Val Val Asn Gly Lys Ile Ser Glu Arg Ser Met
145                 150                 155                 160

His Arg Leu Leu Lys Phe Pro Lys Phe Thr Lys Arg Leu Phe Cys Leu
                165                 170                 175

Ile Asp Lys Phe Cys Val Gln Ser His His Tyr Arg Glu Arg Phe Leu
            180                 185                 190

Lys Leu Gly Ile Pro Glu Glu Lys Ile Val Ile Thr Gly Asn Ile Lys
        195                 200                 205

Phe Asp Asn Ser Phe Pro Lys Leu Thr Glu Glu Glu Leu Val Glu Trp
    210                 215                 220

Lys Ser Lys Phe Gly Ile Arg Pro Glu Asp His Val Leu Val Ala Gly
225                 230                 235                 240

Ser Thr His Asp Pro Glu Glu Arg Ile Ile Leu Asp Ala Cys Phe Glu
                245                 250                 255

Val Trp Lys Asp Asp Pro His Leu Lys Ile Leu Ile Val Pro Arg His
            260                 265                 270

Pro Glu Arg Phe Asn Glu Val Ala Gln Leu Leu Lys Lys Arg Gly Val
        275                 280                 285

Glu Phe Ser Arg Tyr Ser Glu Gly Val Ser His Glu Ala Pro Val Ile
    290                 295                 300

Leu Val Asp Ala Met Gly Val Leu Leu Gln Cys Tyr Gln Ala Ala Thr
305                 310                 315                 320

Leu Ala Ile Val Ala Gly Ser Phe Thr Pro Lys Val Gly Gly His Asn
                325                 330                 335

Ile Val Glu Pro Cys Trp Tyr Gly Val Pro Val Leu Phe Gly Pro Tyr
            340                 345                 350

Leu Tyr Ser Gln Pro Glu Leu Leu Glu Leu Val Gln Glu Tyr Gly Ala
        355                 360                 365

Gly Val Gln Val Glu Pro Glu His Leu Ser Asp Glu Ile Lys Gly Leu
    370                 375                 380

Leu Ser Glu Pro Lys Arg Arg Lys Ala Leu Gly Ala Ala Gly Ile His
385                 390                 395                 400

Leu Ala Asp Ser Leu Gln Gly Ala Thr Glu Arg Thr Leu Val Glu Ile
                405                 410                 415

Ala

<210>  18
<211>  130
<212>  PRT
<213>  Waddlia chondrophila

<400>  18
   Val Ser Thr Lys Thr Glu Lys Leu Val Glu Glu Leu Ser Lys Leu Thr
   1               5                   10                  15
   Val Leu Glu Met Ser Glu Leu Lys Lys Ala Leu Glu Glu His Trp Gly
               20                  25                  30
   Val Glu Ala Ala Ala Ala Ala Val Val Ala Ala Pro Val Ala Ala Ala
           35                  40                  45
   Ala Gly Gly Glu Ala Ala Glu Glu Glu Ala Thr Asp Phe Glu Val Thr
       50                  55                  60
   Leu Thr Glu Val Pro Ala Asp Lys Lys Ile Ala Val Ile Lys Ile Val
   65                  70                  75                  80
   Arg Glu Val Thr Gly Leu Gly Leu Lys Glu Ala Lys Glu Leu Ala Glu
                   85                  90                  95
   Ser Ala Pro Lys Val Leu Lys Glu Ser Ala Pro Lys Ala Glu Ala Glu
               100                 105                 110
   Glu Ile Lys Lys Lys Leu Glu Asp Ala Gly Ala Lys Ala Val Leu Lys
           115                 120                 125
   Gly Val
       130

<210>  19
<211>  568
<212>  PRT
<213>  Waddlia chondrophila

<400>  19
   Met Ser Asn Lys Gln Glu His Ser Trp Asp Asp Ala Asn Ile Leu Asp
   1               5                   10                  15
   Asp Val Gln Tyr Lys Glu Glu Asp Ala Lys Thr Phe Gln Gln Leu Met
               20                  25                  30
   Gln Thr Ser Thr Asp Ala Ser Thr Asp Glu Asn Gln Pro Met Lys Pro
           35                  40                  45
   Gly Ser Ile Leu Lys Gly Lys Ile Val Glu Ile Ser Lys Glu Phe Val
       50                  55                  60
   Val Val Asp Val Gly Leu Lys Ser Glu Gly Leu Val Pro Met Glu Glu
   65                  70                  75                  80
   Phe Ser Asp Pro Ser Gln Ile Ile Leu Gly Asn Glu Ile Glu Val Phe
                   85                  90                  95
   Leu Asp Glu Ala Glu Asp Ser Asn Gly Gln Ile Ile Leu Ser Arg Glu
               100                 105                 110
   Lys Ala Glu Lys Leu Arg Lys Trp Glu Phe Ile Leu Glu His Cys Glu
           115                 120                 125
   Glu Gly Ser Ile Val Lys Gly Lys Val Ile Arg Lys Val Lys Gly Gly
       130                 135                 140
   Leu Met Val Asp Ile Gly Met Glu Ala Phe Leu Pro Gly Ser Gln Leu
   145                 150                 155                 160
   Asp Asn Lys Arg Ile Lys Asn Leu Asp Glu Tyr Ile Gly Gln Thr Tyr
                   165                 170                 175
   Glu Phe Lys Ile Leu Lys Ile Asn Ile Asp Arg Lys Asn Val Val Val
               180                 185                 190
   Ser Arg Arg Glu Leu Leu Glu Ala Glu Arg Ile Ser Lys Lys Ala Glu
           195                 200                 205
   Leu Leu Ala Thr Ile Ser Glu Gly Asp Ile Arg Glu Gly Ile Val Lys
       210                 215                 220
   Asn Ile Thr Asp Phe Gly Val Phe Leu Asp Leu Asp Gly Ile Asp Gly
   225                 230                 235                 240
   Leu Leu His Ile Thr Asp Met Thr Trp Lys Arg Ile Arg His Pro Ser
                   245                 250                 255
   Glu Met Val Lys Ile Gly Asp Lys Leu Glu Val Met Ile Leu Ser Val
               260                 265                 270
   Asp Arg Asp Lys Gly Arg Val Ala Leu Gly Leu Lys Gln Lys Glu Ser
           275                 280                 285
   Asn Pro Trp Asp Glu Ile Glu Gln Lys Tyr Pro Pro Gly Thr Arg Val
       290                 295                 300
   Lys Gly Lys Ile Val Asn Leu Leu Ser Tyr Gly Ala Phe Ile Glu Ile
   305                 310                 315                 320
   Glu Asp Gly Ile Glu Gly Leu Ile His Val Ser Glu Met Ser Trp Val
                   325                 330                 335
   Lys Asn Val Thr Asp Pro Ser Glu Val Val Asn Lys Gly Asp Glu Val
               340                 345                 350
   Glu Ala Ile Val Leu Ser Val Gln Arg Asp Glu Gly Lys Ile Ser Leu
           355                 360                 365
   Gly Ile Lys Gln Thr Glu Arg Asn Pro Trp Asp Glu Val Glu Glu Lys
       370                 375                 380
   Tyr Pro Val Asp Lys Asn Val Lys Val Glu Val Lys Ser Leu Thr Asn
   385                 390                 395                 400
   Tyr Gly Ala Phe Val Glu Leu Glu Pro Gly Val Glu Gly Leu Ile His
                   405                 410                 415
   Ile Ser Asp Leu Ser Trp Ile Lys Lys Val Ser His Pro Ser Glu Ile
               420                 425                 430
   Leu Lys Lys Gly Asp Met Val Asp Ala Val Ile Leu Ser Val Asp Lys
           435                 440                 445
   Glu Ser Lys Lys Ile Thr Leu Gly Val Lys Gln Leu Gly Ser Asn Pro
       450                 455                 460
   Trp Glu Asp Ile Lys Glu Thr Met Pro Ile Asp Ser Ile Val Lys Gly
   465                 470                 475                 480
   Thr Val Ser Lys Ile Thr Ala Tyr Gly Ala Phe Val Glu Leu Asp Asn
                   485                 490                 495
   Gly Leu Glu Gly Leu Ile His Val Thr Glu Leu Ser Asp Glu Ala Phe
               500                 505                 510
   Gly Lys Val Glu Asp Val Val Ser Val Gly Ala Asp Val Thr Ala Lys
           515                 520                 525
   Val Met Lys Val Asp Pro Glu His Lys Lys Ile Ser Leu Ser Ile Lys
       530                 535                 540
   Glu His Met Phe Asp Lys Thr Gln Ser Ser Arg Asp Asp Val Val Val
   545                 550                 555                 560
   Ser Asn Glu Ser Asp Met Arg Asp
                   565

<210>  20
<211>  483
<212>  PRT
<213>  Waddlia chondrophila

<400>  20

Met Asn His Gln Pro Asp Ser Ile Ser Tyr Ala Lys Lys Trp Ser Met
1               5                   10                  15

Val Ala Ala Phe Met Ala Phe Val Cys Phe Pro Ile Ser Gly Phe Cys
            20                  25                  30

Lys Asp Gln Pro Leu Gly Arg Thr Ser Leu Asp Phe Thr Glu Val Val
        35                  40                  45

Lys Lys Ala Thr Pro Ala Val Val Ser Val Lys Val Lys Ser Lys Thr
    50                  55                  60

Lys Arg Asn Ser Phe Gln Phe Gly Asp Ser Asp Pro Phe Asp Phe Phe
65                  70                  75                  80

Asn His Asp Asp Phe Phe Gly His Phe Phe Gly Arg Arg Pro Arg Ser
                85                  90                  95

Pro Ala His Pro Gln Glu Pro Gln Tyr Gln Thr Gly Gln Gly Ser Gly
            100                 105                 110

Phe Ile Ile Ser Glu Asp Gly Tyr Ile Leu Thr Asn Gly His Val Val
        115                 120                 125

Asn Gly Ala Glu Glu Ile Ser Val Leu Phe Asn Asp Gly Arg Glu Tyr
    130                 135                 140

Asn Ala Glu Leu Ile Gly Ile Asp Ser Ser Thr Asp Ile Ala Val Ile
145                 150                 155                 160

Lys Ile Asn Asp Lys Asn Leu Pro Tyr Leu Asn Leu Gly Asn Ser Asp
                165                 170                 175

Asp Leu Glu Val Gly Gln Trp Val Ile Ala Ile Gly Asn Pro Leu Gly
            180                 185                 190

Leu Gln Ala Ser Val Thr Ala Gly Val Val Ser Ala Lys Gly Arg Ser
        195                 200                 205

Gly Leu Asp Leu Ala Arg Ile Glu Asp Phe Ile Gln Thr Asp Ala Ala
    210                 215                 220

Ile Asn Arg Gly Asn Ser Gly Gly Pro Leu Leu Asp Leu Asp Gly Asn
225                 230                 235                 240

Val Ile Gly Met Asn Thr Ala Ile Val Thr Asn Met Gly Ser Gly Gly
                245                 250                 255

Tyr Met Gly Ile Gly Phe Ala Ile Pro Ser Asn Met Ile Gln Asn Ile
            260                 265                 270

Met Glu Gln Leu Ile Ala Asn Gly Ser Val Thr Arg Gly Phe Ile Gly
        275                 280                 285

Ile Asn Leu Gln Pro Ile Asp Asn Asn Leu Ala Gln Ser Phe Ser Leu
    290                 295                 300

Ser Lys Val Lys Gly Ala Leu Val Ser Asp Val Thr Pro Asp Ser Pro
305                 310                 315                 320

Ala Gln Lys Ala Gly Leu Lys Gln Gly Asp Ile Ile Leu Asp Tyr Asn
                325                 330                 335

Gly Lys Glu Val Ser Thr Ile Ser Ser Leu Arg Asn Ala Val Ser Phe
            340                 345                 350

Met Lys Pro Gly Ser Lys Ile Thr Leu Lys Val Leu Arg Asp Gly Lys
        355                 360                 365

Glu Ile His Ile Pro Leu Thr Val Gly Glu Phe Pro Asp Glu Glu Glu
    370                 375                 380

Ile Ala Ser Ser Lys Thr Asn Lys Leu Gly Ile Glu Val Lys Pro Leu
385                 390                 395                 400

Thr Ala Asp Ile Ala His Asn Leu Gly Tyr Val Asp Leu Lys Gly Val
                405                 410                 415

Val Val Ser Lys Ile Ser Ala Gly Ser Pro Ala Ala Met Ala Gly Ile
            420                 425                 430

Lys Gln Gly Ala Leu Ile Thr Ala Val Asn His Val Lys Ile Asp Ser
        435                 440                 445

Val Glu Ala Phe Asn Gln Gln Ile Ser Lys Ala Asp Pro Gln Lys Pro
    450                 455                 460

Ile Leu Leu Leu Val Lys Gln Gly Asp Tyr Met His Phe Val Ser Ile
465                 470                 475                 480

Lys Thr Gly

<210>  21
<211>  1739
<212>  PRT
<213>  Waddlia chondrophila

<400>  21

Met Lys Lys Arg Leu Leu Leu Ile Ile Pro Leu Val Leu Leu Ala Phe
1               5                   10                  15

Ala Gly Ile Phe Arg His Ala Ile Leu Gly Asn Val Leu Thr Tyr Thr
            20                  25                  30

Ala Asn Arg Tyr Thr Leu Asn Lys Phe Gly Val Pro Leu Glu Phe Glu
        35                  40                  45

Lys Ile Ser Lys Lys Gly Arg Leu Trp Glu Ile Arg Ser Leu Ala Ile
    50                  55                  60

Lys Arg Ser Asn Leu Ser Leu Thr Ala Asp Lys Leu Glu Val Ala Phe
65                  70                  75                  80

Glu Trp His Pro Leu Gly Gly Tyr Phe Phe Val Lys Ile Leu Val Arg
                85                  90                  95

Asp Ser Glu Ile Ala Ile Asn Glu Gln Ala Ala Asp Phe Ser Ser Leu
            100                 105                 110

Leu Ala Asp Leu Leu Pro Asn Arg Leu Pro Ile Trp Met Leu Asn Val
        115                 120                 125

Gln Thr Val Cys Glu Ile Ser Asn Gly Arg Val Ser Trp Ile Ala Lys
    130                 135                 140

Gly Gly Glu Ala Glu Ser Ala Val Phe Glu Leu Gln Ala Ser Ser Gly
145                 150                 155                 160

Gly Ser Arg Gln Asp Leu Glu Leu Ser Val Trp Leu Asp Glu Glu Val
                165                 170                 175

Arg Gly Arg Asn Ala Phe Cys Leu Ser Leu Thr Lys Asp Asp Phe Lys
            180                 185                 190

Gly Leu Ser Thr Gly Leu Thr Phe Tyr Gln Val Glu Cys Gly Lys Val
        195                 200                 205

Ala Arg Gly Phe Glu Ala Leu Gly Arg Pro Leu Lys Gly Trp Glu Ile
    210                 215                 220

Gln Arg Gly Met Ile Asp Gly Asn Val Glu Leu Ala Trp Pro Ser Ser
225                 230                 235                 240

His Ala Pro Tyr Ala Ile Gly Lys Ala Ser Val Thr Asp Leu Arg Phe
                245                 250                 255

Asp His Pro Glu Ser Lys Thr Thr Gly Glu Val Arg Glu Ala Val Leu
            260                 265                 270

Asp Leu His Met Arg His Ile Lys Gly Glu Met Pro Ser Leu Glu Gly
        275                 280                 285

Lys Val Glu Phe Thr Gly Gly Thr Ser Leu Ala Phe Gln Arg Lys Gly
    290                 295                 300

Glu Pro Phe Trp Lys Ile Asp Asn Leu Ser Gly Gly Leu Tyr Phe Glu
305                 310                 315                 320

Pro Glu Arg Ala Val Met Ile Asn Met Gly Gly Lys Cys Ser His Glu
                325                 330                 335

Leu Cys Asp Phe Cys Leu Ser Leu Asp Gly Lys Ala Gln Leu Val Asp
            340                 345                 350

Lys Gln Lys Ala Ser Leu Asp Leu Ala Val Arg Leu Phe Ser Glu Gly
        355                 360                 365

Gly Lys Glu Ala Ala Val Arg Phe Ile Ala Glu Gln Leu Gly Pro Lys
    370                 375                 380

Glu His Gln Ala Glu Leu Phe Val Glu Asn Val Gly Pro Ala Glu Phe
385                 390                 395                 400

Ala Phe Val Gln Glu Ala Leu Ala Arg Tyr Ser Asp Asp Trp Asn Gly
                405                 410                 415

Phe His Ile Gln Lys Gly Leu Leu Asp Ala Glu Gly Leu Val Ser Met
            420                 425                 430

Val Gly Trp Gln Leu Thr Asp Leu Asp Ile Arg Gln Leu Lys Val Glu
        435                 440                 445

Gly Met Glu Val Asp Val Pro Ser Leu Asp Leu Ser Ala Asn Ile Glu
    450                 455                 460

Lys Val Thr Gly Gly Leu Ala Ile His Leu Leu Glu Glu Glu Pro Leu
465                 470                 475                 480

Arg Thr Ile Asp Ala Asp Phe Phe Ile Asp Gly Gly Cys Leu Val Ser
                485                 490                 495

Gln Thr Leu Ser Gly Ser Leu Cys Cys Leu Asn Asp Val Arg Thr Glu
            500                 505                 510

Leu Lys Val Arg Gln Gly Val Ile Gln Gln Ser Glu Met Arg Gly Glu
        515                 520                 525

Phe Ala Gly Leu Lys Gly Thr Val Gly Ile Asp Trp Thr Ala Lys Asp
    530                 535                 540

Lys Ile Val Gln Ala Tyr Phe Ser Gly Pro Ser Glu Arg Leu Ala Pro
545                 550                 555                 560

Phe Leu Pro Ala His Phe Arg Ser Pro Phe Leu Gln Ser Phe Ser Ser
                565                 570                 575

Asn Asp Leu Glu Ile Lys Ala Gly Val Val Leu Lys Asn Glu Lys Leu
            580                 585                 590

Ala Val Lys Gly Glu Ala Arg Met Ala Glu Gln Thr Val Ala Phe Gly
        595                 600                 605

Phe His Leu Glu Lys Ser Ser Glu Lys Leu Trp Lys Ser Trp Pro Ala
    610                 615                 620

Asn His Leu Ala Ala Ser Tyr Trp Asp Asn Val Gly Ala Glu Val Met
625                 630                 635                 640

Lys Thr Val Val Pro Pro Ile Ala Ser Pro Ala Val Leu Phe Glu Ala
                645                 650                 655

Asn Trp Ile Arg Ala Glu Thr Gly Ile Ala Gly Leu Val Leu Arg Asn
            660                 665                 670

Gly Trp Phe Lys Ala Lys Glu Val Asp Leu Glu Arg Phe Val Ala Pro
        675                 680                 685

Phe Leu Phe Ser Ser Ser Gln Met Gln Leu Ser Gly Arg Gly Ser Phe
    690                 695                 700

Ile Gly Gly Phe Asn Gln Asn Val Leu Thr Val Glu Tyr Glu Ala Gln
705                 710                 715                 720

Asn Val Val Met Gly Asn Arg Phe Met Glu Ile Asn Ala Glu His Ile
                725                 730                 735

Asp Arg Gly Val His Tyr Phe Asp Phe Lys Lys Gly Val His Phe Gly
            740                 745                 750

Ser Ile Pro Leu Ser Gln Ala Val Tyr Thr Glu Lys Asn Thr Gly Leu
        755                 760                 765

Val Phe Thr Pro Val Asp Met Gln Val Ala Val Glu Gly Glu Lys Met
    770                 775                 780

His Leu Ala Gln Ile Asp Ala Glu Cys Cys Gly Met Arg Met Ala Gly
785                 790                 795                 800

Glu Ile Asp Ile Asp Phe Gly Lys Pro Asp Asp Gly Val Phe Asp Leu
                805                 810                 815

Glu Ile Arg Val Arg Asn Val Lys Gly Gly Leu Lys Asn Val Gln Asp
            820                 825                 830

Leu Leu Ala Arg Phe His Pro Asp Leu Trp Leu Leu Gln Leu Pro Val
        835                 840                 845

Asp Gly Gln Phe Glu Leu Lys Asp Glu Gly Ala Phe Phe Lys Leu Ser
    850                 855                 860

Phe Thr Pro Glu Asp Tyr Ala Val Glu Ala Val Leu Lys Gly Lys Ile
865                 870                 875                 880

Gln Glu Ala Phe Val Ala Ser Gly Pro Tyr Gly Arg Gly Gly Leu Ser
                885                 890                 895

Gly Leu Gln Leu Gln Phe Gly Tyr Asp His Leu Asn Arg Leu Leu Glu
            900                 905                 910

Ile Ser His Leu Gln Ala Asp Ile Tyr Val Asp Asp Gln Phe Thr Gly
        915                 920                 925

Tyr Arg Leu Tyr Gly Asp His Leu Ser Val Asp Asp Leu Glu Glu Gly
    930                 935                 940

Arg Ser Ser Phe Asp Phe Trp Val Gly Asp Gln Asn Arg Asp Leu Ile
945                 950                 955                 960

Arg Phe Val Gly Arg Thr Gln Gln Glu Met Asp Pro Tyr Glu Arg Pro
                965                 970                 975

Tyr Ile Ala Val His Ile Asp His His Leu Thr His Leu Gly Asn Ile
            980                 985                 990

His Pro Ser Ser Phe Leu Leu Arg  Leu Lys Ser Pro Thr  Val Val Asp
        995                 1000                 1005

Gln Phe  Gln Ile Gly Met Gln  Val Asp Leu Glu Ala  Leu Phe Asn
    1010                 1015                 1020

Asp Leu  Phe Ala Val Ser Cys  Ser Gly Gly Gly Ser  Ala Leu Glu
    1025                 1030                 1035

Gln Leu  Gly Lys Glu Ile Gln  Trp Met His Asn Pro  Ala Gly Thr
    1040                 1045                 1050

Leu Asp  Val Gln Leu His Tyr  Asp Leu Arg Ser Asn  Ser Leu Ile
    1055                 1060                 1065

Tyr Arg  Ile Asp Gly Ser Gly  Ile Glu Met Asn Gln  Leu Thr Ala
    1070                 1075                 1080

Lys Lys  Met Lys Leu Ala Gly  Ser Lys Ser Gln Ser  Lys Trp Ile
    1085                 1090                 1095

Val Asp  Gln Leu Gln Trp Asp  Glu Lys Val Ile Ala  Gly Glu Phe
    1100                 1105                 1110

Met Pro  Glu Gly Glu Arg Trp  Lys Ile Glu Gly Phe  Gln Phe Arg
    1115                 1120                 1125

Asp Gln  Asp Ile Leu Ala Leu  Asn Phe Asp Gly Tyr  Phe Ile Pro
    1130                 1135                 1140

Ala Arg  Lys Lys Leu Asp Ala  Arg Val Asn Glu Val  Gln Ile Asp
    1145                 1150                 1155

Leu Ala  Gln Val Ser Gly Thr  Ala Glu Ile Glu Gln  Phe Arg Arg
    1160                 1165                 1170

Glu Asn  Asp Pro His Gly Lys  Leu Thr Gly Tyr Gly  Asn Leu Ser
    1175                 1180                 1185

Val Glu  Trp Gly Ser Ser Arg  Val Pro Trp Lys Phe  Asp Ala Ile
    1190                 1195                 1200

Leu Asp Val Ala Leu Glu Gln Trp Asp Leu Lys Gly Ile Arg Phe
    1205                1210                1215

Asp Asp Ala His Asn Ile Ser Cys His Tyr Ile Ser Gly Gln Gly
    1220                1225                1230

Met Thr Met Arg Lys Leu Arg Thr Lys Met Leu Asp Lys Ala Ser
    1235                1240                1245

Thr Glu Val Leu Ala Gln Val Asp Ile Glu Lys Ile Gln Tyr Asp
    1250                1255                1260

Phe Ser Thr Gly Glu Val Leu Phe Asp Glu Leu Ala Phe Ala Val
    1265                1270                1275

Pro Ala Asp Arg Leu Pro Arg Phe Ser Ala Gln Leu Glu Arg Ser
    1280                1285                1290

Phe Pro Asn Phe Val Thr Pro Val Met Ala Glu Ile Ile Cys Ser
    1295                1300                1305

Cys Lys Gln Glu Gly Val Leu Lys Gly Val Met Gln Tyr Glu Leu
    1310                1315                1320

Thr Pro Pro Tyr Thr Ala Met Lys Leu Thr Leu Glu Asp Gly Ile
    1325                1330                1335

Tyr Asn Phe Leu Asn Ala Ala His Lys Ile Ser Gly Phe Val Met
    1340                1345                1350

Asp Tyr Asp Pro Phe Glu Phe Ser Val Val Ser Gly Tyr Arg Leu
    1355                1360                1365

Ala Gly Arg Asp Val Trp Leu Tyr Ala Arg Ser Ser Ser Pro Ser
    1370                1375                1380

Leu Ala Tyr Gly Glu Leu Val Leu Thr Asp Gln Asp Pro Glu Lys
    1385                1390                1395

Val Ser Gln Gln Arg Pro Lys Glu Ala Leu Tyr Phe Asp Trp Glu
    1400                1405                1410

Asn Asn Ser Glu Thr Gly Ile Cys Phe Thr His Ile Glu Gly Ser
    1415                1420                1425

Tyr Gln Gly Leu Gln Met Glu Leu Glu Lys Asp Pro Thr Gln Ala
    1430                1435                1440

Pro Ser Lys Glu Ala Leu Phe Leu Val Gly Thr Val Gly Ile His
    1445                1450                1455

Gly Gln Lys Ala Lys Glu Phe Phe Pro Glu Gln Met Ala Asp Lys
    1460                1465                1470

Phe Val Ala Trp Gln Val Gly Glu Gly Tyr Ser Leu Gln Gly Lys
    1475                1480                1485

Trp Arg Phe Leu Lys Asn Tyr Ser Glu Asn Tyr Gly Asp Lys Leu
    1490                1495                1500

His Phe Val Gly Met Leu Lys Gly Ser Asp Phe His Leu Lys Gly
    1505                1510                1515

Tyr Gln Leu Asp Ser Leu Gln Ala His Leu Glu Tyr Thr Pro Thr
    1520                1525                1530

Ala Ile Arg Ile Asn Asp Leu Val Ile Asn Asp Arg Cys Gly Val
    1535                1540                1545

Leu Thr Ser Asp Arg Ile Asp Ile Leu Lys Thr Asp Leu Gly Thr
    1550                1555                1560

Trp Ala Phe Ala Met Pro Leu Met Ile Val Asn Gln Phe Arg Pro
    1565                1570                1575

Ser Leu Leu Gln Glu Glu Gly Phe Pro Arg Pro Thr Ala Arg Lys
    1580                1585                1590

Pro Met Val Val Gln Glu Leu Ile Leu Glu Lys Thr Gln Gly Asn
    1595                1600                1605

Leu Ser Asp Ser Arg Thr Met Ile Gly Arg Gly Ser Leu Tyr Phe
    1610                1615                1620

Thr Asn Arg Ser Lys Lys Phe Leu Gln Asp Thr Ile Phe Gln Ile
    1625                1630                1635

Pro Ser Asp Ile Leu Ser Arg Ile Gly Leu Asp Ser Ala Val Leu
    1640                1645                1650

Thr Pro Val Ser Gly Thr Ile Glu Tyr Glu Ile His Asp Gly Arg
    1655                1660                1665

Ile Tyr Leu Thr Arg Phe Lys Glu Met Tyr Ser Asp Ala Lys Leu
    1670                1675                1680

Ser Lys Phe Tyr Leu Ala Ser Ser Pro Ser Ser Thr Val Asp Phe
    1685                1690                1695

Asp Gly Gly Leu Asn Val Gln Val Arg Met Lys Gln Tyr Asn Leu
    1700                1705                1710

Ile Phe  Lys Leu Ala Glu Leu  Phe Thr Phe Asn Ile  Gln Gly Asp
    1715                1720                1725

Leu Lys  Asn Pro Val Tyr Ser  Ile Gln Lys Gln
    1730                1735

<210>  22
<211>  64
<212>  PRT
<213>  Waddlia chondrophila

<400>  22

Met Leu Cys Leu Asn Thr Ser Glu Gly Ile Glu Tyr Cys Thr Asp Thr
1               5                   10                  15

Gly Gly Cys Ala Tyr Glu Asp Ala Tyr Gln Ser Cys Cys Ile Ala Pro
            20                  25                  30

Ala Ile Ala Phe Ala Leu Val Ala Ile Ala Gly Ile Ile Ala Val Gly
        35                  40                  45

Val His Asn Arg Ser Ser Ser Ser His Thr Thr Asn Ala His Thr Gln
    50                  55                  60

<210>  23
<211>  94
<212>  PRT
<213>  Waddlia chondrophila

<400>  23

Met Leu Lys Lys Phe Phe Ala Ser Val Ile Thr Phe Ala Leu Leu Cys
1               5                   10                  15

Phe Thr Pro Gly Asn Val Glu Cys Tyr Gln Gly Cys Gly Ser Ser Gln
            20                  25                  30

Gln Cys Cys Lys Asp Tyr Gly Gly Tyr Ile Pro Ala Ala Gly Arg Ala
        35                  40                  45

Tyr Glu Ser Ala Cys Asn Thr Leu Cys Phe Ser Pro Cys Leu Ala Val
    50                  55                  60

Gly Val Val Ala Val Ala Ser Leu Ala Thr Ile Ile Ala Leu Thr Asn
65                  70                  75                  80

Asn Ser Gly Asp Thr Gly Val Val Ile His Ala His Ser Asp
                85                  90

<210>  24
<211>  173
<212>  PRT
<213>  Waddlia chondrophila

<400>  24

Met Glu Thr Ala Met Asp Phe Pro Thr Thr Ala Thr Ile Thr Ala Ile
1               5                   10                  15

Ala Thr Ile Ile Arg Asn Leu Ile Pro Cys Leu Leu Ala Val Ser Cys
            20                  25                  30

Gln Ser Ser Pro Pro Ser Trp Lys Val Ser Glu Thr Lys Ser Arg His
        35                  40                  45

Pro Ser Tyr Gln Ala Cys Ser Ile Ser Gln Lys Pro Asp Asn Leu Tyr
    50                  55                  60

Arg Gly Met Glu Leu Glu Phe Val Arg Thr Ser Ser Arg Thr His Ala
65                  70                  75                  80

Tyr Leu Asn Val Phe Ser Leu Thr Phe Pro Ser Asp Arg Leu Asp Glu
                85                  90                  95

Asn Lys Thr Lys Val Val Ile Lys Ile Glu Asp Glu Glu Gln Ile Phe
            100                 105                 110

Leu Ala His Arg Leu Glu Gly Gly Gln Arg Leu Leu Leu Pro Glu Asp
        115                 120                 125

Ala Arg Asp Leu Leu Val Gln Ala Leu Leu Glu Asn Lys Pro Val Ala
    130                 135                 140

Met Ile Ala Gly Arg Phe Asp Val Lys Ile Ser Pro Gly Asn Phe Ser
145                 150                 155                 160

Lys Lys His Cys Gln Lys Ile Asn Leu Tyr Glu Lys Arg
                165                 170

<210>  25
<211>  173
<212>  PRT
<213>  Waddlia chondrophila

<400>  25

Val Lys Lys Ser Phe Leu Ser Ile Cys Leu Phe Val Leu Thr Val Cys
1               5                   10                  15

Leu Pro Ala Gln Ala Ala Val Glu Lys Pro Leu Glu Glu Pro Gly Thr
            20                  25                  30

Val Thr Phe Val Pro Pro Lys Asn Trp Arg Met Val Asp Ala Lys Gln
        35                  40                  45

Leu Lys Gly Asn Val Arg Phe Met Val Ile Gly Lys Gly Lys Tyr Glu
    50                  55                  60

Phe Pro Pro Ser Ile Asn Leu Ser Thr Glu Glu Tyr Lys Gly Thr Leu
65                  70                  75                  80

Arg Glu Tyr Leu Ala Ile Val Lys Glu Ile Asn Ser Ser Gln Gly Ser
                85                  90                  95

Glu Trp Lys Asn Leu Gly Met Ile Lys Thr Glu Ala Gly Asp Ala Ser
            100                 105                 110

Leu Ser Gln Ala Asp Ala Ile Thr Glu Trp Gly Pro Ile Arg Met Met
        115                 120                 125

His Val Ile Leu Leu Arg Asp Asp Val Ile Tyr Ile Leu Thr Ala Ala
    130                 135                 140

Ser Leu Lys Lys Glu Phe Pro Thr Leu Tyr Gln Thr Phe Phe Lys Ser
145                 150                 155                 160

Phe Lys Ser Leu Lys Ile Thr Asn Ile Ala Pro His His
                165                 170

<210>  26
<211>  299
<212>  PRT
<213>  Waddlia chondrophila

<400>  26

Met Met Lys Ile Leu Lys Asn Leu Val Phe Gly Ile Leu Leu Thr Ser
1               5                   10                  15

Ala His Phe Thr His Ala Phe Asp Gly Tyr Asp His Cys Asp Pro Cys
            20                  25                  30

Tyr Asp Gln Asn Thr Cys Asp Phe Leu Cys Val Asp Phe Cys Asn Met
        35                  40                  45

Glu Phe Ser Val Tyr Ala Asp Ala Leu Phe Trp His Leu His Ser Thr
    50                  55                  60

Asp Leu Val Leu Gly Asp Gly Ser Phe Asp Lys Leu Thr Pro Gly Asn
65                  70                  75                  80

Asp Trp Gly Trp Arg Val Gly Gly Ser Ala Arg Trp Asn Val Trp Asp
                85                  90                  95

Leu Ser Leu Arg Tyr Thr Ser Phe Arg Ser Lys Ala Asp Lys Glu Ala
            100                 105                 110

His Phe Asp Gln Val Pro Ile Val Ala Lys Tyr Ala Phe Asp Tyr Arg
        115                 120                 125

Val Phe Asp Ala Glu Leu Gly Ala Ser Cys Cys Ile Cys Asp Gly Leu

130                 135                 140

Leu Phe Arg Pro Phe Leu Gly Val Lys Phe Ala Asn Ile Asp Val Thr
145                 150                 155                 160

Asp Asp His Val Ser Glu Leu Glu Arg Asp Phe Thr Gln Gly Leu Asp
                165                 170                 175

Gly Ser Gly Leu Tyr Ile Gly Ala Ser Ser His Trp Lys Leu Cys Ser
            180                 185                 190

Phe Asn Ala Cys Gly Cys Cys Ile Pro Val Ala Phe Val Cys Arg Met
        195                 200                 205

Ser Thr Gly Ile Leu Asp Ser Asp Phe Thr Arg Thr Glu Pro Pro Ala
    210                 215                 220

Pro Thr Glu Lys Glu Arg Arg Glu Ser Lys Ala Phe Arg Phe Ile Pro
225                 230                 235                 240

Val His Glu Val Tyr Ala Gly Leu Glu Phe Arg Met Cys Val Leu Cys
                245                 250                 255

Asp Ala Asp Ala Phe Phe Gln Ile Gly Tyr Glu Ala Gln Tyr Trp Gly
            260                 265                 270

Trp Lys Gly Ile Ala Glu Asp Ala Leu Ser His Ala Tyr Leu Gly Val
        275                 280                 285

Gly Gly Leu Val Leu Arg Leu Gly Ala Asn Phe
    290                 295

<210>  27
<211>  304
<212>  PRT
<213>  Waddlia chondrophila

<400>  27

Met Lys His Leu Lys Asn Leu Val Leu Phe Val Leu Leu Ala Ser Ala
1               5                   10                  15

Gln Phe Ala Tyr Ser Asp Glu Ala Cys Asp Ala Cys Asp Pro Cys Tyr
            20                  25                  30

Met Gly Thr Glu Cys Asp Leu Cys Asn Ile Ser Leu Cys Asp Met Glu
        35                  40                  45

Phe Glu Leu Tyr Ala Asp Ala Leu Tyr Trp His Asn Asp Leu Ser Leu
    50                  55                  60

Ile Thr Asn Asp Gly Arg Lys Asp Gly Leu Asn Asp Gly Lys Thr Asp
65                  70                  75                  80

His Asp Tyr Asn Trp Gly Trp Arg Ile Gly Ala Ser Ala Tyr Trp Asn
                85                  90                  95

Ser Trp Asp Ile Gly Phe Arg Tyr Thr Ser Phe Ser Ser Thr Ala Lys
            100                 105                 110

Arg Asp Val Gln Phe Gly Glu Ser Ser Val Val Ala Ala Ser Glu Phe
        115                 120                 125

Asp Tyr Asp Val Phe Asp Val Glu Leu Gly Arg Ala Cys Cys Ile Cys
    130                 135                 140

Glu Gly Ile Leu Phe Arg Pro Phe Phe Gly Gly Lys Phe Ala Arg Ile
145                 150                 155                 160

Lys Val Asp Ile Arg Asn Ile Glu Phe Ile Phe Asp Lys Gly Leu Asp
                165                 170                 175

Arg Lys Leu Lys Leu Asp Gly Cys Gly Leu Tyr Val Gly Phe Asp Asn
            180                 185                 190

Arg Trp Gln Ile Cys Ser Phe Asn Val Cys Asp Arg Ser Ile Pro Val
        195                 200                 205

Ala Leu Val Ser Arg Phe Ser Thr Gly Val Leu Asp Gly Asp Phe Lys
    210                 215                 220

Tyr Ser Val Gly Glu Glu Val Gly Glu Glu Gly Ser Ala Lys Asp Cys
225                 230                 235                 240

Gln Phe Ile Pro Val His Glu Leu Tyr Ala Gly Leu Glu Phe Arg Met
                245                 250                 255

Cys Gly Leu Cys Asn Ala Asp Ala Phe Val Gln Val Gly Tyr Glu Val
            260                 265                 270

Gln Tyr Trp Gly Trp Arg Gly Glu Leu Val Arg Ala Ile Asp Ser Leu
        275                 280                 285

Ala His Leu Gly Ile Gly Gly Leu Val Leu Arg Leu Gly Ala Asn Phe
    290                 295                 300

<210>  28
<211>  374
<212>  PRT
<213>  Waddlia chondrophila

<400>  28

Met Met Lys Arg Cys Phe Leu Phe Leu Ser Val Phe Val Cys Cys Cys
1               5                   10                  15

Phe Phe Leu Ser Arg Val Asp Ala Gly Asn Cys Cys Ala Ser Gln Ser
            20                  25                  30

Tyr Ala Pro Gly Phe Arg Glu Thr Pro Leu Tyr Arg Pro Ser Cys Cys
        35                  40                  45

Asn Tyr Val Asp Ser Cys Ser Tyr Glu Cys Asp Arg Gln Cys Ser Ile
    50                  55                  60

Glu Ile Gly Ala Glu Phe Leu Tyr Arg Lys Val Cys Leu Asp Glu Phe
65                  70                  75                  80

Asn Trp Ala Phe Thr Arg Thr Glu Thr Thr Glu Ala Asn Val Thr Ser
                85                  90                  95

Ile Asp Leu Ser Tyr Glu Arg Leu Cys Leu Asp Asp Glu Pro Gly Val
            100                 105                 110

Arg Ala Trp Leu Gly Phe Gln Pro Ala Gly Lys Glu Ser Leu Gly Ile
        115                 120                 125

Tyr Phe Gly Tyr Val Tyr Leu Ser Gly Ser Lys Asn Asp Ser Ile Gln
    130                 135                 140

Gln Ser Phe Gly Asn Thr Ile Gly Thr Thr Val Met His Tyr Gly Leu
145                 150                 155                 160

Phe Gln Glu Ser Ala Gly Phe Thr Asp Ile Ala Val Asp Trp Arg Asn
                165                 170                 175

Gln Tyr His Glu Gly Glu Val Val Leu Gly Ser Ser Ala Phe Cys Gly
            180                 185                 190

Lys Asn His Val Phe Leu Pro Tyr Phe Gly Ala Ala Gly Ile Tyr Leu
        195                 200                 205

Glu Gln Lys Phe Ala Leu Asp Leu Ser Asp Pro Asp Leu Val Phe Ala
    210                 215                 220

Gly Asp Ala Gly Ala Val Asp Trp Glu Ser Gln Tyr Trp Gly Val Gly
225                 230                 235                 240

Leu Arg Phe Gly Ser His Tyr Lys Tyr Gln Leu Gly Ser Cys Leu Ala
                245                 250                 255

Phe Ile Gly Arg Phe Asn Ala Ser Leu Leu Val Gly Glu Asn Lys Phe
            260                 265                 270

His Asn Phe Gln Ser Val Ile Phe Ser Ser Glu Ala Glu Glu Leu Gln
        275                 280                 285

Leu Asn Ile Ser Gly Lys Asn Lys Cys His Val Val Pro Gly Tyr Ser
    290                 295                 300

Leu Gly Ala Gly Phe Ala Ile Glu Pro Glu Ile Cys Gly Cys Thr Leu
305                 310                 315                 320

Ser Leu Arg Val Gly Tyr Glu Phe Asn Glu Trp Tyr Asn Ile Pro Lys
                325                 330                 335

His Arg Thr Phe Ser Gly Glu Ser Thr Val Asp Gly Glu Val Ala Tyr
            340                 345                 350

Ser Asp Ser Ala Ser Asn Arg Thr Trp Gly Ser His Gly Leu Phe Ala
        355                 360                 365

Gly Leu Ser Phe Ala Phe
    370

<210>  29
<211>  361
<212>  PRT
<213>  Waddlia chondrophila

<400>  29

Met Ser Trp Lys Lys Trp Ile Val Ala Leu Ser Thr Val Val Ser Ser
1               5                   10                  15

Thr Gln Phe Thr Pro Ala Asn Ala Ser Asp Tyr Tyr Ser Cys Asp Thr
            20                  25                  30

Gly Ser Asp Cys Gly Phe Ser Leu Cys Asp Cys Asp Pro Cys Glu Gly
        35                  40                  45

Trp Ser Ile Tyr Ala Asp Tyr Leu Tyr Trp Arg Pro Arg Arg Cys Asp
    50                  55                  60

Leu Asp Tyr Ala Phe Thr Tyr Val Gly Ala Glu Gly Ala Ile Gly Gly
65                  70                  75                  80

Asp Val Arg Lys Leu Ala Gly Ile Cys Pro Ser Tyr Asp Ser Gly Phe
                85                  90                  95

Arg Ile Gly Val Leu Lys Ala Cys Gly Asp Leu Asp Leu Gly Ile His
            100                 105                 110

Tyr Thr Trp Phe Arg Ser Lys Asp Gly Ser Ser Ile Asn Asn Val Asp
        115                 120                 125

Gln Asp Phe Ala Ile Gly Ile Thr Gly Ser Ser Gly Val Val Phe Ser
    130                 135                 140

Asp Ile Ala Gly Glu Thr Pro Pro Leu Ile Glu Phe Ala Ala Ser Asp
145                 150                 155                 160

Tyr Lys Val Glu Leu Asn Gln Val Asp Val Glu Leu Gly Tyr His Leu
                165                 170                 175

Glu Leu Ser Asp Cys Leu Ala Thr Arg Leu Phe Ser Gly Phe Arg Tyr
            180                 185                 190

Ala Asn Ile Lys Gln Thr Arg Asp Ser Ile Tyr Ser Ser Ser Ile Glu
        195                 200                 205

Asp Pro Leu Gly Glu Asp Ser Ala Ala Asp Val Ile Phe Gln Lys Ala
    210                 215                 220

Asp Leu Asp Phe Tyr Gly Leu Tyr Ile Gly Asn Lys Ala Ser Tyr Lys
225                 230                 235                 240

Ile Cys Asp Cys Phe Asp Phe Phe Gly Gly Leu Ser Leu Gly Ile Gly
                245                 250                 255

Val Gly Glu Ala Ser Gln Asp Phe Arg Tyr Ser Gly Lys Ser Ala Gly
            260                 265                 270

Glu Gly Asn Glu Phe Ile Gln Gly Pro Leu Val Ser Gly Ser Cys Trp
        275                 280                 285

Lys Ser Ile Ala Val Leu Asp Leu Asn Val Gly Ile Thr Phe Pro Leu
    290                 295                 300

Cys Asn Val Cys Cys Thr Asp Leu Ala Phe Ser Val Gly Tyr Glu Phe
305                 310                 315                 320

His Gln Trp Phe Asn Leu Gly Gly Phe Thr Asp Leu Ser Lys Ser Gly
                325                 330                 335

Asp Ile Gly Leu Val Ser Ser Asn Cys Cys Asp Leu Gly Phe Asp Gly
            340                 345                 350

Leu Phe Val Arg Leu Ser Ala Ala Phe
        355                 360

<210>  30
<211>  365
<212>  PRT
<213>  Waddlia chondrophila

<400>  30

Met Ser Trp Lys Lys Trp Ile Val Ala Leu Ser Thr Val Val Phe Ser
1               5                   10                  15

Thr Gln Phe Ser Pro Ala Asn Ala Gly Ser Tyr Ser Ser Cys Asp Thr

20                  25                  30

Gly Ser Asp Cys Gly Phe Ser Phe Cys Asp Ser Asp Pro Cys Glu Gly
        35                  40                  45

Trp Ser Ile Tyr Ala Asp Tyr Leu Tyr Trp Arg Pro Arg Lys Cys Asp
    50                  55                  60

Leu Asp Tyr Ala Ile Thr Tyr Ile Glu Asn Gly Ser Glu Leu Gln Glu
65                  70                  75                  80

Ala Phe Ala Thr Gly Leu Ser Gly Ile Cys Pro Ser Tyr Asp Ser Gly
                85                  90                  95

Phe Arg Ile Gly Ile Leu Lys Ala Phe Gly Asp Leu Asp Phe Gly Val
            100                 105                 110

His Tyr Thr Trp Phe Arg Ser Lys Asp Gly Ser Ser Ile Asn Asn Val
        115                 120                 125

Gly Glu Asp Phe Leu Ile Leu Pro Thr Lys Ile Gly Ser Ala Ser Leu
    130                 135                 140

Phe Val Thr Asp Gly Ile Gln Leu Ala Ala Ser Asp Tyr Lys Ile Glu
145                 150                 155                 160

Leu Asn Gln Val Asp Val Glu Leu Gly Tyr His Leu Glu Leu Ser Asp
                165                 170                 175

Cys Leu Ala Ala Arg Leu Phe Ser Gly Phe Arg Tyr Ala Asn Ile Lys
            180                 185                 190

Gln Thr Gln Asp Ser Val Tyr Ser Ser Asn Ile Asn Asp Gln Phe Ala
        195                 200                 205

Glu Ser Ser Leu Asn Gln Val Asp Val Ile His Gln Lys Ala Asp Leu
    210                 215                 220

Asp Phe Tyr Gly Leu Tyr Val Gly Asn Lys Ala Ser Tyr Lys Ile Cys
225                 230                 235                 240

Asp Cys Phe Asp Phe Phe Gly Gly Leu Ser Leu Gly Ile Gly Val Gly
                245                 250                 255

Glu Val Ser Gln Asp Phe Asp His Lys Gly Lys Ser Ile Gly Gly Thr
            260                 265                 270

Asp Tyr Thr Gln Ala Asp Leu Val Asn Glu Ser Cys Trp Lys Ser Ile
        275                 280                 285

Ala Val Leu Asp Leu Asn Val Gly Ile Thr Phe Pro Leu Cys Asn Val

290                 295                 300

Cys Cys Thr Asp Leu Ala Phe Ser Val Gly Tyr Glu Phe His Gln Trp
305                 310                 315                 320

Phe Asn Leu Gly Gly Phe Thr Ser Leu Ser Arg Thr Val Asp Ser Glu
                325                 330                 335

Gly Glu Ala Ala Gly Arg Gly Leu Val Ser Ser Asn Cys Cys Asp Leu
            340                 345                 350

Gly Phe Asp Gly Leu Phe Val Arg Leu Ser Ala Ala Phe
        355                 360                 365

<210>  31
<211>  356
<212>  PRT
<213>  Waddlia chondrophila

<400>  31

Met Ser Trp Lys Lys Trp Ile Val Ala Leu Ser Thr Val Val Phe Ser
1               5                   10                  15

Thr Gln Phe Ser Pro Ala Ser Ala Gly Asn Tyr Tyr Asn Cys Asp Thr
            20                  25                  30

Gly Ser Asp Cys Gly Phe Ser Phe Cys Asp Ser Asp Pro Cys Glu Gly
        35                  40                  45

Trp Ser Ile Tyr Ala Asp Tyr Leu Tyr Trp Arg Pro Arg Arg Cys Asp
    50                  55                  60

Leu Asn Tyr Ala Phe Thr Thr Leu Asp Thr Glu Asn Glu Asn Leu Ala
65                  70                  75                  80

Phe Val Thr Gly Leu Ser Gly Ile Cys Pro Ser Tyr Asp Ser Gly Phe
                85                  90                  95

Arg Ile Gly Ile Leu Lys Ala Phe Gly Asp Leu Asp Phe Gly Val His
            100                 105                 110

Tyr Thr Trp Phe Arg Ser Lys Asp Gly Ser Ser Ile Asn Asn Val Gly
        115                 120                 125

Glu Asp Phe Ile Ile Gly Gln Thr Lys Val Ile Ser Pro Phe Val Thr
    130                 135                 140

Asp Pro Ile Glu Leu Ala Ala Ser Asp Tyr Lys Val Glu Leu Asn Gln
145                 150                 155                 160

Ile Asp Ala Glu Leu Gly Tyr His Leu Glu Leu Ser Asp Cys Leu Ala
                165                 170                 175

Ala Arg Ile Phe Ser Gly Phe Arg Tyr Ala Lys Ile Lys Gln Ser Gln
            180                 185                 190

Asn Ser Val Tyr Ser Ser Asp Ile Asn Asp Gln Tyr Ala Asn Ser Asn
        195                 200                 205

Pro Leu Ala Gln Val Asp Val Ile Gln Gln Lys Ala Asp Leu Asp Phe
    210                 215                 220

Tyr Gly Leu Tyr Val Gly Asn Lys Ala Ser Tyr Lys Ile Cys Asp Cys
225                 230                 235                 240

Phe Asp Phe Phe Gly Gly Leu Ser Leu Gly Ile Gly Val Gly Asp Val
                245                 250                 255

Ser Gln Arg Ile Asp Gln Gln Gly Lys Ala Asp Gly Gly Pro Asp Val
            260                 265                 270

Glu Phe Ala Gln Gly Asp Leu Val Ser Thr Ser Cys Trp Lys Ser Ile
        275                 280                 285

Ala Val Leu Asp Leu Asn Val Gly Ile Thr Phe Pro Leu Cys Asn Val
    290                 295                 300

Cys Cys Thr Asp Leu Ala Phe Ser Val Gly Tyr Glu Phe His Gln Trp
305                 310                 315                 320

Phe Asn Val Ser Asp Phe Ile Ala Phe Pro Phe Gly Asn Tyr Gly Val
                325                 330                 335

Asp Ser His Cys Cys Asp Leu Ser Phe Asp Gly Leu Phe Val Arg Val
            340                 345                 350

Ser Ala Ala Phe
        355

<210>  32
<211>  373
<212>  PRT
<213>  Waddlia chondrophila

<400>  32

Met Arg Pro Asp Pro His Leu Leu Thr Ile Phe Ile Val His Asn Phe
1               5                   10                  15

Glu His Tyr Leu Arg Leu Leu Thr His Asp Met Glu Glu Thr Met Arg
            20                  25                  30

Ser Ile Lys Asn Ser Ile Leu Ile Leu Leu Ser Ala Ala Gly Cys Leu
        35                  40                  45

Thr Leu Ser Pro Val Gly Ala Ala Glu Ile Cys Glu Cys Gly Phe Thr
    50                  55                  60

Ile Gly Gly Asp Tyr Leu Tyr Trp Ser Pro Cys Leu Ser Asn Leu His
65                  70                  75                  80

Phe Ala Ala Glu Gly Gln Ser Ser Phe Thr Glu Glu Ser Asn Val Ser
                85                  90                  95

Asn Ile Val Asn Tyr His Phe Ile Asp Pro Asp Trp Asn Gly Gly Phe
            100                 105                 110

Arg Leu Tyr Leu Gly Lys Glu Asp Leu Cys Gly Cys Phe Asp Val Met
        115                 120                 125

Ala Thr Tyr Thr Asn Phe Ser Ser Lys Cys Phe Asp Ser Ile Val Asn
    130                 135                 140

Lys Asp Pro Leu Leu Ser Leu Thr Trp Pro Thr Pro Leu Pro Val Glu
145                 150                 155                 160

Tyr Gly Arg Tyr Ala Thr Thr Asp Trp Lys Ile Asp Phe Gln Arg Ala
                165                 170                 175

Glu Leu Val Val Gly Tyr Ser Ile Glu Phe Gly Lys Gly Cys Gly Leu
            180                 185                 190

Cys Leu Lys Pro Phe Ala Gly Ala Asp Tyr Ala Ser Ile Cys Gln Asp
        195                 200                 205

Arg Ile Asp Lys Met His Asp Val Lys Pro Ile Glu Gly Asp Asp Lys
    210                 215                 220

Asn Ser Pro Val Asp Pro Ser Leu Thr Asn Thr Leu Thr Arg Ser Leu
225                 230                 235                 240

Asp Tyr Lys Gly Ile Gly Pro Met Val Gly Ile Gly Tyr Asp Phe Glu
                245                 250                 255

Leu Phe Glu Gly Leu Ser Thr Phe Gly Lys Ala Ser Leu Ser Leu Leu
            260                 265                 270

Val Gly Lys Ala Asp Ile Glu Asp Glu Gln Glu Ile Phe Asn Asn Gly
        275                 280                 285

Thr Thr Thr Val Asn Val Gln Lys Tyr Lys Asp Lys Cys Val Cys Val
    290                 295                 300

Pro Gly Val His Leu Gln Thr Gly Leu Ser Leu Lys Thr Leu Val Cys
305                 310                 315                 320

Asn Lys Trp Leu Leu Leu Arg Ala Gly Tyr Glu Tyr Leu Gln Tyr Ile
                325                 330                 335

His Ala Pro Ser His Leu Met Tyr Asn Thr Glu Ser Pro Gly Ser Phe
            340                 345                 350

Thr Gly Leu Asn His Asn Ser Val Thr Phe Gln Gly Phe Phe Gly Gly
        355                 360                 365

Val Ala Phe Ser Phe
    370

<210>  33
<211>  333
<212>  PRT
<213>  Waddlia chondrophila

<400>  33

Met Ile Asn Met Lys Lys Ala Val Ala Ala Ile Leu Leu Thr Phe Ala
1               5                   10                  15

Ser Leu Thr Thr Thr Ala Gln Ala Asp Cys Trp Asp Ser Ser Cys Asn
            20                  25                  30

Asp Cys Cys Pro Asp Ile Thr Val Gly Val Asp Trp Leu Tyr Trp Ser
        35                  40                  45

Pro Cys Ile Asn Asp Arg His Phe Ala Leu Thr Thr Asp Gly Val Asp
    50                  55                  60

Val Asn Thr His Tyr Phe Cys Asn Glu Trp Asp Phe Gly Ser Arg Val
65                  70                  75                  80

Tyr Ala Lys Leu Gly Asn Trp Trp Asn Gly Phe Asn Gly Ala Leu Ile
                85                  90                  95

Tyr Thr Tyr Ile Asn Pro Lys Thr Ser Ser Ser Leu Lys Ser Asp Ala
            100                 105                 110

Ile Ile Leu Ser Val Ala Ala Pro Asp Glu Ile Pro Ser Phe Asn Asp
        115                 120                 125

Leu Asp Ala Lys Trp Glu Leu Gln Tyr Gln Thr Leu Asp Ala Val Leu
    130                 135                 140

Ser Tyr Ser Ile Asp Val Thr Gln Asn Arg Cys Phe Lys Ile Glu Val
145                 150                 155                 160

Phe Ser Gly Leu Thr Trp Ile Asp Val Lys Gln Lys Thr Glu Tyr Val
                165                 170                 175

Phe Asp Leu Asn Glu Gly Gly Phe Thr Asn Arg Asn Phe Asp Arg Gln
            180                 185                 190

Asn Asp Phe Trp Ala Val Gly Pro Cys Leu Gly Leu Asn Ser Ser Phe
        195                 200                 205

Ser Phe Phe Asp Cys Phe Lys Ile Phe Gly Thr Leu Lys Thr Asn Leu
    210                 215                 220

Val Val Gly Glu Thr Ala Ser Lys Asp Val Leu Phe Arg Lys Val Ala
225                 230                 235                 240

Gly Asp Glu Lys Asp Ser Val Lys Asp Glu Ile Leu Thr Thr Cys Lys
                245                 250                 255

Val Lys Asp Arg Cys Phe Cys Leu Pro Gly Leu His Leu Ala Ser Gly
            260                 265                 270

Ile Glu Tyr Glu Ile Cys Leu Tyr Glu Thr Thr Phe Gly Leu Arg Leu
        275                 280                 285

Gly Trp Glu Tyr Val Gln Trp Ile Asn Ala Pro Thr Phe Pro Cys Tyr
    290                 295                 300

Glu Leu Asp Gly Ser Arg Val Arg Ser Ala Pro Ser Thr Asn Asn Leu
305                 310                 315                 320

Thr Met Gln Gly Ile Phe Phe Gly Leu Asn Ser Thr Phe
                325                 330

<210>  34
<211>  341
<212>  PRT
<213>  Waddlia chondrophila

<400>  34

Met Met Ser Ile Lys Lys Thr Met Ala Ala Ile Phe Leu Ser Val Ala
1               5                   10                  15

Ser Leu Thr Thr Ser Val Gln Ala Asn Tyr Trp Gly Ser Cys Asn Asp
            20                  25                  30

Cys Cys Pro Asp Val Thr Val Gly Val Asp Trp Leu Tyr Trp Ser Pro
        35                  40                  45

Cys Ala Ser Asp Arg His Phe Ala Leu Thr Thr Glu Gly Ile Asp Ser
    50                  55                  60

Glu Val Asn Val Lys Thr His Tyr Leu Cys Asp Glu Trp Asp Ser Gly
65                  70                  75                  80

Val Arg Val Tyr Gly Lys Leu Gly Asn Leu Trp Asn Gly Phe Asn Gly

85                  90                  95

Gly Leu Ile Tyr Thr Tyr Ile Asn Pro Lys Thr Ser Gly Ser Val Glu
            100                 105                 110

Gln Ile Gly Asn Asn Gly Val Val Phe Ser Phe Gly Leu Pro Ile Pro
        115                 120                 125

Gly Glu Ser Gly Gly Leu Leu Gln Gly Ser Arg Leu Asp Ala Lys Trp
    130                 135                 140

Glu Leu Gln Tyr Gln Thr Leu Asp Ala Ile Leu Ser Tyr Ser Ile Asp
145                 150                 155                 160

Val Thr Gln Asn Arg Cys Phe Lys Ile Glu Ala Phe Ser Gly Leu Thr
                165                 170                 175

Trp Val Asp Val Lys Gln Lys Ala Ser Tyr Thr Leu Asp Gln Val Gly
            180                 185                 190

Gly Asn Glu Ile Glu Ile Arg Val Asp Phe Asp Arg Asn Asn Asp Phe
        195                 200                 205

Trp Ala Ile Gly Pro Cys Phe Gly Leu Asn Thr Ser Phe Arg Phe Cys
    210                 215                 220

Asp Cys Phe Asn Ile Phe Gly Leu Val Lys Thr Ser Leu Val Val Gly
225                 230                 235                 240

Glu Ser Lys Asn Lys Asp Val Ile Tyr Gly Thr Arg Gly Ser Ser Glu
                245                 250                 255

Thr Ser Thr Phe Val Thr Val Thr Ala Lys Asp Lys Cys Phe Cys Phe
            260                 265                 270

Pro Gly Leu His Leu Ala Thr Gly Ile Asp Tyr Glu Leu Cys Leu Cys
        275                 280                 285

Asn Ile Thr Leu Asp Leu Arg Leu Gly Trp Glu Tyr Val Gln Trp Ile
    290                 295                 300

Asn Ala Pro Thr Phe Pro Tyr Tyr Glu Leu Asp Gly Leu Gly Val Arg
305                 310                 315                 320

Ser Ala Pro Ser Thr Asn Asp Leu Thr Met Gln Gly Ile Phe Leu Gly
                325                 330                 335

Leu Asn Thr Thr Phe
            340

<210>  35

<211>  372
<212>  PRT
<213>  Waddlia chondrophila

<400>  35

Met Thr Arg Arg Leu Leu Leu Ser Ile Phe Cys Ala Leu Ile Cys Thr
1               5                   10                  15

Asn Pro Phe Leu Glu Ala Gln Cys Gly Lys Ser Trp Leu Ser Thr Ser
            20                  25                  30

Pro Cys Pro Pro Val Thr Thr His Thr Tyr Trp Asp Tyr Ser Pro Cys
        35                  40                  45

Asn Thr Ala Ser Asn Gln Trp Lys Ser Ser Pro Cys Glu Tyr Gly Cys
    50                  55                  60

Asn Asn Arg Cys Gly Phe Asn Ala Gly Ile Asp Tyr Leu Tyr Trp Thr
65                  70                  75                  80

Val Cys Gln Asp Asn Leu Asp Phe Ser Val Asp His Thr Ile Ala Asp
                85                  90                  95

Asp Pro Pro Thr Leu Leu Gly Thr Gly Lys Thr His Phe Leu Lys Tyr
            100                 105                 110

Gly Trp Ser Ser Gly Val Arg Ala Tyr Leu Gly Leu Glu Leu Leu Gly
        115                 120                 125

Phe Glu Ile Arg Gly Gln Tyr Thr Trp Ile Glu Asn Lys Ala Lys Glu
    130                 135                 140

Thr Lys Asp Tyr Thr Asp Thr Asp Ala Val Leu Val Ala Ser Leu Leu
145                 150                 155                 160

His Pro Asn Thr Gly Leu Ala Asn Ala Arg Ile Ala Gln Gly Phe Gln
                165                 170                 175

Asp Leu Trp Tyr Glu Thr Ala Asp Leu Leu Phe Ser Arg Glu Met Glu
            180                 185                 190

Phe Phe Asp Tyr Lys Leu Ile Leu Arg Pro Phe Ile Gly Ala Lys Trp
        195                 200                 205

Met Arg Ile Lys Gln Lys Leu Lys Val Thr Tyr Glu Glu Glu Asp Phe
    210                 215                 220

Ala Glu Asn Pro Glu Gln Ile Arg Trp Asn Ser Thr Leu Ser Gly Pro
225                 230                 235                 240

Gly Ala His Ala Gly Phe Glu Leu Phe Tyr Arg Trp Val Ser Gly Leu
                245                 250                 255

Gly Met Tyr Gly Gln Leu Ala Gly Ser Val Ile Gly Ala Val Val Asp
            260                 265                 270

Asn His His Ile Gln Val Ser Leu Asp Glu Asn Gly Ala Asp Val Thr
        275                 280                 285

Pro Pro Arg Ile Gln Leu Lys Glu Thr Gln Arg Leu Cys Val Pro Gly
    290                 295                 300

Tyr Gln Leu Glu Ala Gly Ile Thr Trp Asp Met Cys Cys Leu Asn Asn
305                 310                 315                 320

Tyr Asn Ile Arg Phe Arg Phe Cys Tyr Glu Leu Asn Gln Trp Leu Asn
                325                 330                 335

Thr Pro Glu Val Arg Arg Tyr His Tyr Glu Asn Glu Gly Val Ser Ser
            340                 345                 350

Ala Ala Thr Asp Gly Ser Ile Ala Leu His Gly Ala Thr Phe Gly Ile
        355                 360                 365

Asp Leu Arg Phe
    370

<210>  36
<211>  374
<212>  PRT
<213>  Waddlia chondrophila

<400>  36

Met Lys Arg His Ile Ala Lys Phe Leu Ile Phe Ile Phe Leu Ser Ser
1               5                   10                  15

Ile Asn Val Glu Ala Asp Cys Cys Lys Asn Ile Thr Tyr Thr Lys Pro
            20                  25                  30

Pro Pro Pro Ile Arg Pro Cys Cys Cys Ser Asp Asp Cys Tyr Ser Glu
        35                  40                  45

Pro Pro Arg Thr Tyr Asp Pro Tyr Cys Tyr Ala Cys Asp Gln Phe Cys
    50                  55                  60

Pro Gly Glu Cys His Phe Phe Phe Gly Ala Glu Gly Leu Trp Trp Thr
65                  70                  75                  80

Ala Cys Glu Asn Asp Leu Asp Phe Ala Val Asp Phe Asn Thr Glu Gln
                85                  90                  95

Thr Glu Ile Leu Gly Ile Gly Asn Thr His Phe Ala Ser Tyr Asp Trp
            100                 105                 110

Asp Trp Gly Val Arg Gly Trp Leu Gly Trp Asn Trp Cys Cys Gly Trp
        115                 120                 125

Asp Thr Val Val Ser Tyr Thr Trp Phe Lys Thr Glu Gly Asn Gln Phe
    130                 135                 140

Ile Asp Thr Thr Asp Glu Asp Ile Ser Leu Lys Ala Ser Leu Leu His
145                 150                 155                 160

Pro Asn Thr Gly Leu Ser Asp Ala Asp Thr Ala Thr Ala Lys Leu Asp
                165                 170                 175

Leu Lys Tyr Gln Ala Leu Asp Val Leu Phe Gly Arg Thr Ile Val Tyr
            180                 185                 190

Cys Glu Asp Thr Val Gln Leu His Pro Phe Phe Gly Phe His Gly Ile
        195                 200                 205

Trp Leu Lys His Asp Gln Ser Tyr Leu Tyr Glu Gly Gly Asp Phe Gly
    210                 215                 220

Thr Gly Ser Leu Ala Ala Pro Ala Arg Val Thr Trp Asn Ser Thr Leu
225                 230                 235                 240

Lys Gly Ala Gly Leu Ile Gly Gly Ala Asp Val Asn Leu Lys Trp Pro
                245                 250                 255

Cys Gly Ile Gly Ile Phe Gly Ser Met Gly Gly Ser Ile Leu Ala Ser
            260                 265                 270

Lys Thr Asp Ile Glu His Leu Gln Glu Ile Leu Asp Ile Ser Gly Asn
        275                 280                 285

Ile Thr Ser Thr Asp Ile Asp Leu Lys Glu Asp Gln Cys Val Cys Ile
    290                 295                 300

Pro Gly Leu His Leu Lys Thr Gly Leu Ser Phe Gln Phe Ala Cys Gly
305                 310                 315                 320

Gln Cys Leu Leu Met Lys Phe His Leu Met Tyr Glu Phe Asn Asn Tyr
                325                 330                 335

Phe Asn Thr Pro His Leu Arg Arg Tyr Ser Tyr Asn Asn Glu Gly Val
            340                 345                 350

Ser Ser Ser Ser Ala Ser Gly Asn Thr Ala Leu Gln Gly Ile Thr Val
        355                 360                 365

Gly Gly Glu Ile Phe Phe
    370

<210>  37
<211>  36
<212>  DNA
<213>  Artificial

<220>
<223>  Primer WaA3 forward W. chondrophila

<400>  37
aaaaaaccat ggaaactagt acagggggaa tacctc                                36


<210>  38
<211>  32
<212>  DNA
<213>  Artificial

<220>
<223>  Primer WaA3 reverse W. chondrophila

<400>  38
gatgtcgaca ctttgtcgat caaccactga aa                                    32


<210>  39
<211>  34
<212>  DNA
<213>  Artificial

<220>
<223>  Primer WaA4 forward W. chondrophila

<400>  39
aaaaaaccat gggtacagac agaacgcaaa acct                                  34


<210>  40
<211>  39
<212>  DNA
<213>  Artificial

<220>
<223>  Primer WaA4 reverse W. chondrophila

<400>  40
aaaaaactcg agagttgttt cttcttcctg ctcctgttg                             39


<210>  41
<211>  36
<212>  DNA
<213>  Artificial

<220>
<223>  Primer WaA22 forward W. chondrophila

<400>  41
aaaaaaccat gggtaaattt tcaacagttg cttctc                                36


<210>  42
<211>  40
<212>  DNA
<213>  Artificial

<220>
<223>  Primer WaA22 reverse W. chondrophila

<400>  42
aaaaaactcg agtagatggt tttctaaaaa ctccatcatc                              40


<210>  43
<211>  32
<212>  DNA
<213>  Artificial

<220>
<223>  Primer WaA 24 forward W. chondrophila

<400>  43
aaaaaaccat ggaaaagagg cagtttcttt cc                                      32


<210>  44
<211>  30
<212>  DNA
<213>  Artificial

<220>
<223>  Primer WaA24 reverse W. chondrophila

<400>  44
aaaaaactcg agtaagtgaa tgggtttttc                                         30